# EUROPÄISCHE PATENTSCHRIFT

(11) **EP 2 350 074 B1**
(45) Veröffentlichungstag und Bekanntmachung des Hinweises auf die Patenterteilung: **06.03.2013**
(21) Anmeldenummer: 09784000.3
(22) Anmeldetag: 14.10.2009
(51) Int. Cl.: C07D 471/04, A01N 43/90

(54) **SUBSTITUIERTE PYRIDINE MIT HERBIZIDER WIRKUNG**
SUBSTITUTED PYRIDINES HAVING A HERBICIDAL EFFECT
PYRIDINES SUBSTITUÉES À ACTION HERBICIDE

(30) Priorität: 29.10.2008 EP 08167850
(43) Veröffentlichungstag der Anmeldung: 03.08.2011
(73) Patentinhaber: BASF SE, 67056 Ludwigshafen (DE)
(72) Erfinder: SONG, Dschun, 68167 Mannheim (DE); HUPE, Eike, 68161 Mannheim (DE); PILGER, Christian, 67063 Ludwigshafen (DE); NEWTON, Trevor, William, 67435 Neustadt (DE); WITSCHEL, Matthias, 67098 Bad Dürkheim (DE); MOBERG, William, Karl, 67433 Neustadt (DE); PARRA RAPADO, Liliana, 77654 Offenburg (DE); QU, Tao, 67063 Ludwigshafen (DE); STELZER, Frank, 68309 Mannheim (DE); VESCOVI, Andrea, E-08010 Barcelona (ES); SEITZ, Thomas, 68519 Viernheim (DE); EHRHARDT, Thomas, 67346 Speyer (DE); KREUZ, Klaus, 79211 Denzlingen (DE); GROSSMANN, Klaus, 67141 Neuhofen (DE); REINHARD, Robert, 67117 Limburgerhof (DE); SIMON, Anja, 69469 Weinheim (DE); NIGGEWEG, Ricarda, 68165 Mannheim (DE); SIEVERNICH, Bernd, 67454 Haßloch (DE)
(86) Internationale Anmeldenummer: PCT/EP2009/063387
(87) Internationale Veröffentlichungsnummer: WO 2010/049270

(56) Entgegenhaltungen:
- WO-A-2004/018419
- WO-A-2005/010000
- WO-A-2008/071918
- WO-A-2009/090401
- US-A- 5 801 183
- US-A1- 2004 087 577
- FRAZIER, KELLY ET AL: "Design and structure?activity relationship of heterocyclic analogs of 4-amino-3-benzimidazol-2-ylhydroquinolin-2 -ones as inhibitors of receptor tyrosine kinases" BIOORGANIC & MEDICINAL CHEMISTRY LETTERS; ENGLISH, Bd. 16, Nr. 8, 2006, Seiten 2247-2251, XP002557592
- ZHOU, ZHANG-LIN ET AL: "Synthesis and SAR of 5-, 6-, 7- and 8-Aza Analogues of 3-Aryl-4-hydroxyquinolin-2(1H)-one as NMDA/Glycine Site Antagonists" BIOORGANIC & MEDICINAL CHEMISTRY; ENGLISH, Bd. 9, Nr. 8, 2001, Seiten 2061-2072, XP002557591
- CHEN, JIAN-LONG ET AL: "Synthesis of some Benzofuronaphthyridines and Benzofuronaphthyridine Derivatives" JOURNAL OF HETEROCYCLIC CHEMISTRY; ENGLISH, Bd. 30, Nr. 4, 1993, Seiten 909-912, XP009027298

## Beschreibung

Die vorliegende Erfindung betrifft substituierte Pyridine der Formel I 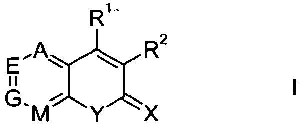 worin die Variablen folgende Bedeutung haben
R¹ O-R-^{A}, S(O)ₙ-R^{A} oder O-S(O)ₙ-R^{A};
R^{A} Wasserstoff, C₁-C₄-Alkyl, Z-C₃-C₆-Cycloalkyl, C₁-C₄-Haloalkyl, C₂-C₆-Alkenyl, Z-C₃-C₆-Cycloalkenyl, C₂-C₆-Alkinyl, Z-(Tri-C₁-C₄-alkyl)silyl, Z-C(=O)-R^{a}, Z-NRⁱ-C(O)-NRⁱRⁱⁱ, Z-P(=O)(R^{a})₂, NRⁱRⁱⁱ, 3- bis 7-gliedriger monocyclischer oder 9- oder 10-gliedriger bicyclischer gesättigter, ungesättigter oder aromatischer Heterocyclus, enthaltend 1, 2, 3 oder 4 Heteroatome ausgewählt aus O, N und S, der teilweise oder vollständig durch Gruppen R^{a} und/oder R^{b} substituiert sein kann,
R^{a} Wasserstoff, OH, C₁-C₈-Alkyl, C₁-C₄-Haloalkyl, Z-C₃-C₆-Cycloalkyl, C₂-C₈-Alkenyl, Z-C₅-C₆-Cycloalkenyl, C₂-C₈-Alkinyl, Z-C₁-C₆-Alkoxy, Z-C₁-C₄-Haloalkoxy, Z-C₃-C₈-Alkenyloxy, Z-C₃-C₈-Alkinyloxy, NRⁱRⁱⁱ, C₁-C₆-Alkylsulfonyl, Z-(Tri-C₁-C₄-alkyl)silyl, Z-Phenyl, Z-Phenoxy, Z-Phenylamino und 5- oder 6-gliedriger monocyclischer oder 9- oder 10-gliedriger bicyclischer Heterocyclus, enthaltend 1, 2, 3 oder 4 Heteroatome ausgewählt aus O, N und S, wobei die cyclischen Gruppen unsubstituiert oder durch 1, 2, 3 oder 4 Gruppen R^{b} substituiert sind, bedeutet;
Rⁱ,Rⁱⁱ unabhängig voneinander Wasserstoff, C₁-C₈-Alkyl, C₁-C₄-Haloalkyl, C₃-C₈-Alkenyl, C₃-C₈-Alkinyl, Z-C₃-C₆-Cycloalkyl, Z-C₁-C₈-Alkoxy, Z-C₁-C₈-Haloalkoxy, Z-C(=O)-R^{a}, Z-Phenyl, über Z gebundener 3- bis 7-gliedriger monocyclischer oder 9- oder 10-gliedriger bicyclischer gesättigter, ungesättigter oder aromatischer Heterocyclus, enthaltend 1, 2, 3 oder 4 Heteroatome ausgewählt aus O, N und S;
Rⁱ und Rⁱⁱ können auch gemeinsam mit dem N-Atom, an das sie gebunden sind, einen 5- oder 6-gliedrigen monocyclischen oder 9- oder 10-gliedrigen bicyclischer Heterocyclus bilden, enthaltend 1, 2, 3 oder 4 Heteroatome ausgewählt aus O, N und S;
Z eine kovalente Bindung oder C₁-C₄-Alkylen;
n 0, 1 oder 2;
R² Phenyl, Naphthyl oder und 5- oder 6-gliedriger monocyclischer oder 9- oder 10-gliedriger bicyclischer aromatischer Heterocyclus, enthaltend 1, 2, 3 oder 4 Heteroatome ausgewählt aus O, N und S, wobei die cyclischen Gruppen unsubstituiert oder durch 1, 2, 3 oder 4 Gruppen R^{b} substituiert sind, bedeutet;
R^{b} unabhängig voneinander Z-CN, Z-OH, Z-NO₂, Z-Halogen, C₁-C₈-Alkyl, C₁-C₄-Haloalkyl, C₂-C₈-Alkenyl, C₂-C₈-Alkinyl, Z-C₁-C₈-Alkoxy, Z-C₁-C₈-Halo-alkoxy, Z-C₃-C₁₀-Cycloalkyl, O-Z-C₃-C₁₀-Cycloalkyl, Z-C(=O)-R^{a}, NRⁱRⁱⁱ, Z-(Tri-C₁-C₄-alkyl)silyl, Z-Phenyl und S(O)ₙR^{bb}, wobei
R^{bb} C₁-C₈-Alkyl und C₁-C₆-Haloalkyl bedeutet und n für 0, 1 oder 2 steht; R^{b} kann auch gemeinsam mit der an das benachbarte C-Atom gebundene Gruppe R^{b} einen fünf- oder sechsgliedrigen gesättigten, teilweise oder vollständig ungesättigten Ring bilden, der neben Kohlenstoff- 1, 2 oder 3 Heteroatome ausgewählt aus O, N und S enthalten kann;
X O, S oder N-R³;
R³ Wasserstoff, C₁-C₆-Alkyl, C₁-C₄-Haloalkyl, C₂-C₆-Alkenyl, C₃-C₆-Alkinyl, Z-C₃-C₁₀-Cycloalkyl, C₁-C₆-Alkoxy-C₁-C₆-alkyl, C₁-C₆-Cyanoalkyl, Z-Phenyl, Z-C(=O)-R^{a2} und Tri-C₁-C₄-alkylsilyl;
R^{a2} C₁-C₆-Alkyl, C₁-C₄-Haloalkyl, Z-C₁-C₆-Alkoxy, Z-C₁-C₄-Haloalkoxy und NRⁱRⁱⁱ;
Y O oder S;
A,E,G,M N und C-R^{c}, wobei eine Gruppe davon N bedeutet,
R^{c} Wasserstoff oder eine der bei R^{b} genannten Gruppen;
wobei in Gruppen R^{A}, R³ und deren Untersubstituenten die Kohlenstoffketten und/oder die cyclischen Gruppen teilweise oder vollständig durch Gruppen R^{b} substituiert sein können,
sowie deren N-Oxide und landwirtschaftlich geeignete Salze.

Außerdem betrifft die Erfindung Verfahren und Zwischenprodukte zur Herstellung der Pyridine der Formel I und deren N-Oxide, deren landwirtschaftlich brauchbaren Salze, sowie sie enthaltende Wirkstoffkombinationen, sie enthaltende Mittel und deren Verwendung als Herbizide, d.h. zur Bekämpfung von Schadpflanzen, sowie ein Verfahren zur Bekämpfung von unerwünschtem Pflanzenwuchs, bei dem man eine herbizid wirksame Menge mindestens einer Pyridinverbindung der Formel I oder eines landwirtschaftlich brauchbaren Salzes von I auf Pflanzen, deren Samen und/oder deren Lebensraum einwirken läßt.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemä-βen Gegenstandes nicht nur in der jeweils angegebenen Kombination, sondern auch in anderen Kombinationen verwendbar sind, ohne den Rahmen der Erfindung zu verlassen.

In WO 2008/009908 und WO 2008/071918 werden herbizide Pyridopyrazine beschrieben, ihre herbizide Wirkung bei niedrigen Aufwandmengen, bzw. Verträglichkeit gegenüber Kulturpflanzen bleibt jedoch verbesserungsbedürftig.

Eine Aufgabe der vorliegenden Erfindung ist die Bereitstellung von Verbindungen mit herbizider Wirkung. Insbesondere sollen Wirkstoffe zur Verfügung gestellt werden, die eine hohe herbizide Wirkung, insbesondere bereits bei niedrigen Aufwandmengen, aufweisen und deren Verträglichkeit gegenüber Kulturpflanzen für eine kommerzielle Verwertung hinreichend ist.

Diese und weitere Aufgaben werden durch die eingangs definierten Verbindungen der Formel I und durch ihre N-Oxide, sowie deren landwirtschaftlich geeigneten Salze gelöst.

Die erfindungsgemäßen Verbindungen können analog der in WO 2008/009908 und WO 2008/071918 beschriebenen Syntheserouten nach Standardverfahren der organischen Chemie hergestellt werden, beispielsweise nach der folgenden Syntheseroute:

Pyridincarbonsäuren der Formel II können mit Carbonylverbindungen der Formel III zu Verbindungen der Formel IV umgesetzt werden. In Formeln II und III haben die Variablen die für Formel I angegebenen Bedeutung. Die Gruppe Hal steht für ein Halogenatom oder eine andere geeignete nucleophile Abgangsgruppe, wie Alkoxy oder Phenoxy.

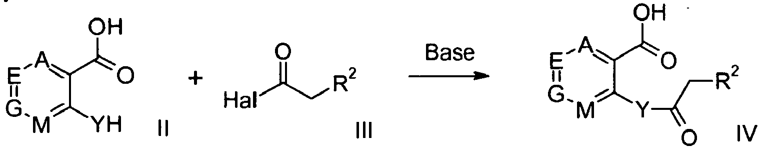

Diese Umsetzung erfolgt üblicherweise bei Temperaturen von -78 °C bis 120°C, vorzugsweise -20°C bis 50°C, in einem inerten organischen Lösungsmittel in Gegenwart einer Base wie z. B. Triethylamin (vgl. J. Agric. and Food Chem. 1994, 42(4), 1019 - 1025.), eines Katalysators wie z. B. Dicyclohexylcarbodiimid (vgl. Egyptian Journal of Chemistry 1994, 37(3), 273-282.) oder anderer bekannter Kupplungsreagenzien.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid, Dimethylformamid und Dimethylacetamid, besonders bevorzugt halogenierte Kohlenwasserstoffe wie Methylenchlorid, Chloroform und Chlorbenzol. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat, Kaliumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tributylamin, Di-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden tertiäre Amine wie Trimethylamin, Triethylamin, Tributylamin, Di-isopropylethylamin. Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Die Verbindungen der Formel IV werden durch Einführung einer Abgangsgruppe L¹ aktiviert. Als Abgangsgruppen L¹ kommen allgemein solche Gruppen in Frage, die die Elektrophilie der Carbonylgruppe erhöhen, beispielsweise O-Alkyl, O-Aryl, Halogenide, aktivierte Ester oder Aldehyde (wie z. B. Weinreb-Amid), insbesondere Pentafluorphenoxy.

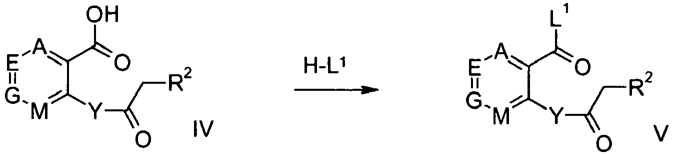

Diese Umsetzung erfolgt üblicherweise bei Temperaturen von -78 °C bis 120°C, vorzugsweise -20°C bis 50°C, in einem inerten organischen Lösungsmittel in Gegenwart einer Base wie z. B. Triethylamin (vgl. J. Agric. and Food Chem. 1994, 42(4), 1019 - 1025.),eines Katalysators wie z. B. Dicyclohexylcarbodiimid (vgl. Egyptian Journal of Chemistry 1994, 37(3), 273-282) oder anderer bekannter Kupplungsreagenzien.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid, Dimethylformamid und Dimethylacetamid, besonders bevorzugt Methylenchlorid und Toluol. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat, Kaliumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tributylamin, Di-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Alkalimetall- und Erdalkalimetallcarbonate wie Lithiumcarbonat, Kaliumcarbonat, Calziumcarbonat, Cäsiumcarbonat und Rubidiumcarbonat. Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt.

Als Agenz H-L¹ kommen Alkohole, ggf. subst. Phenole, N,O-Dialkylhydroxylamin, insbesondere Pentafluorphenol oder N,O-Dimethylhydroxylamin in Frage.

Die Verbindungen der Formel V werden zu den Verbindungen der Formel I cyclisiert.

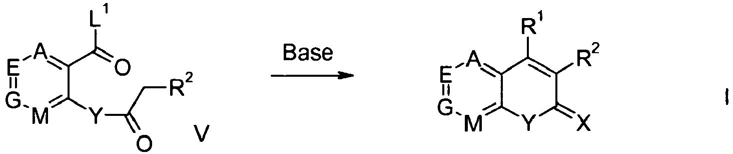

Diese Umsetzung erfolgt üblicherweise bei Temperaturen von -78°C bis 120°C, vorzugsweise -20°C bis 50°C, in einem inerten organischen Lösungsmittel in Gegenwart einer Base, bzw. einer Lewissäure oder eines Katalysators [vgl. Silverman, Richard B. J. Am. Chem. Soc. 1981, 103(13), 3910].

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid, Dimethylformamid und Dimethylacetamid, besonders bevorzugt Acetonitril und Dimethylformamid. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat, Kaliumcarbonat, Calziumcarbonat, Cäsiumcarbonat und Rubidiumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tributylamin, Di-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Alkalimetall- und Erdalkalimetallcarbonate wie Lithiumcarbonat, Kaliumcarbonat, Calziumcarbonat, Cäsiumcarbonat und Rubidiumcarbonat.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt.

Alternativ können die Verbindungen der Formel I auch über eine umgekehrte Reaktionsfolge erhalten werden, d. h. aus der Umsetzung der Verbindungen der Formel II mit Verbindungen H-L¹ werden die aktivierten Derivate der Formel VI erhalten.

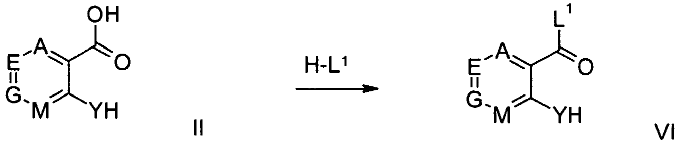

Diese Umsetzung erfolgt an sich unter den für die Umsetzung der Formel IV mit H-L¹ genannten Bedingungen.

Die Verbindungen der Formel VI können anschließend mit Verbindungen III zu den Derivaten der Formel V umgesetzt werden.

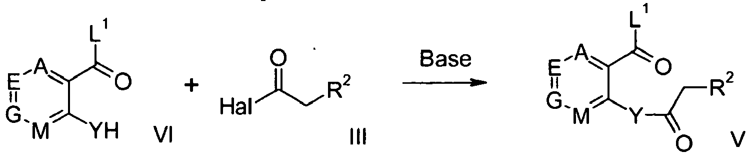

Diese Umsetzung erfolgt an sich unter den für die Umsetzung der Formel II mit III genannten Bedingungen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Sofern einzelne Verbindungen I nicht auf den voranstehend beschriebenen Wegen zugänglich sind, können sie durch Derivatisierung anderer Verbindungen I hergestellt werden.

Sofern bei der Synthese Isomerengemische anfallen, ist im allgemeinen jedoch eine Trennung nicht unbedingt erforderlich, da sich die einzelnen isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder in der zu bekämpfenden Schadpflanze erfolgen.

Die für die Substituenten der erfindungsgemäßen Verbindungen genannten organischen Molekülteile stellen Sammelbegriffe für individuelle Aufzählungen der einzelnen Gruppenmitglieder dar. Sämtliche Kohlenwasserstoffketten, wie Alkyl, Halo(gen)alkyl, Alkenyl, Alkinyl, sowie die Alkylteile und Alkenylteile in Alkoxy, Halo(gen)alkoxy, Alkylamino, Dialkylamino, N-Alkylsulfonylamino, Alkenyloxy, Alkinyloxy, Alkoxyamino, Alkylaminosulfonylamino, Dialkylaminosulfonylamino, Alkenylamino, Alkinylamino, N-(Alkenyl)-N-(alkyl)-amino, N-(Alkinyl)-N-(alkyl)-amino, N-(Alkoxy)-N-(alkyl)-amino, N-(Alkenyl)-N-(alkoxy)-amino oder N-(Alkinyl)-N-(alkoxy)-amino können geradkettig oder verzweigt sein.

Das Präfix Cₙ-Cₘ- gibt die jeweilige Kohlenstoffzahl der Kohlenwasserstoffeinheit an. Sofern nicht anders angegeben tragen halogenierte Substituenten vorzugsweise ein bis fünf gleiche oder verschiedene Halogenatome, insbesondere Fluoratome oder Chloratome.

Die Bedeutung Halogen steht jeweils für Fluor, Chlor, Brom oder Iod.

### Ferner bedeuten beispielsweise:

Alkyl sowie die Alkylteile beispielsweise in Alkoxy, Alkylamino, Dialkylamino, N-Alkylsulfonylamino, Alkylaminosulfonylamino, Dialkylaminosulfonylamino, N-(Alkenyl)-N-(alkyl)-amino, N-(Alkinyl)-N-(alkyl)-amino, N-(Alkoxy)-N-(alkyl)-amino: gesättigte, geradkettige oder verzweigte Kohlenwasserstoffreste mit einem oder mehr C-Atomen, z.B. 1 bis 2, 1 bis 4, oder 1 bis 6 Kohlenstoffatomen, z.B. C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl. In einer erfindungsgemäßen Ausführungsform steht Alkyl für kleine Alkylgruppen wie C₁-C₄-Alkyl. In einer anderen erfindungsgemäßen Ausführungsform steht Alkyl für größere Alkylgruppen wie C₅-C₆-Alkyl.

Halogenalkyl (auch als Haloalkyl bezeichnet): einen Alkylrest wie vorstehend genannt, dessen Wasserstoffatome partiell oder vollständig durch Halogenatome wie Fluor, Chlor, Brom und/oder Iod substituiert sind, z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-lodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl.

Cycloalkyl sowie die Cycloalkylteile beispielsweise in Cycloalkoxy oder Cycloalkylcarbonyl: monocyclische, gesättigte Kohlenwasserstoffgruppen mit drei oder mehr C-Atomen, z.B. 3 bis 6 Kohlenstoffringgliedern, wie Cyclopropyl, Cyclobutyl, Cyclopentyl und Cyclohexyl.

Alkenyl sowie Alkenylteile beispielsweise in Alkenylamino, Alkenyloxy, N-(Alkenyl)-N-(alkyl)-amino, N-(Alkenyl)-N-(alkoxy)-amino: einfach ungesättigte, geradkettige oder verzweigte Kohlenwasserstoffreste mit zwei oder mehr C-Atomen, z. B. 2 bis 4, 2 bis 6 oder 3 bis 6 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl.

Cycloalkenyl: monocyclische, einfach ungesättigte Kohlenwasserstoffgruppen mit 3 bis 6, vorzugsweise 5 bis 6 Kohlenstoffringgliedern, wie Cyclopenten-1-yl, Cyclopenten-3-yl, Cyclohexen-1-yl, Cyclohexen-3-yl, Cyclohexen-4-yl.

Alkinyl sowie Alkinylteile beispielsweise in Alkinyloxy, Alkinylamino, N-(Alkinyl)-N-(alkyl)-amino oder N-(Alkinyl)-N-(alkoxy)-amino: geradkettige oder verzweigte Kohlenwasserstoffgruppen mit zwei oder mehr C-Atomen, z. B. 2 bis 4, 2 bis 6, oder 3 bis 6 Kohlenstoffatomen und einer Dreifachbindung in beliebiger Position, z. B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl, 1-Ethyl-1-methyl-2-propinyl.

Alkoxy: Alkyl, wie vorstehend definiert, das über ein O-Atom gebunden ist: z. B. Methoxy, Ethoxy, n-Propoxy, 1-Methylethoxy, Butoxy, 1-Methylpropoxy, 2-Methylpropoxy oder 1,1-Dimethylethoxy, Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy.

3- bis 7-gliedriger monocyclischer oder 9- oder 10-gliedriger bicyclischer gesättigter, ungesättigter oder aromatischer Heterocyclus, enthaltend 1, 2, 3 oder 4 Heteroatome ausgewählt aus O, N und S kann über über C oder N gebunden sein. Bevorzugt sind davon 5- oder 6-gliedrige Heterocyclen.

Über N gebundene gesättigte oder ungesättigte heterocyclische Gruppen, wie: Pyridazin-3-yl, Pyridazin-4-yl, Pyrimidin-2-yl, Pyrimidin-4-yl, Pyrimidin-5-yl, Pyrazin-2-yl, 2-Furyl, 3-Furyl, 2-Thienyl, 3-Thienyl, Pyrazol-1-yl, Pyrazol-3-yl, Pyrazol-4-yl, Isoxazol-3-yl, Isoxazol-4-yl, Isoxazol-5-yl, Isothiazol-3-yl, Isothiazol-4-yl, Isothiazol-5-yl, Imidazol-1-yl, Imidazol-2-yl, Imidazol-4-yl, Oxazol-2-yl, Oxazol-4-yl, Oxazol-5-yl, Thiazol-2-yl, Thiazol-4-yl und Thiazol-5-yl.

Über C-gebundene heteroaromatische Gruppen, wie: Pyrazol-3-yl, Imidazol-5-yl, Oxazol-2-yl, Thiazol-2-yl, Thiazol-4-yl, Thiazol-5-yl, Pyridin-2-yl, Pyridin-3-yl, Pyridin-4-yl, Pyrimidin-2-yl, Pyrimidin-4-yl, Pyrimidin-5-yl, Pyridazin-4-yl, Pyrazin-2-yl, [1 H]-Tetrazol-5-yl und [2H]-Tetrazol-5-yl.

Die Verbindungen der Formel I können, je nach Substitutionsmuster, ein oder mehrere weitere Chiralitätszentren enthalten. Die erfindungsgemäßen Verbindungen können daher als reine Enantiomere oder Diastereomere oder als Enantiomeren- oder Diastereomerengemische vorliegen. Gegenstand der Erfindung sind sowohl die reinen Enantiomeren oder Diastereomeren als auch deren Gemische.

Die Verbindungen der Formel I können auch in Form der N-Oxide und/oder ihrer landwirtschaftlich brauchbaren Salze vorliegen, wobei es auf die Art des Salzes in der Regel nicht ankommt. Im Allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium oder Kalium, der Erdalkalimetalle, vorzugsweise Calcium oder Magnesium, und der Übergangsmetalle, vorzugsweise Mangan, Kupfer, Zink oder Eisen in Betracht. Ebenso kann als Kation Ammonium verwendet werden, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium. Als Ammoniumkation kommt auch das durch Alkylierung oder Arylierung quaternisierte Pyridin-Stickstoffatom der Formel I in Frage. Des Weiteren kommen Phosphoniumionen, Sulfoniumionen, vorzugsweise Tri(C₁-C₄-alkyl)sulfonium oder Sulfoxoniumionen, vorzugsweise Tri(C₁-C₄-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat, Butyrat oder Trifluoracetat.

Die besonders bevorzugten Ausführungsformen der Zwischenprodukte in Bezug auf die Variablen entsprechen denen der Gruppen der Formel I.

In einer besonderen Ausführungsform haben die Variablen der Verbindungen der Formel I folgende Bedeutungen, wobei diese sowohl für sich allein betrachtet als auch in Kombination miteinander besondere Ausgestaltungen der Verbindungen der Formel I darstellen:
In einer bevorzugten Ausführungsform der Verbindungen der Formel I steht A für N und E, G und M stehen für C-R^{c}. Diese Verbindungen entsprechen der Formel I.1, 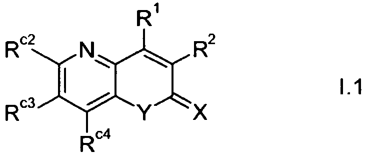 worin die Gruppen R^{c2}, R^{c3} und R^{c4} jeweils einer Gruppe R^{c} entsprechen und bevorzugt folgende Bedeutungen haben:
   R^{c2} H, OH, CN, Halogen, Alkyl, Alkoxy, Haloalkyl, insbesondere H, Br, OH und OCH₃;
   R^{c3} H, OH, CN, Halogen, Alkyl, Alkoxy, Haloalkyl, insbesondere H;
   R^{c4} H, OH, CN, Halogen, Alkyl, Alkoxy, Haloalkyl, insbesondere H.

In besonders bevorzugten Ausführungsformen der Verbindungen der Formel I und insbesondere solcher der Formel I.1 ist R¹ ausgewählt aus OH, OCH₃, OC(O)CH₃, OC(O)CH₂CH₃, OC(O)CH(CH₃)₂, OC(O)C(CH₃)₃, OC(O)-c-C₃H₅, OC(O)-C₆H₅, OC(O)-CH₂C₆H₅, OC(O)CH₂Cl, OC(O)-CF₃, OC(O)-CH₂OCH₃, OC(O)-N(CH₃)₂ und OC(O)-OCH₂CH₃.

In besonders bevorzugten Ausführungsformen der Verbindungen der Formel I und insbesondere solcher der Formel I.1 bedeutet R² Phenyl, welches substituiert ist durch eine Gruppe ausgewählt aus 2-Br, 2-Cl, 2,4-Cl₂, 2-Cl-4-F, 2-Cl-5-F, 2-Cl-6-F, 2-CI-4-CF₃, 2-Cl-5-CF₃, 2-Cl-6-CF₃, 2-Cl-3,6-F₂, 2-F, 2,4-F₂, 2,5-F₂, 2,6-F₂, 2-F-4-CF₃, 2-F-5-CF₃, 2-F-6-CF₃, 2,3,6-F₃, 2-NO₂, 2-NO₂-4-F, 2-NO₂-5-F, 2-NO₂-6-F, 2-NO₂-4-CF₃, 2-NO₂-5-CF₃, 2-NO₂-6-CF₃, 2-NO₂-3,6-F₂, 2-CN, 2-CH₃, 2-CH₃-4-F, 2-CH₃-5-F, 2-CH₃-6-F, 2-CH₃-4-CF₃, 2-CH₃-5-CF₃, 2-CH₃-6-CF₃, 2-CH₃-3,6-F₂, 2-OCH₃, 2-OCH₃-4-F, 2-OCH₃-5-F, 2-OCH₃-6-F, 2-OCH₃-4-CF₃, 2-OCH₃-5-CF₃, 2-OCH₃-6-CF₃, 2-OCH₃-3,6-F₂, 2-CHF₂, 2-CHF₂-4-F, 2-CHF₂-5-F, 2-CHF₂-6-F, 2-CHF₂-4-CF₃, 2-CHF₂-5-CF₃, 2-CHF₂-6-CF₃, 2-CHF₂-3,6-F₂, 2-CF₃, 2-CF₃-4-F, 2-CF₃-5-F, 2-CF₃-6-F, 2-CF₃-4-CF₃, 2-CF₃-5-CF₃, 2-CF₃-6-CF₃, 2-CF₃-3,6-F₂, 2-OCHF₂, 2-OCHF₂-4-F, 2-OCHF₂-5-F, 2-OCHF₂-6-F, 2-OCHF₂-4-CF₃, 2-OCHF₂-5-CF₃, 2-OCHF₂-6-CF₃, 2-OCHF₂-3,6-F₂, 2-OCF₃, 2-OCF₃-4-F, 2-OCF₃-5-F, 2-OCF₃-6-F, 2-OCF₃-4-CF₃, 2-OCF₃-5-CF₃, 2-OCF₃-6-CF₃ oder 2-OCF₃-3,6-F₂.

In besonders bevorzugten Ausführungsformen der Verbindungen der Formel I und insbesondere solcher der Formel I.1 ist X ausgewählt aus Sauerstoff und Schwefel.

In einer weiteren Ausführungsform der Verbindungen der Formel I steht A, G und M für C-R^{c} und E für N. Diese Verbindungen entsprechen der Formel I.2, 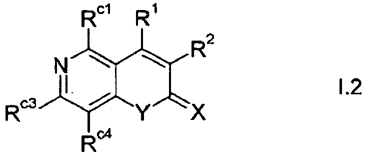 worin die Gruppen R^{c1}, R^{c3} und R^{c4} jeweils einer Gruppe R^{c} entsprechen. In einer weiteren Ausführungsform der Verbindungen der Formel I steht A, E und M für C-R^{c} und G für N. Diese Verbindungen entsprechen der Formel I.3, 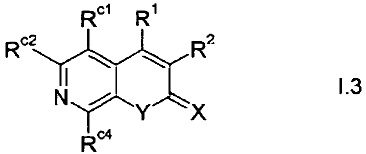 worin die Gruppen R^{c1}, R^{c2} und R^{c4} jeweils einer Gruppe R^{c} entsprechen und bevorzugt folgende Bedeutungen haben:
R^{c1} H, OH, CN, Halogen, Alkyl, Alkoxy, Haloalkyl.
R^{c2} H, OH, CN, Halogen, Alkyl, Alkoxy, Haloalkyl, insbesondere H, Br, OH und OCH₃;
R^{c4} H, OH, CN, Halogen, Alkyl, Alkoxy, Haloalkyl, insbesondere H, Br, OH und OCH₃;

In einer weiteren Ausführungsform der Verbindungen der Formel I steht A, E und G für C-R^{c} und M für N. Diese Verbindungen entsprechen der Formel I.4, 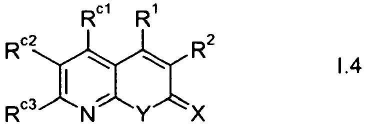 worin die Gruppen R^{c1}, R^{c2} und R^{c3} jeweils einer Gruppe R^{c} entsprechen und bevorzugt folgende Bedeutungen haben:
R^{c1} H, OH, CN, Halogen, Alkyl, Alkoxy, Haloalkyl, insbesondere H;
R^{c2} H, OH, CN, Halogen, Alkyl, Alkoxy, Haloalkyl, insbesondere H;
R^{c3} H, OH, CN, Halogen, Alkyl, Alkoxy, Haloalkyl, insbesondere H, Br, OH und OCH₃;
Besonders bevorzugte Ausgestaltungen der Verbindungen der Formel I betreffen solche jeder der Formeln I.1 bis 1.4, in denen die Variablen die für Formel bevorzugten Bedeutungen haben.

In einer ersten bevorzugten Ausführungsform der Erfindung steht R¹ für O-R^{A}.

In einer weiteren bevorzugten Ausführungsform der Erfindung steht R¹ für S(O)ₙ-R^{A}, darin steht n bevorzugt für 0 oder 2, insbesondere für 2.

In einer weiteren bevorzugten Ausführungsform steht R¹ für O-S(O)ₙ-R^{A}, darin steht n bevorzugt für 0 oder 2, insbesondere für 2, wie beispielsweise OS(O)₂-CH₃, OS(O)₂-C₂H₅, OS(O)₂-C₃H₇, OS(O)₂-C₆H₅ oder OS(O)₂-(4-CH₃-C₆H₄).

In einer weiteren bevorzugten Ausführungsform steht R¹ für O-S(O)ₙ-NRⁱRⁱⁱ, insbesondere mit den nachfolgend bevorzugt genannten Gruppen NRⁱRⁱⁱ.

R^{A} steht insbesondere für H, C₁-C₆-Alkylcarbonyl, wie C(O)CH₃, C(O)CH₂CH₃, C(O)CH(CH₃)₂ oder C(O)C(CH₃)₃; C₁-C₆-Cycloalkylcarbonyl, wie Cyclopropylcarbonyl, Cyclopentylcarbonyl oder Cyclohexylcarbonyl; C₂-C₆-Alkenylcarbonyl, wie C(O)CH=CH₂ oder C(O)CH₂CH=CH₂, ggf. subst. Benzoyl, wie C(O)C₆H₅, C(O)[2-CH₃-C₆H₄], C(O)[4-CH₃-C₆H₄], C(O)[2-F-C₆H₄], C(O)[4-F-C₆H₄], oder ggf. subst. Heteroaryl, wie Pyridin, welches über eine Carbonylgruppe gebunden ist. Besonders bevorzugt steht R^{A} für H oder C₁-C₆-Alkylcarbonyl.

Weiter besonders bevorzugt ist R^{A} ausgewählt aus der Gruppe H, OCH₃, C(O)CH₃, C(O)CH₂CH₃, C(O)CH(CH₃)₂, C(O)C(CH₃)₃, C(O)-c-C₃H₅, C(O)-C₆H₅, C(O)-CH₂C₆H₅, C(O)CH₂Cl, C(O)CF₃, C(O)CH₂OCH₃, C(O)N(CH₃)₂ und C(O)OCH₂CH₃.

In einer weiteren bevorzugten Ausführungsform der Erfindung steht R^{A} für NRⁱRⁱⁱ.

In einer weiteren bevorzugten Ausführungsform der Erfindung steht R^{A} für Z-NRⁱ-C(O)-NRⁱRⁱⁱ, wobei Rⁱ und Rⁱⁱ wie eingangs und bevorzugt wie nachstehend definiert ist. In weiteren Ausführungsformen kommen für Rⁱ und Rⁱⁱ unabhängig voneinander auch C₁-C₄-Alkoxy, C₁-C₄-Haloalkoxy und C₁-C₄-Alkoxy-C₁-C₄-alkyl in Frage, insbesondere OCH₃, OC₂H₅, CH₂CH₂OCH₃und CH₂CH₂Cl.

Rⁱ und Rⁱⁱ stehen vorzugsweise für C₁-C₈-Alkyl, C₁-C₄-Haloalkyl, Z-C₃-C₆-Cycloalkyl, Z-C₁-C₈-Alkoxy, Z-C₁-C₈-Haloalkoxy, Z-Phenyl, Z-C(=O)-R^{a} oder Z-Hetaryl. Bevorzugt sind dabei CH₃, C₂H₅, n-Propyl, CH(CH₃)₂, Butyl, 2-Chorethyl, Cyclopentyl, Cyclohexyl, 2-Ethoxymethyl, 2-Chlorethoxy, Phenyl, Pyrimidine oder Triazine, welche Ringe unsubstituiert oder substituiert sind. Bevorzugte Substituenten sind dabei C₁-C₄-Alkylcarbonyl oder C₁-C₄-Haloalkylcarbonyl, insbesondere C(=O)-CH₃, C(=O)-C₂H₅, C(=O)-C₃H₇, C(=O)-CH(CH₃)₂, Butylcarbonyl und C(=O)-CH₂Cl. Besonders bevorzugte Ausgestaltungen der Gruppe NRⁱRⁱⁱ sind N(Di-C₁-C₄-alkyl), insbesondere N(CH₃)-C₁-C₄-alkyl, wie N(CH₃)₂, N(CH₃)CH₂CH₃, N(CH₃)C₃H₇ und N(CH₃)CH(CH₃)₂.

Weitere besonders bevorzugte Ausgestaltungen für NRⁱRⁱⁱ sind NH-Aryl, wobei Aryl bevorzugt für Phenyl steht, welches - insbesondere in der 2- und 6-Position - substituiert ist durch eine bis drei gleiche oder verschiedene Gruppen Halogen, CH₃, Halogen-C₁-C₂-alkyl, Halogen-C₁-C₂-alkoxy und Carboxyl, wie 2-Cl,6-COOH-C₆H₃, 2,6-Cl₂-C₆H₃, 2,6-F₂-C₆H₃, 2,6-Cl₂ 3-C₆H₂, 2-CF₃,6-CH₂CHF₂-C₆H₃, 2-CF₃,6-OCF₃-C₆H₃ und 2-CF₃,6-CH₂CHF₂-C₆H₃.

Weitere Ausgestaltungen für NRⁱRⁱⁱ sind NH-Heteroaryl, wobei Heteroaryl bevorzugt für eine der nachstehenden bevorzugten heteroaromatischen Gruppen steht, insbesondere für Triazinyl, Pyrimidinyl oder Triazolopyrimidinyl, wie [1,2,4]triazolo[1,5-a]pyrimidin-2-yl, steht, welche Gruppen substituiert sein können, insbesondere durch C₁-C₄-Alkoxy und/oder Halogen. Besonders bevorzugt sind 5,7-Dimethoxy-[1,2,4]triazolo[1,5-a]pyrimidin-2-yl, 5,7-Diethoxy-[1,2,4]triazolo[1,5-a]pyrimidin-2-yl, 5-Fluor-7-methoxy-[1,2,4]triazolo[1,5-a]pyrimidin-2-yl und 5-Fluor-7-ethoxy-[1,2,4]triazolo[1,5-a]pyrimidin-2-yl.

In einer weiteren bevorzugten Ausführungsform der Erfindung steht R^{A} für einen ggf. durch R^{b} substituierten 5- oder 6-gliedrigen Heterocyclus wie zuvor definiert, der vorzugsweise entweder 1, 2, 3 oder 4 Stickstoffatome oder 1 Sauerstoff oder 1 Schwefelatom und gegebenenfalls 1 oder 2 Stickstoffatome als Ringlieder aufweist und die unsubstituiert ist oder 1 oder 2 aus R^{b} ausgewählte Substituenten aufweisen kann. Bevorzugt sind über N gebundene gesättigte oder ungesättigte Gruppen, wie z.B.: Heteroaromatische Gruppen: Pyridazin-3-yl, Pyridazin-4-yl, Pyrimidin-2-yl, Pyrimidin-4-yl, Pyrimidin-5-yl, Pyrazin-2-yl, 2-Furyl, 3-Furyl, 2-Thienyl, 3-Thienyl, Pyrazol-1-yl, Pyrazol-3-yl, Pyrazol-4-yl, Isoxazol-3-yl, Isoxazol-4-yl, Isoxazol-5-yl, Isothiazol-3-yl, Isothiazol-4-yl, Isothiazol-5-yl, Imidazol-1-yl, Imidazol-2-yl, Imidazol-4-yl, Oxazol-2-yl, Oxazol-4-yl, Oxazol-5-yl, Thiazol-2-yl, Thiazol-4-yl und Thiazol-5-yl.

In einer anderen Ausgestaltung steht R^{A} für eine über C-gebundene heteroaromatische Gruppe wie Pyrazol-3-yl, Imidazol-5-yl, Oxazol-2-yl, Thiazol-2-yl, Thiazol-4-yl, Thiazol-5-yl, Pyridin-2-yl, Pyridin-3-yl, Pyridin-4-yl, Pyrimidin-2-yl, Pyrimidin-4-yl, Pyrimidin-5-yl, Pyridazin-4-yl, Pyrazin-2-yl, [1 H]-Tetrazol-5-yl und [2H]-Tetrazol-5-yl, wobei die hier exemplarisch genannten Heterocyclen 1 oder 2 aus R^{b} ausgewählte Substituenten aufweisen können. Bevorzugte Gruppen R^{b} sind insbesondere F, Cl, CN, NO₂, CH₃, C₂H₅, OCH₃, OC₂H₅, OCHF₂, OCF₃ und CF₃.

In einer weiteren bevorzugten Ausgestaltung steht R² für Phenyl, welches unsubstituiert ist oder teilweise oder vollständig durch Gruppen R^{b} substituiert ist. Besonders bevorzugt sind solche Verbindungen, in denen eine Gruppe R^{b} in ortho-Position steht. Solche Verbindungen der Formel I werden durch die Formel I.A beschrieben:

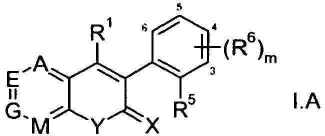

In Formel I.A steht der Index m für Null oder eine ganze Zahl von eins bis vier, bevorzugt für 0, 1 oder 2, insbesondere for 0 oder 1. R⁵ und R⁶ stehen für Gruppen R^{b}, wie eingangs definiert, bevorzugt für Halogen, NO₂, C₁-C₄-Alkyl, C₁-C₂-Halogenalkyl und C₁-C₄-Alkoxy. Eine Gruppe R⁶ steht bevorzugt in Position 5. Eine Gruppe R⁶ in der Position 3 stellt eine weitere bevorzugte Ausführungsform dar.

Besonders bevorzugt steht R⁵ für Br, F, NO₂, CN, CH₃, OCH₃, CHF₂ oder OCHF₂. R⁶ steht besonders bevorzugt für Halogen oder Halogenmethyl, wie Cl, F oder CF₃. Insbesondere bevorzugt ist (R⁶)ₘ ausgewählt aus 4-F, 5-F, 6-F, 4-CF₃, 5-CF₃ und 3,6-F₂.

In einer bevorzugten Ausführungsform steht X für O.

In einer weiteren Ausführungsform steht X für S.

In einer weiteren Ausführungsform steht X für NR³.

In einer bevorzugten Ausführungsform steht Y für O.

In einer weiteren Ausführungsform steht Y für S.

R³ steht bevorzugt für H, C₁-C₆-Alkyl, wie CH₃, C₂H₅, n-C₃H₇, CH(CH₃)₂, n-C₃H₉, oder C(CH₃)₃; C₃-C₆-Cycloalkyl-C₁-C₄-alkyl, wie Cyclopropylmethyl, C₃-C₆-Alkenyl, wie CH₂CH=CH₂, CH₂C(CH₃)=CH₂, CH₂CH₂CH=CH₂, CH₂CH₂C(CH₃)=CH₂, CH₂CH₂CH₂CH=CH₂, CH₂CH₂CH₂C(CH₃)=CH₂, oder ggf. subst. Phenyl, wie C₆H₅, 4-CH₃-C₆H₄, 4-F-C₆H₄ oder S(O)ₙ-R^{N}, worin R^{N} für C₁-C₆-Halogenalkyl steht, wie CH₂CF₃, CH₂CHF₂.

Eine weitere Ausführungsform betrifft die N-Oxide der Verbindungen der Formel I. Eine weitere Ausführungsform betrifft Salze der Verbindungen der Formel I, insbesondere solche erhältlich durch Quaternisierung des Pyridin-Stickstoffatoms, die bevorzugt erfolgen kann durch Alkylierung oder Arylierung der Verbindungen der Formel I. Entsprechend sind bevorzugte Salze der Verbindungen die N-Alkyl-, insbesondere die N-Methyl-, bzw. die N-Phenyl-Salze.

Insbesondere sind im Hinblick auf ihre Verwendung die in den folgenden Tabellen zusammengestellten Verbindungen der Formel I, welche der Formel I.1A entsprechen, bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

### Tabelle 1

Verbindungen der Formel I, in denen X und Y O bedeuten, der Index m in (R⁶)ₘ Null bedeutet und die Kombination von R¹ und R⁵ für eine Verbindung jeweils einer Zeile der Tabelle A entspricht

### Tabelle 2

Verbindungen der Formel I, in denen X und Y O bedeuten, (R⁶)ₘ für 4-Cl steht und die Kombination von R¹ und R⁵ für eine Verbindung jeweils einer Zeile der Tabelle A entspricht

### Tabelle 3

Verbindungen der Formel I, in denen X und Y O bedeuten, (R⁶)ₘ für 3-F steht und die Kombination von R¹ und R⁵ für eine Verbindung jeweils einer Zeile der Tabelle A entspricht

### Tabelle 4

Verbindungen der Formel I, in denen X und Y O bedeuten, (R⁶)ₘ für 4-F steht und die Kombination von R¹ und R⁵ für eine Verbindung jeweils einer Zeile der Tabelle A entspricht

### Tabelle 5

Verbindungen der Formel I, in denen X und Y O bedeuten, (R⁶)ₘ für 5-F steht und die Kombination von R¹ und R⁵ für eine Verbindung jeweils einer Zeile der Tabelle A entspricht

### Tabelle 6

Verbindungen der Formel I, in denen X und Y O bedeuten, (R⁶)ₘ für 6-F steht und die Kombination von R¹ und R⁵ für eine Verbindung jeweils einer Zeile der Tabelle A entspricht

### Tabelle 7

Verbindungen der Formel I, in denen X und Y O bedeuten, (R⁶)ₘ für 4-CF₃ steht und die Kombination von R¹ und R⁵ für eine Verbindung jeweils einer Zeile der Tabelle A entspricht

### Tabelle 8

Verbindungen der Formel I, in denen X und Y O bedeuten, (R⁶)ₘ für 5-CF₃ steht und die Kombination von R¹ und R⁵ für eine Verbindung jeweils einer Zeile der Tabelle A entspricht

### Tabelle 9

Verbindungen der Formel I, in denen X und Y O bedeuten, (R⁶)ₘ für 3,6-F₂ steht und die Kombination von R¹ und R⁵ für eine Verbindung jeweils einer Zeile der Tabelle A entspricht

### Tabelle 10

Verbindungen der Formel I, in denen X O und Y S bedeuten, der Index m in (R⁶)ₘ Null bedeutet und die Kombination von R¹ und R⁵ für eine Verbindung jeweils einer Zeile der Tabelle A entspricht

### Tabelle 11

Verbindungen der Formel I, in denen X O und Y S bedeuten, (R⁶)ₘ für 4-Cl steht und die Kombination von R¹ und R⁵ für eine Verbindung jeweils einer Zeile der Tabelle A entspricht

### Tabelle 12

Verbindungen der Formel I, in denen X O und Y S bedeuten, (R⁶)ₘ für 3-F steht und die Kombination von R¹ und R⁵ für eine Verbindung jeweils einer Zeile der Tabelle A entspricht

### Tabelle 13

Verbindungen der Formel I, in denen X O und Y S bedeuten, (R⁶)ₘ für 4-F steht und die Kombination von R¹ und R⁵ für eine Verbindung jeweils einer Zeile der Tabelle A entspricht

### Tabelle 14

Verbindungen der Formel I, in denen X O und Y S bedeuten, (R⁶)ₘ für 5-F steht und die Kombination von R¹ und R⁵ für eine Verbindung jeweils einer Zeile der Tabelle A entspricht

### Tabelle 15

Verbindungen der Formel I, in denen X O und Y S bedeuten, (R⁶)ₘ für 6-F steht und die Kombination von R¹ und R⁵ für eine Verbindung jeweils einer Zeile der Tabelle A entspricht

### Tabelle 16

Verbindungen der Formel I, in denen X O und Y S bedeuten, (R⁶)ₘ für 4-CF₃ steht und die Kombination von R¹ und R⁵ für eine Verbindung jeweils einer Zeile der Tabelle A entspricht

### Tabelle 17

Verbindungen der Formel I, in denen X O und Y S bedeuten, (R⁶)ₘ für 5-CF₃ steht und die Kombination von R¹ und R⁵ für eine Verbindung jeweils einer Zeile der Tabelle A entspricht

### Tabelle 18

Verbindungen der Formel I, in denen X O und Y S bedeuten, (R⁶)ₘ für 3,6-F₂ steht und die Kombination von R¹ und R⁵ für eine Verbindung jeweils einer Zeile der Tabelle A entspricht

### Tabelle 19

Verbindungen der Formel I, in denen X und Y S bedeuten, der Index m in (R⁶)ₘ Null bedeutet und die Kombination von R¹ und R⁵ für eine Verbindung jeweils einer Zeile der Tabelle A entspricht

### Tabelle 20

Verbindungen der Formel I, in denen X und Y S bedeuten, (R⁶)ₘ für 4-Cl steht und die Kombination von R¹ und R⁵ für eine Verbindung jeweils einer Zeile der Tabelle A entspricht

### Tabelle 21

Verbindungen der Formel I, in denen X und Y S bedeuten, (R⁶)ₘ für 3-F steht und die Kombination von R¹ und R⁵ für eine Verbindung jeweils einer Zeile der Tabelle A entspricht

### Tabelle 22

Verbindungen der Formel I, in denen X und Y S bedeuten, (R⁶)ₘ für 4-F steht und die Kombination von R¹ und R⁵ für eine Verbindung jeweils einer Zeile der Tabelle A entspricht

### Tabelle 23

Verbindungen der Formel I, in denen X und Y S bedeuten, (R⁶)ₘ für 5-F steht und die Kombination von R¹ und R⁵ für eine Verbindung jeweils einer Zeile der Tabelle A entspricht

### Tabelle 24

Verbindungen der Formel I, in denen X und Y S bedeuten, (R⁶)ₘ für 6-F steht und die Kombination von R¹ und R⁵ für eine Verbindung jeweils einer Zeile der Tabelle A entspricht

### Tabelle 25

Verbindungen der Formel I, in denen X und Y S bedeuten, (R⁶)ₘ für 4-CF₃ steht und die Kombination von R¹ und R⁵ für eine Verbindung jeweils einer Zeile der Tabelle A entspricht

### Tabelle 26

Verbindungen der Formel I, in denen X und Y S bedeuten, (R⁶)ₘ für 5-CF₃ steht und die Kombination von R¹ und R⁵ für eine Verbindung jeweils einer Zeile der Tabelle A entspricht

### Tabelle 27

Verbindungen der Formel I, in denen X und Y S bedeuten, (R⁶)ₘ für 3,6-F₂ steht und die Kombination von R¹ und R⁵ für eine Verbindung jeweils einer Zeile der Tabelle A entspricht

### Tabelle 28

Verbindungen der Formel I, in denen X S und Y O bedeuten, der Index m in (R⁶)ₘ Null bedeutet und die Kombination von R¹ und R⁵ für eine Verbindung jeweils einer Zeile der Tabelle A entspricht

### Tabelle 29

Verbindungen der Formel I, in denen X S und Y O bedeuten, (R⁶)ₘ für 4-Cl steht und die Kombination von R¹ und R⁵ für eine Verbindung jeweils einer Zeile der Tabelle A entspricht

### Tabelle 30

Verbindungen der Formel I, in denen X S und Y O bedeuten, (R⁶)ₘ für 3-F steht und die Kombination von R¹ und R⁵ für eine Verbindung jeweils einer Zeile der Tabelle A entspricht

### Tabelle 31

Verbindungen der Formel I, in denen X S und Y O bedeuten, (R⁶)ₘ für 4-F steht und die Kombination von R¹ und R⁵ für eine Verbindung jeweils einer Zeile der Tabelle A entspricht

### Tabelle 32

Verbindungen der Formel I, in denen X S und Y O bedeuten, (R⁶)ₘ für 5-F steht und die Kombination von R¹ und R⁵ für eine Verbindung jeweils einer Zeile der Tabelle A entspricht

### Tabelle 33

Verbindungen der Formel I, in denen X S und Y O bedeuten, (R⁶)ₘ für 6-F steht und die Kombination von R¹ und R⁵ für eine Verbindung jeweils einer Zeile der Tabelle A entspricht

### Tabelle 34

Verbindungen der Formel I, in denen X S und Y O bedeuten, (R⁶)ₘ für 4-CF₃ steht und die Kombination von R¹ und R⁵ für eine Verbindung jeweils einer Zeile der Tabelle A entspricht

### Tabelle 35

Verbindungen der Formel I, in denen X S und Y O bedeuten, (R⁶)ₘ für 5-CF₃ steht und die Kombination von R¹ und R⁵ für eine Verbindung jeweils einer Zeile der Tabelle A entspricht

### Tabelle 36

Verbindungen der Formel I, in denen X S und Y O bedeuten, (R⁶)ₘ für 3,6-F₂ steht und die Kombination von R¹ und R⁵ für eine Verbindung jeweils einer Zeile der Tabelle A entspricht

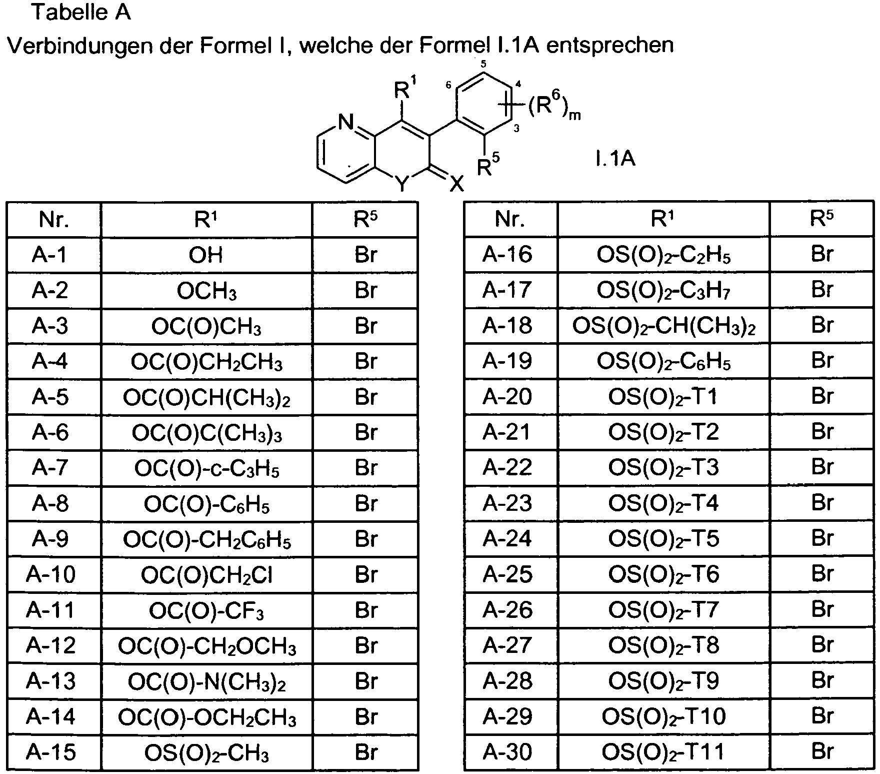

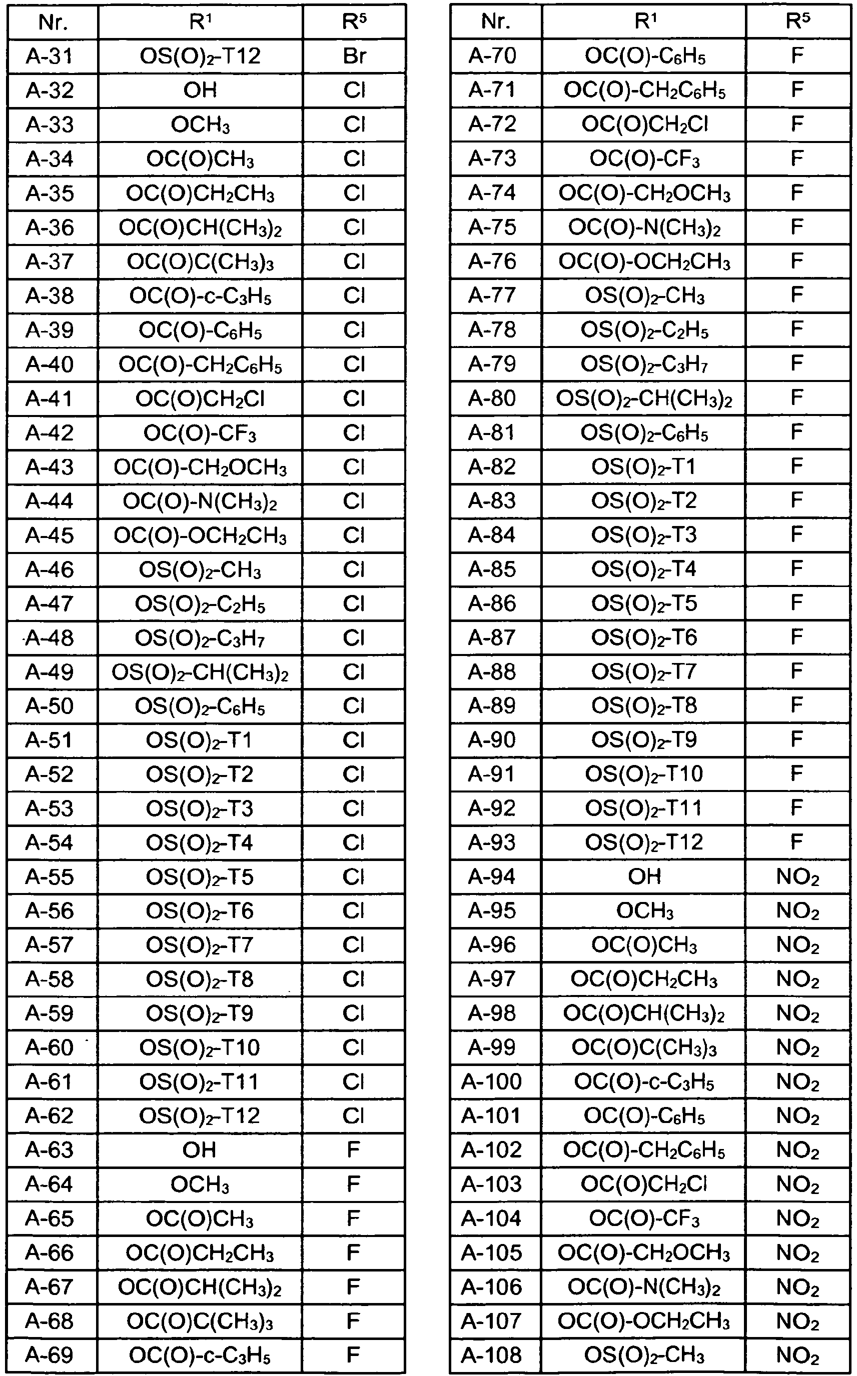

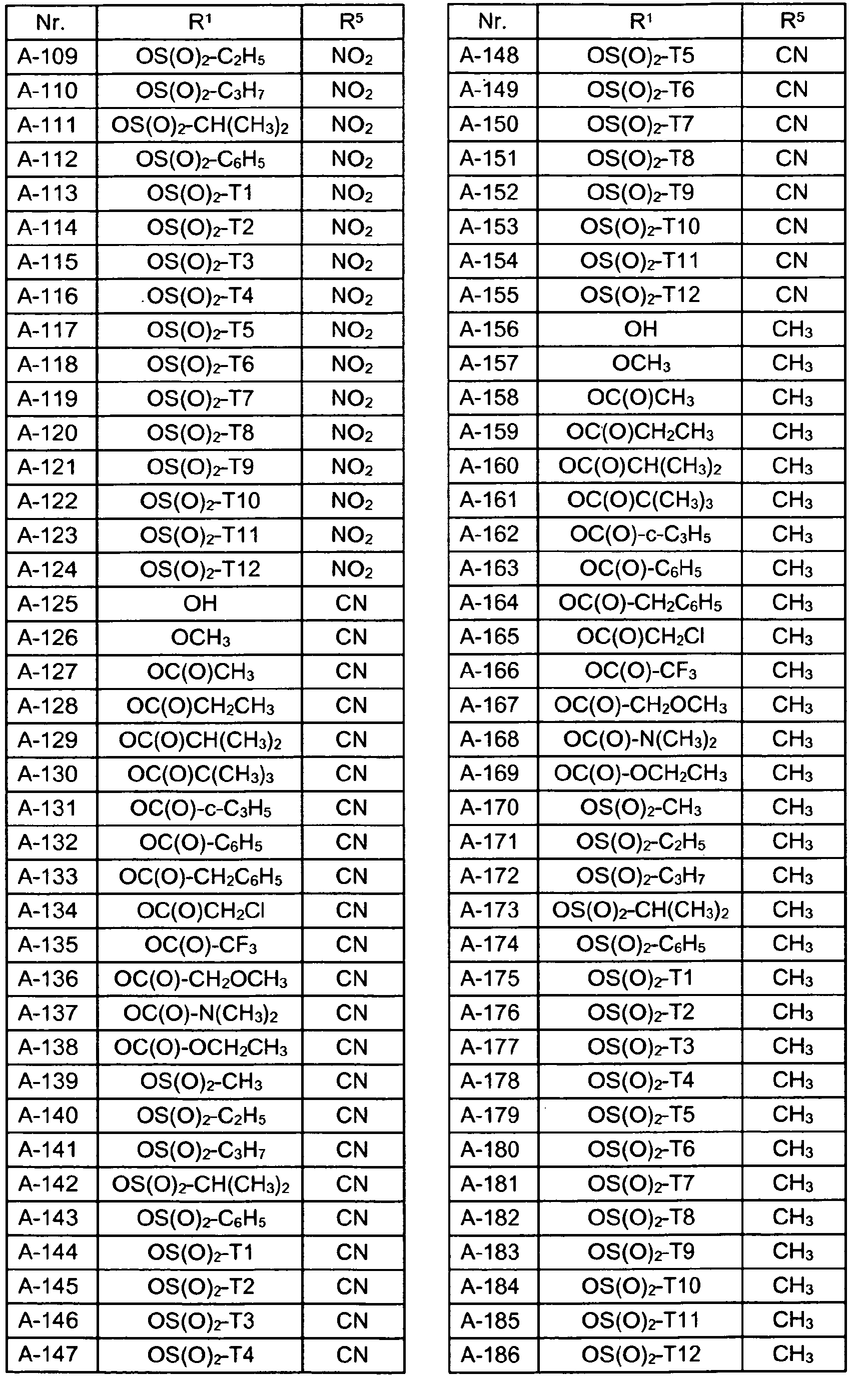

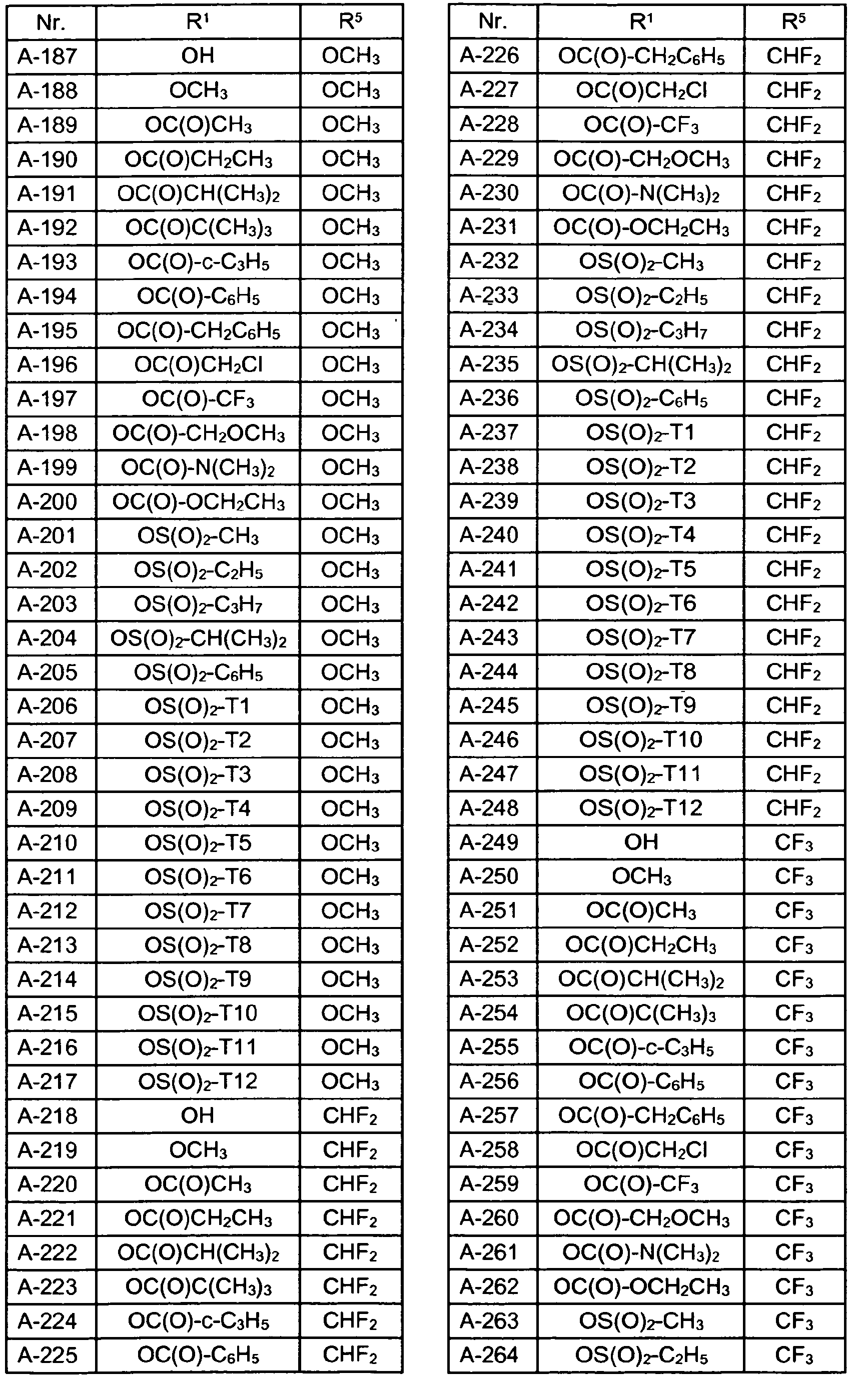

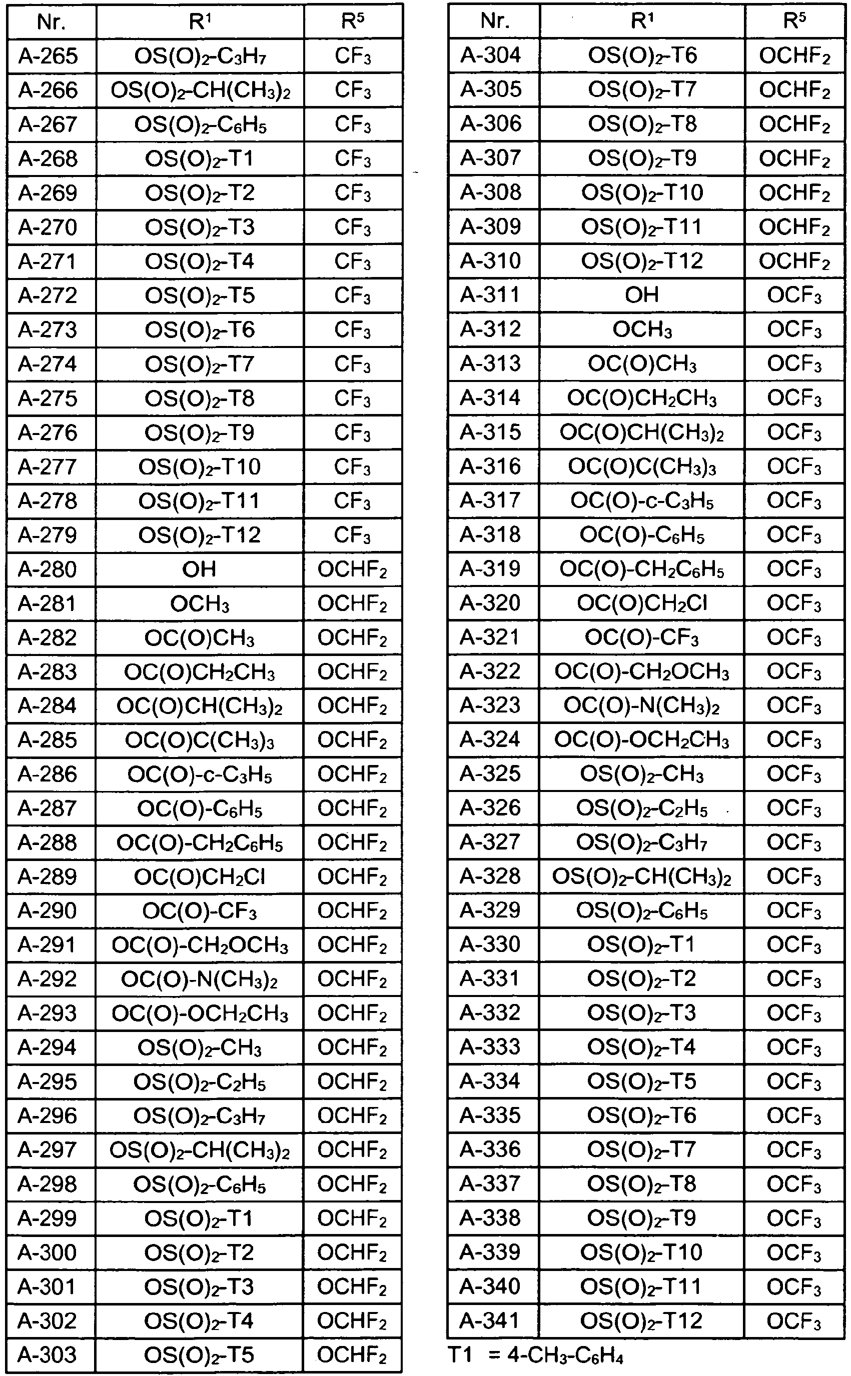

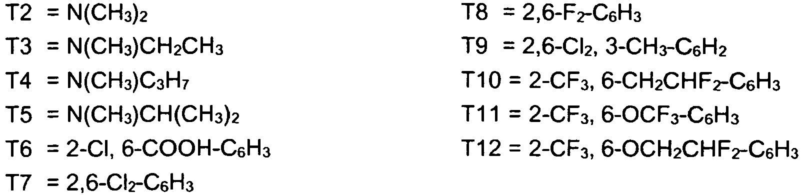

Die Verbindungen I und deren landwirtschaftlich brauchbaren Salze eignen sich - sowohl als Isomerengemische als auch in Form der reinen Isomeren - als Herbizide. Sie eignen sich als solche oder als entsprechend formuliertes Mittel. Die herbiziden Mittel, die die Verbindung I, insbesondere die bevorzugten Ausgestaltungen davon, enthalten, bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I, insbesondere die bevorzugten Ausgestaltungen davon, bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Avena sativa, Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Prunus armeniaca, Prunus cerasus, Prunus dulcis und prunus domestica, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticum aestivum, Triticale, Triticum durum, Vicia faba, Vitis vinifera, Zea mays.

Der Begriff Kulturpflanzen schließt auch solche ein, die durch Züchtung, Mutagenese oder gentechnische Methoden verändert wurden. Gentechnisch veränderte Pflanzen sind Pflanzen, deren genetisches Material in einer Weise verändert worden ist, wie sie unter natürlichen Bedingungen durch Kreuzen, Mutationen oder natürliche Rekombination (d.h. Neuzusammenstellung der Erbinformation) nicht vorkommt. Dabei werden in der Regel ein oder mehrere Gene in das Erbgut der Pflanze integriert, um die Eigenschaften der Pflanze zu verbessern.

Der Begriff Kulturpflanzen umfasst somit auch Pflanzen, die durch züchterische und gentechnische Maßnahmen eine Toleranz gegen bestimmter Herbizidklassen, wie Hydroxyphenylpyruvat-Dioxygenase (HPPD)-Inhibitoren, Acetolactat-Synthase (ALS)-Inhibitoren, wie z. B. Sulfonylharnstoffe (EP-A-0257993, US 5,013,659) oder Imidazolinone (siehe z. B. US 6,222,100, WO 01/82685, WO 00/26390, WO 97/41218, WO 98/02526, WO 98/02527, WO 04/106529, WO 05/20673, WO 03/14357, WO 03/13225, WO 03/14356, WO 04/16073), Enolpyruvylshikimat-3-Phosphat-Synthase (EPSPS)-Inhibitoren wie z. B. Glyphosat (siehe z. B. WO 92/00377), Glutaminsynthetase (GS)-Inhibitoren wie z. B. Glufosinat (siehe z. B. EP-A-0242236, EP-A-242246) oder Oxynil-Herbizide (siehe z. B. US 5,559,024) erworben haben.

Mit Hilfe klassischer Züchtungsmethoden (Mutagenese) wurden zahlreiche Kulturpflanzen, z. B. Clearfield®-Raps, erzeugt, die eine Toleranz gegen Imidazolinone, z. B. Imazamox, haben. Mit Hilfe gentechnischer Methoden wurden Kulturpflanzen, wie Soja, Baumwolle, Mais, Rüben und Raps, erzeugt, die resistent gegen Glyphosat oder Glufosinat sind, erzeugt, welche unter den Handelsnamen RoudupReady^{®} (Glyphosat) und Liberty Link^{®} (Glufosinat) erhältlich sind.

Der Begriff Kulturpflanzen umfasst somit auch Pflanzen, die mit Hilfe gentechnischer Maßnahmen ein oder mehrere Toxine, z. B. solche aus dem Bakterienstamm Bacillus ssp., produzieren. Toxine, die durch solche gentechnisch veränderten Pflanzen hergestellt werden, umfassen z. B. insektizide Proteine von Bacillus spp., insbesondere von B. thuringiensis, wie die Endotoxine Cry1Ab, Cry1Ac, Cry1F, Cry1Fa2, Cry2Ab, Cry3A, Cry3Bb1, Cry9c, Cry34Ab1 oder Cry35Ab1; oder vegetative insektizide Proteine (VIPs), z. B. VIP1, VIP2, VIP3, oder VIP3A; insektizide Proteine von Nematodenkolonisierenden Bakterien, z. B. Photorhabdus spp. oder Xenorhabdus spp.; Toxine aus tierischen Organismen, z. B. Wepsen,-, Spinnen- oder Skorpionstoxine; pilzliche Toxine, z. B. aus Streptomyceten; pflanzliche Lektine, z. B. aus Erbse oder Gerste; Agglutinine; Proteinase-Inhibitoren, z. B. Trypsin-Inhibitoren, Serinprotease-Inhibitoren, Patatin, Cystatin oder Papain-Inhibitoren; Ribosomen-inaktivierende Proteine (RIPs), z. B. Ricin, Mais-RIP, Abrin, Luffin, Saporin oder Bryodin; Steroid-metabolisierende Enzyme, z. B. 3-Hydroxysteroid-Oxidase, Ecdysteroid-IDP-Glycosyl-Transferase, Cholesterinoxidase, Ecdyson-Inhibitoren oder HMG-CoA-Reduktase; Ionenkanalblocker, z. B. Inhibitoren von Natrium- oder Calziumkanälen; Juvenilhormon-Esterase; Rezeptoren für das diuretischen Hormon (Helicokininrezeptoren); Stilbensynthase, Bibenzylsynthase, Chitinasen und Glucanasen. Diese Toxine können in den Pflanzen auch als Prätoxine, Hybridproteine, verkürzte oder anderweitig modfizierte Proteine produziert werden. Hybridproteine zeichnen sich durch eine neue Kombination von verschiedenen Proteindomänen aus (siehe z. B. WO 2002/015701). Weitere Besipiele für derartige Toxine oder gentechnisch veränderte Pflanzen, die diese Toxine produzieren sind in EP-A 374 753, WO 93/007278, WO 95/34656, EP-A 427 529, EP-A 451 878,

WO 03/018810 und WO 03/052073 offenbart. Die Methoden zur Herstellung dieser gentechnisch veränderten Pflanzen sind dem Fachmann bekannt und z. B. in den oben erwähnten Publikationen dargelegt. Zahlreiche der zuvor genannten Toxine verleihen den Pflanzen, die diese produzieren, eine Toleranz gegen Schädlinge aus allen taxonomischen Arthropodenklassen, insbesondere gegen Käfer (Coeleropta), Zweiflügler (Diptera) und Schmetterlinge (Lepidoptera) und gegen Nematoden (Nematoda).

Gentechnisch veränderte Pflanzen, die ein oder mehrere Gene, die für insektizide Toxine kodieren, produzieren sind z. B. in den oben erwähnten Publikationen beschrieben und zum Teil kommerziell erhältlich, wie z. B. YieldGard^{®} (Maissorten, die das Toxin Cry1Ab produzieren), YieldGard^{®} Plus (Maissorten, die die Toxine CrylAb und Cry3Bb1 produzieren), Starlink^{®} (Maissorten, die das Toxin Cry9c produzieren), Herculex^{®} RW (Maissorten, die die Toxine Cry34Ab1, Cry35Ab1 und das Enzym Phosphinothricin-N-Acetyltransferase [PAT] produzieren); NuCOTN^{®} 33B (Baumwollsorten, die das Toxin Cry1Ac produzieren), Bollgard^{®} I (Baumwollsorten, die das Toxin Cry1Ac produzieren), Bollgard^{®} II (Baumwollsorten, die die Toxine Cry1Ac und Cry2Ab2 produzieren); VIPCOT^{®} (Baumwollsorten, die ein VIP-Toxin produzieren); NewLeaf^{®} (Kartoffelsorten, die das Toxin Cry3A produzieren); Bt-Xtra^{®}, NatureGard^{®}, KnockOut^{®}, BiteGard^{®}, Protecta^{®}, Bt11 (z. B. Agrisure^{®} CB) und Bt176 von Syngenta Seeds SAS, Frankreich, (Maissorten, die das Toxin Cry1Ab und das PAT-Enyzm produzieren), MIR604 von Syngenta Seeds SAS, Frankreich (Maissorten, die ein modifizierte Version des Toxins Cry3A produzieren, siehe hierzu WO 03/018810), MON 863 von Monsanto Europe S.A., Belgien (Maissorten, die das Toxin Cry3Bb1 produzieren), IPC 531 von Monsanto Europe S.A., Belgien (Baumwollsorten, die eine modifizierte Version des Toxins Cry1Ac produzieren) und 1507 von Pioneer Overseas Corporation, Belgien (Maissorten, die das Toxin Cry1F und das PAT-Enyzm produzieren).

Der Begriff Kulturpflanzen umfasst somit auch Pflanzen, die mit Hilfe gentechnischer Maßnahmen ein oder mehrere Proteine produzieren, die eine erhöhte Resistenz oder Widerstandfähigkeit gegen bakterielle, virale oder pilzliche Pathogene bewirken, wie z. B. sogenannte Pathogenesis-related-Proteine (PR-Proteine, siehe EP-A 0 392 225), Resistenzproteine (z. B. Kartoffelsorten, die zwei Resistenzgene gegen Phytophthora infestans aus der mexikanischen Wildkartoffel Solanum bulbocastanum produzieren) oder T4-Lysozym (z. B. Kartoffelsorten, die durch die Produktion diese Proteins resistent gegen Bakterien wie Erwinia amylvora ist).

Der Begriff Kulturpflanzen umfasst somit auch Pflanzen, deren Produktivität mit Hilfe gentechnischer Methoden verbessert wurde, indem z. B. die Ertragsfähigkeit (z. B. Biomasse, Kornertrag, Stärke-, Öl- oder Proteingehalt), die Toleranz gegenüber Trockenheit, Salz oder anderen begrenzenden Umweltfaktoren oder die Widerstandsfähigkeit gegenüber Schädlingen und pilzlichen, bakteriellen und viralen Pathogenen gesteigert wird.

Der Begriff Kulturpflanzen umfasst auch Pflanzen, deren Inhaltsstoffe insbesondere zur Verbesserung der menschlichen oder tierischen Ernährung mit Hilfe gentechnischer Methoden verändert wurden, indem z. B. Ölpflanzen gesundheitsfördernde langkettige Omega-3-Fettsäuren oder einfach ungesättigte Omega-9-Fettsäuren (z. B. Nexera^{®}-Raps) produzieren.

Der Begriff Kulturpflanzen umfasst auch Pflanzen, die zur verbesserten Produktion von Rohstoffen mit Hilfe gentechnischer Methoden verändert wurden, indem z. B. der Amylopektin-Gehalt von Kartoffeln (Amflora^{®}-Kartoffel) erhöht wurde.

Des Weiteren wurde gefunden, dass die Verbindungen der Formel I auch zur Defoliation und/oder Desikkation von Pflanzenteilen geeignet ist, wofür Kulturpflanzen wie Baumwolle, Kartoffel, Raps, Sonnenblume, Sojabohne oder Ackerbohnen, insbesondere Baumwolle, in Betracht kommen. Diesbezüglich wurden Mittel zur Desikkation und /oder Defoliation von Pflanzen, Verfahren zur Herstellung dieser Mittel und Verfahren zur Desikkation und/oder Defoliation von Pflanzen mit der Verbindungen der Formel I gefunden.

Als Desikkantien eignen sich die Verbindungen der Formel I insbesondere zur Austrocknung der oberirdischen Teile von Kulturpflanzen wie Kartoffel, Raps, Sonnenblume und Sojabohne aber auch Getreide. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, d.h., die Förderung der Ausbildung von Trenngewebe zwischen Frucht- oder Blatt- und Sprossteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Qualität der Faser nach der Ernte.

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen, Gießen oder Behandlung des Saatgutes bzw. Mischen mit dem Saatgut angewendet werden. Die Anwendungsformen richten sich nach den Verwendungszwecken; sie sollten in jedem Fall möglichst die feinste Verteilung der erfindungsgemäßen Wirkstoffe gewährleistet.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsstoffe.

Beispiele für die Formulierung von Pflanzenschutzmitteln übliche Hilfsmittel sind inerte Hilfsstoffe, feste Trägerstoffe, oberflächenaktive Stoffe (wie Dispergiermittel Schutzkolloide, Emulgatoren, Netzmittel und Haftmittel), organische und anorganische Verdicker, Bakterizide, Frostschutzmittel, Entschäumer ggf. Farbstoffe und für Saatgutformulierungen Kleber.

Beispiele für Verdicker (d.h. Verbindungen, die der Formulierung ein modifiziertes Fließverhalten verleihen, d.h. hohe Viskosität im Ruhezustand und niedrige Viskosität im bewegten Zustand) sind Polysaccharide wie Xanthan Gum (Kelzan® der Fa. Kelco), Rhodopol® 23 (Rhone Poulenc) oder Veegum® (Firma R.T. Vanderbilt) sowie organische und anorganische Schichtmineralienwie Attaclay® (Firma Engelhardt).

Beispiele für Antischaummittel sind Silikonemulsionen (wie z.Bsp. Silikon® SRE, Firma Wacker oder Rhodorsil® der Firma Rhodia), langkettige Alkohole, Fettsäuren, Salze von Fettsäuren, fluororganische Verbindungen und deren Gemische.

Bakterizide können zur Stabilisierung der wäßrigen Herbizid-Formulierung zugesetzt werden. Beispiele für Bakterizide sind Bakterizide basierend auf Diclorophen und Benzylalkoholhemiformal (Proxel® der Fa. ICI oder Acticide® RS der Fa. Thor Chemie und Kation® MK der Firma Rohm & Haas) sowie Isothiazolinonderivaten wie Alkylisothiazolinonen und Benzisothiazolinonen (Acticide MBS der Fa. Thor Chemie)

Beispiele für Frostschutzmittel sind Ethylenglycol, Propylenglycol, Harnstoff oder Glycerin.

Beispiele für Farbmittel sind sowohl in Wasser wenig lösliche Pigmente als auch in Wasser lösliche Farbstoffe. Als Beispiele genannt seien die unter den Bezeichnungen Rhodamin B, C.I. Pigment Red 112 und C.I. Solvent Red 1 bekannten Farbstoffe, sowie pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

Beispiele für Kleber sind Polyvinylpyrrolidon, Polyvinylacetat, Polyvinylalkohol und Tylose.

Als inerte Zusatzstoffe kommen beispielsweise in Betracht:
Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z. B. Amine wie N-Methylpyrrolidon oder Wasser.

Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Als oberflächenaktive Stoffe (Adjuvantien, Netz-, Haft-, Dispergier- sowie Emulgiermittel) kommen die Alkali-, Erdalkali-, Ammoniumsalze von aromatischen Sulfonsäuren, z.B. Ligninsulfonsäuren (z.B. Borrespers-Typen, Borregaard), Phenolsulfonsäuren, Naphthalinsulfonsäuren (Morwet-Typen, Akzo Nobel) und Dibutylnaphthalinsulfonsäure (Nekal-Typen, BASF SE), sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen sowie Proteine, denaturierte Proteine, Polysaccharide (z.B. Methylcellulose), hydrophob modifizierte Stärken, Polyvinylalkohol (Mowiol typen Clariant), Polycarboxylate (BASF SE, Sokalan-Typen), Polyalkoxylate, Polyvinylamin (BASF SE, Lupamin-Typen), Polyethylenimin (BASF SE, Lupasol-Typen), Polyvinylpyrrolidon und deren Copolymere in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden.

Wässrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Verbindungen der Formel I oder la als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Die Konzentrationen der Verbindungen der Formel I in den anwendungsfertigen Zubereitungen können in weiten Bereichen variiert werden. Die Formulierungen enthalten im Allgemeinen 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die erfindungsgemäßen Verbindungen I können beispielsweise wie folgt formuliert werden:
1. Produkte zur Verdünnung in Wasser
   A Wasserlösliche Konzentrate
      10 Gew.-Teile Wirkstoff werden mit 90 Gew.-Teilen Wasser oder einem wasserlöslichen Lösungsmittel gelöst. Alternativ werden Netzmittel oder andere Hilfsmittel zugefügt. Bei der Verdünnung in Wasser löst sich der Wirkstoff. Man erhält auf diese Weise eine Formulierung mit 10 Gew.-% Wirkstoffgehalt.
   B Dispergierbare Konzentrate
      20 Gew.-Teile Wirkstoff werden in 70 Gew.-Teilen Cyclohexanon unter Zusatz von 10 Gew.-Teilen eines Dispergiermittels z.B. Polyvinylpyrrolidon gelöst. Bei Verdünnung in Wasser ergibt sich eine Dispersion. Der Wirkstoffgehalt beträgt 20 Gew.-%
   C Emulgierbare Konzentrate
      15 Gew.-Teile Wirkstoff werden in 75 Gew.-Teilen eines organisches Lösungsmittels (z.B. Alkylaromaten)-unter Zusatz von Ca-Dodecylbenzolsulfonat und Ricinusölethoxylat (jeweils 5 Gew.-Teile) gelöst. Bei der Verdünnung in Wasser ergibt sich eine Emulsion. Die Formulierung hat 15 Gew.-% Wirkstoffgehalt.
   D Emulsionen
      25 Gew.-Teile Wirkstoff werden in 35 Gew.-Teilen eines organisches Lösungsmittels (z.B. Alkylaromaten) unter Zusatz von Ca-Dodecylbenzolsulfonat und Ricinusölethoxylat (jeweils 5 Gew.-Teile) gelöst. Diese Mischung wird mittels einer Emulgiermaschine (z.B. Ultraturax) in 30 Gew.Teile Wasser gegeben und zu einer homogenen Emulsion gebracht. Bei der Verdünnung in Wasser ergibt sich eine Emulsion. Die Formulierung hat einen Wirkstoffgehalt von 25 Gew.-%.
   E Suspensionen
      20 Gew.-Teile Wirkstoff werden unter Zusatz von 10 Gew.-Teilen Dispergier- und Netzmitteln und 70 Gew.-Teilen Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs. Der Wirkstoffgehalt in der Formulierung beträgt 20 Gew.-% .
   F Wasserdispergierbare und wasserlösliche Granulate
      50 Gew.-Teile Wirkstoff werden unter Zusatz von 50 Gew-Teilen Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs. Die Formulierung hat einen Wirkstoffgehalt von 50 Gew.-%.
   G Wasserdispergierbare und wasserlösliche Pulver
      75 Gew.-Teile Wirkstoff werden unter Zusatz von 25 Gew.-Teilen Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs. Der Wirkstoffgehalt der Formulierung beträgt 75 Gew.-%.
   H Gelformulierungen
      In einer Kugelmühle werden 20 Gew.-Teile Wirkstoff, 10 Gew.-Teile Dispergiermittel, 1 Gew.-Teil Geliermittel und 70 Gew.-Teile Wasser oder eines organischen Lösungsmittels zu einer feinen Suspension vermahlen. Bei der Verdünnung mit Wasser ergibt sich eine stabile Suspension mit 20 Gew.-% Wirkstoffgehalt.
2. Produkte für die Direktapplikation
   I Stäube
      5 Gew.-Teile Wirkstoff werden fein gemahlen und mit 95 Gew.-Teilen feinteiligem Kaolin innig vermischt. Man erhält dadurch ein Stäubemittel mit 5 Gew.-% Wirkstoffgehalt.
   J Granulate (GR, FG, GG, MG)
      0,5 Gew-Teile Wirkstoff werden fein gemahlen und mit 99,5 Gewichtsteilen Trägerstoffe verbunden. Gängige Verfahren sind dabei die Extrusion, die Sprühtrocknung oder die Wirbelschicht. Man erhält dadurch ein Granulat für die Direktapplikation mit 0,5 Gew.-% Wirkstoffgehalt.
   K ULV- Lösungen (UL)
      10 Gew.-Teile Wirkstoff werden in 90 Gew.-Teilen eines organischen Lösungsmittels z.B. Xylol gelöst. Dadurch erhält man ein Produkt für die Direktapplikation mit 10 Gew.-% Wirkstoffgehalt.

Die Applikation der Verbindungen I oder der sie enthaltenden herbiziden Mittel kann im Vorauflauf-, im Nachauflaufverfahren oder zusammen mit dem Saatgut einer Kulturpflanze erfolgen. Es besteht auch die Möglichkeit, die herbiziden Mittel bzw. Wirkstoffe dadurch zu applizieren, dass mit den herbiziden Mitteln bzw. Wirkstoffen vorbehandeltes Saatgut einer Kulturpflanze ausgebracht wird. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

In einer weiteren Ausführungsform kann die Applikation der Verbindungen der Formel I bzw. der herbiziden Mittel durch Behandlung von Saatgut erfolgen.

Die Behandlung von Saatgut umfasst im Wesentlichen alle dem Fachmann geläufigen Techniken (seed dressing, seed coating, seed dusting, seed soaking, seed film coating, seed multilayer coating, seed encrusting, seed dripping, und seed pelleting) basierend auf den erfindungsgemäßen Verbindungen der Formel I bzw. daraus hergestellten Mitteln. Hierbei können die herbiziden Mittel verdünnt oder unverdünnt aufgetragen werden.

Der Begriff Saatgut umfasst Saatgut aller Arten, wie z.B. Körner, Samen, Früchte, Knollen, Stecklinge und ähnliche Formen. Bevorzugt beschreibt der Begriff Saatgut hier Körner und Samen.

Als Saatgut kann Saatgut der oben erwähnten Nutzpflanzen aber auch das Saatgut transgener oder durch herkömmliche Züchtungsmethoden erhaltener Pflanzen eingesetzt werden.

Die Aufwandmengen an Wirkstoff betragen je nach Bekämpfungsziel, Jahreszeit, Zielpflanzen und Wachstumsstadium 0.001 bis 3.0, vorzugsweise 0.01 bis 1.0 kg/ha aktive Substanz (a. S.). Zur Saatgutbehandlung werden die Verbindungen I üblicherweise in Mengen von 0,001 bis 10 kg pro 100 kg Saatgut eingesetzt.

Es kann auch von Vorteil sein, die Verbindungen der Formel I in Kombination mit Safenern zu verwenden. Safener sind chemische Verbindungen, die Schaden an Nutzpflanzen verhindern oder reduzieren, ohne die herbizide Wirkung der Verbindungen der Formel I auf unerwünschte Pflanzen wesentlich zu beeinflussen. Sie können sowohl vor der Aussaat (beispielsweise bei Saatgutbehandlungen, bei Stecklingen oder Setzlingen) als auch im Vor- oder Nachauflauf der Nutzpflanze verwendet werden. Die Safener und die Verbindungen der Formel I können gleichzeitig oder nacheinander verwendet werden. Geeignete Safener sind beispielsweise (Chinolin-8-oxy)essigsäuren, 1-Phenyl-5-haloalkyl-1H-1,2,4-triazol-3-carbonsäuren, 1-Phenyl-4,5-dihydro-5-alkyl-1 H-pyrazol-3,5-dicarbonsäuren, 4,5-Dihydro-5,5-diaryl-3-isoxazolcarbonsäuren, Dichloroacetamide, alpha-Oximinophenylacetoritrile, Acetophenonoxime, 4,6-Dihalo-2-phenylpyrimidine, N-[[4-(Aminocarbonyl)phenyl]sulfonyl]-2-benzoesäureamide, 1,8-Naphthalsäureanhydrid, 2-Halo-4-(haloalkyl)-5-thiazolcarbonsäuren, Phosphorthiolate und N-Alkyl-O-phenylcarbamate sowie ihre landwirtschaftlich brauchbaren Salze, und vorausgesetzt sie haben eine Säurefunktion, ihre landwirtschaftlich brauchbaren Derivate, wie Amide, Ester und Thioester.

Zur Verbreiterung des Wirkungsspektrums und zur Erzielung synergistischer Effekte können die Verbindungen der Formel I mit zahlreichen Vertretern anderer herbizider oder wachstumsregulierender Wirkstoffgruppen oder mit Safenern gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile sowie Phenylpyrazoline und Isoxazoline und deren Derivate in Betracht.

Außerdem kann es von Nutzen sein, die Verbindungen I allein oder in Kombination mit anderen Herbiziden oder auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch weitere Additve wie nicht phytotoxische Öle und Ölkonzentrate zugesetzt werden.

Beispiele für Herbizide, die in Kombination mit den Pyridinverbindungen der Formel I gemäß der vorliegenden Erfindung verwendet werden können, sind:
b1) aus der Gruppe der Lipid-Biosynthese-Inhibitoren:
   Alloxydim, Alloxydim-natrium, Butroxydim, Clethodim, Clodinafop, Clodinafop-propargyl, Cycloxydim, Cyhalofop, Cyhalofop-butyl, Diclofop, Diclofop-methyl, Fenoxaprop, Fenoxaprop-ethyl, Fenoxaprop-P, Fenoxaprop-P-ethyl, Fluazifop, Fluazifop-butyl, Fluazifop-P, Fluazifop-P-butyl, Haloxyfop, Haloxyfop-methyl, Haloxyfop-P, Haloxyfop-P-methyl, Metamifop, Pinoxaden, Profoxydim, Propaquizafop, Quizalofop, Quizalofopethyl, Quizalofop-tefuryl, Quizalofop-P, Quizalofop-P-ethyl, Quizalofop-P-tefuryl, Sethoxydim, Tepraloxydim, Tralkoxydim, Benfuresat, Butylat, Cycloat, Dalapon, Dimepiperat, EPTC, Esprocarb, Ethofumesat, Flupropanat, Molinat, Orbencarb, Pebulat, Prosulfocarb, TCA, Thiobencarb, Tiocarbazil, Triallat und Vernolat;
b2) aus der Gruppe der ALS-Inhibitoren:
   Amidosulfuron, Azimsulfuron, Bensulfuron, Bensulfuron-methyl, Bispyribac, Bispyribac-natrium, Chlorimuron, Chlorimuron-ethyl, Chlorsulfuron, Cinosulfuron, Cloransulam, Cloransulam-methyl, Cyclosulfamuron, Diclosulam, Ethametsulfuron, Ethametsulfuronmethyl, Ethoxysulfuron, Flazasulfuron, Florasulam, Flucarbazon, Flucarbazon-natrium, Flucetosulfuron, Flumetsulam, Flupyrsulfuron, Flupyrsulfuron-methyl-natrium, Foramsulfuron, Halosulfuron, Halosulfuron-methyl, Imazamethabenz, Imazamethabenzmethyl, Imazamox, Imazapic, Imazapyr, Imazaquin, Imazethapyr, Imazosulfuron, lodosulfuron, lodosulfuron-methyl-natrium, Mesosulfuron, Metosulam, Metsulfuron, Metsulfuron-methyl, Nicosulfuron, Orthosulfamuron, Oxasulfuron, Penoxsulam, Primisulfuron, Primisulfuron-methyl, Propoxycarbazon, Propoxycarbazon-natrium, Prosulfuron, Pyrazosulfuron, Pyrazosulfuron-ethyl, Pyribenzoxim, Pyrimisulfan, Pyriftalid, Pyriminobac, Pyriminobac-methyl, Pyrithiobac, Pyrithiobac-natrium, Pyroxsulam, Rimsulfuron, Sulfometuron, Sulfometuron-methyl, Sulfosulfuron, Thiencarbazon, Thiencarbazon-methyl, Thifensulfuron, Thifensulfuron-methyl, Triasulfuron, Tribenuron, Tribenuron-methyl, Trifloxysulfuron, Triflusulfuron, Triflusulfuron-methyl und Tritosulfuron;
b3) aus der Gruppe der Photosynthese-Inhibitoren:
   Ametryn, Amicarbazon, Atrazin, Bentazon, Bentazon-natrium, Bromacil, Bromofenoxim, Bromoxynil und seine Salze und Ester, Chlorobromuron, Chloridazon, Chlorotoluron, Chloroxuron, Cyanazin, Desmedipham, Desmetryn, Dimefuron, Dimethametryn, Diquat, Diquat-dibromid, Diuron, Fluometuron, Hexazinon, loxynil und seine Salze und Ester, Isoproturon, Isouron, Karbutilat, Lenacil, Linuron, Metamitron, Methabenzthiazuron, Metobenzuron, Metoxuron, Metribuzin, Monolinuron, Neburon, Paraquat, Paraquat-dichlorid, Paraquat-dimetilsulfat, Pentanochlor, Phenmedipham, Phenmediphamethyl, Prometon, Prometryn, Propanil, Propazin, Pyridafol, Pyridat, Siduron, Simazin, Simetryn, Tebuthiuron, Terbacil, Terbumeton, Terbuthylazin, Terbutryn, Thidiazuron und Trietazin;
b4) aus der Gruppe der Protoporphyrinogen-IX-Oxidase-Inhibitoren:
   Acifluorfen, Acifluorfen-natrium, Azafenidin, Bencarbazon, Benzfendizon, Bifenox, Butafenacil, Carfentrazon, Carfentrazon-ethyl, Chlomethoxyfen, Cinidon-ethyl, Fluazolat, Flufenpyr, Flufenpyr-ethyl, Flumiclorac, Flumiclorac-pentyl, Flumioxazin, Fluoroglycofen, Fluoroglycofen-ethyl, Fluthiacet, Fluthiacet-methyl, Fomesafen, Halosafen, Lactofen, Oxadiargyl, Oxadiazon, Oxyfluorfen, Pentoxazon, Profluazol, Pyraclonil, Pyraflufen, Pyraflufen-ethyl, Saflufenacil, Sulfentrazon, Thidiazimin, 2-Chlor-5-[3,6-dihydro-3-methyl-2,6-dioxo-4-(trifluormethyl)-1 (2H)-pyrimidinyl]-4-fluor-N-[(isopropyl)methylsulfamoyl]benzamid (H-1; CAS 372137-35-4), [3-[2-Chlor-4-fluor-5-(1-methyl-6-trifluormethyl-2,4-dioxo-1,2,3,4,-tetrahydropyrimidin-3-yl)phenoxy]-2-pyridyloxy]essigsäureethylester (H-2; CAS 353292-31-6), N-Ethyl-3-(2,6-dichlor-4-trifluormethylphenoxy)-5-methyl-1 H-pyrazol-1-carboxamid (H-3; CAS 452098-92-9), N-Tetrahydrofurfuryl-3-(2,6-dichlor-4-trifluormethylphenoxy)-5-methyl-1 H-pyrazol-1-carboxamid (H-4; CAS 915396-43-9), N-Ethyl-3-(2-chlor-6-fluor-4-trifluormethylphenoxy)-5-methyl-1 H-pyrazol-1-carboxamid (H-5; CAS 452099-05-7) und N-Tetrahydrofurfuryl-3-(2-chlor-6-fluor-4-trifluormethylphenoxy)-5-methyl-1 H-pyrazol-1-carboxamid (H-6; CAS 45100-03-7);
b5) aus der Gruppe der Bleacher-Herbizide:
   Aclonifen, Amitrol, Beflubutamid, Benzobicyclon, Benzofenap, Clomazon, Diflufenican, Fluridon, Flurochloridon, Flurtamon, Isoxaflutol, Mesotrion, Norflurazon, Picolinafen, Pyrasulfutol, Pyrazolynat, Pyrazoxyfen, Sulcotrion, Tefuryltrion, Tembotrion, Topramezon, 4-Hydroxy-3-[[2-[(2-methoxyethoxy)methyl]-6-(trifluormethyl)-3-pyridyl]carbonyl]bicyclo[3.2.1]oct-3-en-2-one (H-7; CAS 352010-68-5) und 4-(3-Trifluormethylphenoxy)-2-(4-trifluormethylphenyl)pyrimidin (H-8; CAS 180608-33-7);
b6) aus der Gruppe der EPSP-Synthase-Inhibitoren:
   Glyphosat, Glyphosat-isopropylammonium und Glyphosat-trimesium (Sulfosat);
b7) aus der Gruppe der Glutamin-Synthase-Inhibitoren:
   Bilanaphos (Bialaphos), Bilanaphos-natrium, Glufosinat und Glufosinat-ammonium;
b8) aus der Gruppe der DHP-Synthase-Inhibitoren: Asulam;
b9) aus der Gruppe der Mitose-Inhibitoren:
   Amiprophos, Amiprophos-methyl, Benfluralin, Butamiphos, Butralin, Carbetamid, Chlorpropham, Chlorthal, Chlorthal-dimethyl, Dinitramin, Dithiopyr, Ethalfluralin, Fluchloralin, Oryzalin, Pendimethalin, Prodiamin, Propham, Propyzamid, Tebutam, Thiazopyr und Trifluralin;
b10) aus der Gruppe der VLCFA-Inhibitoren:
   Acetochlor, Alachlor, Anilofos, Butachlor, Cafenstrol, Dimethachlor, Dimethanamid, Dimethenamid-P, Diphenamid, Fentrazamid, Flufenacet, Mefenacet, Metazachlor, Metolachlor, Metolachlor-S, Naproanilid, Napropamid, Pethoxamid, Piperophos, Pretilachlor, Propachlor, Propisochlor, Pyroxasulfon (KIH-485) und Thenylchlor;

Verbindungen der Formel 2: 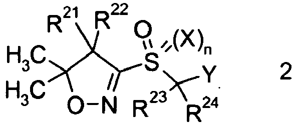 worin die Variablen folgende Bedeutungen haben:
Y Phenyl oder 5- oder 6-gliedriges Heteroaryl wie eingangs definiert, welche durch eine bis drei Gruppen R^{aa} substituiert sein können; R²¹,R²²,R²³,R²⁴ H, Halogen, oder C₁-C₄-Alkyl; X O oder NH; n 0 oder 1.

Verbindungen der Formel 2 weisen insbesondere die folgenden Bedeutungen auf: Y 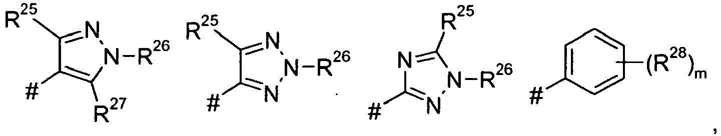 wobei # die Bindung zu dem Molekülgerüst bedeutet; und
R²¹,R²²,R²³,R²⁴ H, Cl, F oder CH₃; R²⁵ Halogen, C₁-C₄-Alkyl oder C₁-C₄-Haloalkyl; R²⁶ C₁-C₄-Alkyl; R²⁷ Halogen, C₁-C₄-Alkoxy oder C₁-C₄-Haloalkoxy; R²⁸ H, Halogen, C₁-C₄-Alkyl, C₁-C₄-Haloalkyl oder C₁-C₄-Haloalkoxy; m 0, 1, 2 oder 3; X Sauerstoff;
n 0 oder 1.

Bevorzugte Verbindungen der Formel 2 weisen folgende Bedeutungen auf: Y

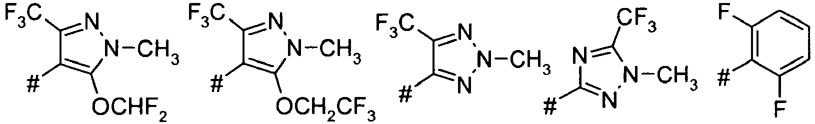

R²¹ H; R²², R²³ F; R²⁴ H oder F; X Sauerstoff; n 0 oder 1.

Besonders bevorzugte Verbindungen der Formel 2 sind:
3-[5-(2,2-Difluor-ethoxy)-1-methyl-3-trifluormethyl-1H-pyrazol-4-ylmethansulfonyl]-4-fluor-5,5-dimethyl-4,5-dihydro-isoxazol (2-1); 3-{[5-(2,2-Difluor-ethoxy)-1-methyl-3-trifluormethyl-1 H-pyrazol-4-yl]-fluor-methansulfonyl}-5,5-dimethyl-4,5-dihydro-isoxazol (2-2); 4-(4-Fluor-5,5-dimethyl-4,5-dihydro-isoxazol-3-sulfonylmethyl)-2-methyl-5-trifluormethyl-2H-[1,2,3]triazol (2-3); 4-[(5,5-Dimethyl-4,5-dihydro-isoxazol-3-sulfonyl)-fluormethyl]-2-methyl-5-trifluormethyl-2H-[1,2,3]triazol (2-4); 4-(5,5-Dimethyl-4,5-dihydro-isoxazol-3-sulfonylmethyl)-2-methyl-5-trifluormethyl-2H-[1,2,3]triazol (2-5); 3-{[5-(2,2-Difluor-ethoxy)-1-methyl-3-trifluormethyl-1 H-pyrazol-4-yl]-difluor-methansulfonyl}-5,5-dimethyl-4,5-dihydro-isoxazol (2-6); 4-[(5,5-Dimethyl-4,5-dihydro-isoxazol-3-sulfonyl)-difluor-methyl]-2-methyl-5-trifluormethyl-2H-[1,2,3]triazol (2-7); 3-{[5-(2,2-Difluorethoxy)-1-methyl-3-trifluormethyl-1 H-pyrazol-4-yl]-difluor-methansulfonyl}-4-fluor-5,5-dimethyl-4,5-dihydro-isoxazol (2-8); 4-[Difluor-(4-fluor-5,5-dimethyl-4,5-dihydro-isoxazol-3-sulfonyl)-methyl]-2-methyl-5-trifluormethyl-2H-[1,2,3]triazol (2-9);
b11) aus der Gruppe der Cellulose-Biosynthese-Inhibitoren:
   Chlorthiamid, Dichlobenil, Flupoxam und Isoxaben;
b12) aus der Gruppe der Entkoppler-Herbizide:
   Dinoseb, Dinoterb und DNOC und seine Salze;
b13) aus der Gruppe der Auxin-Herbizide:
   2,4-D und seine Salze und Ester, 2,4-DB und seine Salze und Ester, Aminopyralid und seine Salze wie Aminopyralid-tris(2-hydroxypropyl)ammonium und seine Ester, Benazolin, Benazolin-ethyl, Chloramben und seine Salze und Ester, Clomeprop, Clopyralid und seine Salze und Ester, Dicamba und seine Salze und Ester, Dichlorprop und seine Salze und Ester, Dichlorprop-P und seine Salze und Ester, Fluroxypyr, Fluroxypyr-butometyl, Fluroxypyr-meptyl, MCPA und seine Salze und Ester, MCPA-thioethyl, MCPB und seine Salze und Ester, Mecoprop und seine Salze und Ester, Mecoprop-P und seine Salze und Ester, Picloram und seine Salze und Ester, Quinclorac, Quinmerac, TBA (2,3,6) und seine Salze und Ester, Triclopyr und seine Salze und Ester, und 5,6-Dichlor-2-cyclopropyl-4-pyrimidincarbonsäure (H-9; CAS 858956-08-8) und seine Salze und Ester;
b14) aus der Gruppe der Auxin-Transport-Inhibitoren: Diflufenzopyr, Diflufenzopyr-natrium, Naptalam und Naptalam-natrium;
b15) aus der Gruppe der sonstigen Herbizide: Bromobutid, Chlorflurenol, Chlorflurenol-methyl, Cinmethylin, Cumyluron, Dalapon, Dazomet, Difenzoquat, Difenzoquat-metilsulfate, Dimethipin, DSMA, Dymron, Endothal und seine Salze, Etobenzanid, Flamprop, Flamprop-isopropyl, Flamprop-methyl Flamprop-M-isopropyl, Flamprop-M-methyl, Flurenol, Flurenol-butyl, Flurprimidol, Fosamin, Fosamine-ammonium, Indanofan, Maleinsäure-hydrazid, Mefluidid, Metam, Methylazid, Methylbromid, Methyl-dymron, Methyljodid. MSMA, Ölsäure, Oxaziclomefon, Pelargonsäure, Pyributicarb, Quinoclamin, Triaziflam, Tridiphan und 6-Chlor-3-(2-cyclopropyl-6-methylphenoxy)-4-pyridazinol (H-10; CAS 499223-49-3) und seine Salze und Ester.

Beispiele für bevorzugte Safener C sind Benoxacor, Cloquintocet, Cyometrinil, Cyprosulfamid, Dichlormid, Dicyclonon, Dietholate, Fenchlorazol, Fenclorim, Flurazol, Fluxofenim, Furilazol, Isoxadifen, Mefenpyr, Mephenat, Naphthalsäureanhydrid, Oxabetrinil, 4-(Dichloracetyl)-1-oxa-4-azaspiro[4.5]decan (H-11; MON4660, CAS 71526-07-3) und 2,2,5-Trimethyl-3-(dichloracetyl)-1,3-oxazolidin (H-12; R-29148, CAS 52836-31-4). Die Wirkstoffe der Gruppen b1) bis b15) und die Safener C sind bekannte Herbizide und Safener, siehe z. B. The Compendium of Pesticide Common Names (http://www.alanwood.net/pesticides/); B. Hock, C. Fedtke, R. R. Schmidt, Herbizide, Georg Thieme Verlag, Stuttgart 1995. Weitere herbizide Wirkstoffe sind aus WO 96/26202, WO 97/41116, WO 97/41117, WO 97/41118, WO 01/83459 und WO 2008/074991 sowie aus W. Krämer et al. (ed.) "Modern Crop Protection Compounds", Vol. 1, Wiley VCH, 2007 und der darin zitierten Literatur bekannt.

Die Erfindung betrifft auch Zusammensetzungen in Form eines als 1-Komponentenzusammensetzung formulierten Pflanzenschutzmittels, enthaltend eine Wirkstoffkombination, die wenigstens eine Pyridinverbindung der Formel I und wenigstens einen weiteren Wirkstoff, bevorzugt ausgewählt aus den Wirkstoffen der Gruppen b1 bis b15, und wenigstens einen festen oder flüssigen Träger und/oder eine oder mehrere grenzflächenaktive Substanzen und gewünschtenfalls einen oder mehrere für Pflanzenschutzmittel übliche weitere Hilfsstoffe.

Die Erfindung betrifft auch Zusammensetzungen in Form eines als 2-Komponentenzusammensetzung formulierten Pflanzenschutzmittels, umfassend eine erste Komponente, enthaltend wenigstens eine Pyridinverbindung der Formel I, einen festen oder flüssigen Träger und/oder eine oder mehrere grenzflächenaktive Substanzen, und eine zweite Komponente, enthaltend wenigstens einen weiteren Wirkstoff, ausgewählt aus den Wirkstoffen der Gruppen b1 bis b15, einem festen oder flüssigen Träger und/oder eine oder mehrere grenzflächenaktive Substanzen, wobei zusätzlich beide Komponenten auch weitere, für Pflanzenschutzmittel üblichen Hilfsmittel enthalten können.

In binären Zusammensetzungen, die wenigstens eine Verbindung der Formel I als Komponente A und wenigstens ein Herbizid B enthalten, liegt das Gewichtsverhältnis der Wirkstoffe A:B in der Regel im Bereich von 1:1000 bis 1000:1, vorzugsweise im Bereich von 1:500 bis 500:1, insbesondere im Bereich von 1:250 bis 250:1 und besonders bevorzugt im Bereich von 1:75 bis 75:1.

In binären Zusammensetzungen, die wenigstens eine Verbindung der Formel I als Komponente A und wenigstens einen Safener C enthalten, liegt das Gewichtsverhältnis der Wirkstoffe A:C in der Regel im Bereich von 1:1000 bis 1000:1, vorzugsweise im Bereich von 1:500 bis 500:1, insbesondere im Bereich von 1:250 bis 250:1 und besonders bevorzugt im Bereich von 1:75 bis 75:1.

In ternären Zusammensetzungen, die sowohl wenigstens eine Verbindung der Formel I als Komponente A, wenigstens ein Herbizid B und wenigstens einen Safener C enthalten, liegen die relativen Gewichtsanteile der Komponenten A:B in der Regel im Bereich von 1:1000 bis 1000:1, vorzugsweise im Bereich von 1:500 bis 500:1, insbesondere im Bereich von 1:250 bis 250:1 und besonders bevorzugt im Bereich von 1:75 bis 75:1, das Gewichtsverhältnis der Komponente A:C in der Regel im Bereich von 1:1000 bis 1000:1, vorzugsweise im Bereich von 1:500 bis 500:1, insbesondere im Bereich von 1:250 bis 250:1 und besonders bevorzugt im Bereich von 1:75 bis 75:1, und das Gewichtsverhältnis der Komponenten B:C in der Regel im Bereich von 1:1000 bis 1000:1, vorzugsweise im Bereich von 1:500 bis 500:1, insbesondere im Bereich von 1:250 bis 250:1 und besonders bevorzugt im Bereich von 1:75 bis 75:1. Vorzugsweise liegt das Gewichtsverhältnis der Komponenten A + B zur Komponente C im Bereich von 1:500 bis 500:1, insbesondere im Bereich von 1:250 bis 250:1 und besonders bevorzugt im Bereich von 1:75 bis 75:1.

Beispiele für besonders bevorzugte erfindungsgemäße Zusammensetzungen, enthaltend jeweils eine individualisierte Verbindung der Formel I und einen Mischungspartner, bzw. eine Mischungspartnerkombination sind in der folgenden Tabelle B angegeben.

Einer weitere Ausgestaltung der Erfindung betrifft die in der Tabelle B aufgeführten Zusammensetzungen B-1 bis B-1227, wobei jeweils eine Zeile der Tabelle B einer herbiziden Zusammensetzung entspricht, umfassend eine in der vorliegenden Beschreibung individualisierten Verbindungen der Formel I (Komponente 1) und den jeweils in der betreffenden Zeile angegebenen weiteren Wirkstoff aus den Gruppen b1) bis b15) und/oder Safener C (Komponente 2). Die Wirkstoffe in den beschriebenen Zusammensetzungen liegen jeweils vorzugsweise in synergistisch wirksamen Mengen vor.

**Tabelle B:**

| | Herbizid(e) B | Safener C |
|---|---|---|
| B-1 | Clodinafop-propargyl | - |
| B-2 | Cycloxydim | - |
| B-3 | Cyhalofop-butyl | - |
| B-4 | Fenoxaprop-P-ethyl | - |
| B-5 | Pinoxaden | - |
| B-6 | Profoxydim | - |
| B-7 | Tepraloxydim | - |
| B-8 | Tralkoxydim | - |
| B-9 | Esprocarb | - |
| B-10 | Prosulfocarb | - |
| B-11 | Thiobencarb | - |
| B-12 | Triallat | - |
| B-13 | Bensulfuron-methyl | - |
| B-14 | Bispyribac-natrium | - |
| B-15 | Cyclosulfamuron | - |
| B-16 | Flumetsulam | - |
| B-17 | Flupyrsulfuron-methyl-natrium | - |
| B-18 | Foramsulfuron | - |
| B-19 | Imazamox | - |
| B-20 | Imazapic | - |
| B-21 | Imazapyr | - |
| B-22 | Imazaquin | - |
| B-23 | Imazethapyr | - |
| B-24 | Imazosulfuron | - |
| B-25 | lodosulfuron-methyl-natrium | - |
| B-26 | Mesosulfuron | - |
| B-27 | Nicosulfuron | - |
| B-28 | Penoxsulam | - |
| B-29 | Propoxycarbazon-natrium | - |
| B-30 | Pyrazosulfuron-ethyl | - |
| B-31 | Pyroxsulam | - |
| B-32 | Rimsulfuron | - |
| B-33 | Sulfosulfuron | - |
| B-34 | Thiencarbazon-methyl | - |
| B-35 | Tritosulfuron | - |
| B-36 | 2,4-D und seine Salze und Ester | - |
| B-37 | Aminopyralid und seine Salze und Ester | - |
| B-38 | Clopyralid und seine Salze und Ester | - |
| B-39 | Dicamba und seine Salze und Ester | - |
| B-40 | Fluroxypyr-meptyl | - |
| B-41 | Quinclorac | - |
| B-42 | Quinmerac | - |
| B-43 | H-9 | - |
| B-44 | Diflufenzopyr | - |
| B-45 | Diflufenzopyr-natrium | - |
| B-46 | Clomazon | - |
| B-47 | Diflufenican | - |
| B-48 | Flurochloridon | - |
| B-49 | Isoxaflutol | - |
| B-50 | Mesotrion | - |
| B-51 | Picoli nafen | - |
| B-52 | Sulcotrion | - |
| B-53 | Tefuryltrion | - |
| B-54 | Tembotrion | - |
| B-55 | Topramezon | - |
| B-56 | HHH-7 | - |
| B-57 | Atrazin | - |
| B-58 | Diuron | - |
| B-59 | Fluometuron | - |
| B-60 | Hexazinon | - |
| B-61 | Isoproturon | - |
| B-62 | Metribuzin | - |
| B-63 | Propanil | - |
| B-64 | Terbuthylazin | - |
| B-65 | Paraquat-dichlorid | - |
| B-66 | Flumioxazin | - |
| B-67 | Oxyfluorfen | - |
| B-68 | Sulfentrazon | - |
| B-69 | H-1 | - |
| B-70 | H-2 | - |
| B-71 | Glyphosat | - |
| B-72 | Glyphosat-isopropylammonium | - |
| B-73 | Glyphosat-trimesium (sulfosate) | - |
| B-74 | Glufosinat | - |
| B-75 | Glufosinat-ammonium | - |
| B-76 | Pendimethalin | - |
| B-77 | Trifluralin | - |
| B-78 | Acetochlor | - |
| B-79 | Cafenstrol | - |
| B-80 | Dimethenamid-P | - |
| B-81 | Fentrazamid | - |
| B-82 | Flufenacet | - |
| B-83 | Mefenacet | - |
| B-84 | Metazachlor | - |
| B-85 | Metolachlor-S | - |
| B-86 | Pyroxasulfon | - |
| B-87 | Isoxaben | - |
| B-88 | Dymron | - |
| B-89 | Indanofan | - |
| B-90 | Oxaziclomefon | - |
| B-91 | Triaziflam | - |
| B-92 | Atrazin + H-1 | - |
| B-93 | Atrazin + Glyphosat | - |
| B-94 | Atrazin + Mesotrion | - |
| B-95 | Atrazin + Nicosulfuron | - |
| B-96 | Atrazin + Tembotrion | - |
| B-97 | Atrazin + Topramezone | - |
| B-98 | Clomazon + Glyphosat | - |
| B-99 | Diflufenican + Clodinafop-propargyl | - |
| B-100 | Diflufenican + Fenoxaprop-P-ethyl | - |
| B-101 | Diflufenican + Flupyrsulfuron-methyl-natrium | - |
| B-102 | Diflufenican + Glyphosat | - |
| B-103 | Diflufenican + Mesosulfuron-methyl | - |
| B-104 | Diflufenican + Pinoxaden | - |
| B-105 | Diflufenican + Pyroxsulam | - |
| B-106 | Flumetsulam + Glyphosat | - |
| B-107 | Flumioxazin + Glyphosat | - |
| B-108 | Imazapic + Glyphosat | - |
| B-109 | Imazethapyr + Glyphosat | - |
| B-110 | Isoxaflutol + H-1 | - |
| B-111 | Isoxaflutol + Glyphosat | - |
| B-112 | Metazachlor + H-1 | - |
| B-113 | Metazachlor + Glyphosat | - |
| B-114 | Metazachlor + Mesotrion | - |
| B-115 | Metazachlor + Nicosulfuron | - |
| B-116 | Metazachlor + Terbuthylazin | - |
| B-117 | Metazachlor + Topramezon | - |
| B-118 | Metribuzin + Glyphosat | - |
| B-119 | Pendimethalin + H-1 | - |
| B-120 | Pendimethalin + Clodinafop-propargyl | - |
| B-121 | Pendimethalin + Fenoxaprop-P-ethyl | - |
| B-122 | Pendimethalin + Flupyrsulfuron-methyl-natrium | - |
| B-123 | Pendimethalin + Glyphosat | - |
| B-124 | Pendimethalin + Mesosulfuron-methyl | - |
| B-125 | Pendimethalin + Mesotrione | - |
| B-126 | Pendimethalin + Nicosulfuron | - |
| B-127 | Pendimethalin + Pinoxaden | - |
| B-128 | Pendimethalin + Pyroxsulam | - |
| B-129 | Pendimethalin + Tembotrione | - |
| B-130 | Pendimethalin + Topramezone | - |
| B-131 | Pyroxasulfon + Tembotrion | - |
| B-132 | Pyroxasulfon + Topramezon | - |
| B-133 | Sulfentrazon + Glyphosat | - |
| B-134 | Terbuthylazin + H-1 | - |
| B-135 | Terbuthylazin + Foramsulfuron | - |
| B-136 | Terbuthylazin + Glyphosat | - |
| B-137 | Terbuthylazin + Mesotrion | - |
| B-138 | Terbuthylazin + Nicosulfuron | - |
| B-139 | Terbuthylazin + Tembotrion | - |
| B-140 | Terbuthylazin + Topramezon | - |
| B-141 | Trifluralin + Glyphosat | - |
| B-142 | - | Benoxacor |
| B-143 | - | Cloquintocet |
| B-144 | - | Cyprosulfamid |
| B-145 | - | Dichlormid |
| B-146 | - | Fenchlorazol |
| B-147 | - | Isoxadifen |
| B-148 | - | Mefenpyr |
| B-149 | - | H-11 |
| B-150 | - | H-12 |
| B-151 | Clodinafop-propargyl | Benoxacor |
| B-152 | Cycloxydim | Benoxacor |
| B-153 | Cyhalofop-butyl | Benoxacor |
| B-154 | Fenoxaprop-P-ethyl | Benoxacor |
| B-155 | Pinoxaden | Benoxacor |
| B-156 | Profoxydim | Benoxacor |
| B-157 | Tepraloxydim | Benoxacor |
| B-158 | Tralkoxydim | Benoxacor |
| B-159 | Esprocarb | Benoxacor |
| B-160 | Prosulfocarb | Benoxacor |
| B-161 | Thiobencarb | Benoxacor |
| B-162 | Triallat | Benoxacor |
| B-163 | Bensulfuron-methyl | Benoxacor |
| B-164 | Bispyribac-natrium | Benoxacor |
| B-165 | Cyclosulfamuron | Benoxacor |
| B-166 | Flumetsulam | Benoxacor |
| B-167 | Flupyrsulfuron-methyl-natrium | Benoxacor |
| B-168 | Foramsulfuron | Benoxacor |
| B-169 | Imazamox | Benoxacor |
| B-170 | Imazapic | Benoxacor |
| B-171 | Imazapyr | Benoxacor |
| B-172 | Imazaquin | Benoxacor |
| B-173 | Imazethapyr | Benoxacor |
| B-174 | Imazosulfuron | Benoxacor |
| B-175 | Iodosulfuron-methyl-natrium | Benoxacor |
| B-176 | Mesosulfuron | Benoxacor |
| B-177 | Nicosulfuron | Benoxacor |
| B-178 | Penoxsulam | Benoxacor |
| B-179 | Propoxycarbazon-natrium | Benoxacor |
| B-180 | Pyrazosulfuron-ethyl | Benoxacor |
| B-181 | Pyroxsulam | Benoxacor |
| B-182 | Rimsulfuron | Benoxacor |
| B-183 | Sulfosulfuron | Benoxacor |
| B-184 | Thiencarbazon-methyl | Benoxacor |
| B-185 | Tritosulfuron | Benoxacor |
| B-186 | 2,4-D und seine Salze und Ester | Benoxacor |
| B-187 | Aminopyralid und seine Salze und Ester | Benoxacor |
| B-188 | Clopyralid und seine Salze und Ester | Benoxacor |
| B-189 | Dicamba und seine Salze und Ester | Benoxacor |
| B-190 | Fluroxypyr-meptyl | Benoxacor |
| B-191 | Quinclorac | Benoxacor |
| B-192 | Quinmerac | Benoxacor |
| B-193 | H-9 | Benoxacor |
| B-194 | Diflufenzopyr | Benoxacor |
| B-195 | Diflufenzopyr-natrium | Benoxacor |
| B-196 | Clomazon | Benoxacor |
| B-197 | Diflufenican | Benoxacor |
| B-198 | Flurochloridon | Benoxacor |
| B-199 | lsoxaflutol | Benoxacor |
| B-200 | Mesotrion | Benoxacor |
| B-201 | Picolinafen | Benoxacor |
| B-202 | Sulcotrion | Benoxacor |
| B-203 | Tefuryltrion | Benoxacor |
| B-204 | Tembotrion | Benoxacor |
| B-205 | Topramezon | Benoxacor |
| B-206 | H-7 | Benoxacor |
| B-207 | Atrazin | Benoxacor |
| B-208 | Diuron | Benoxacor |
| B-209 | Fluometuron | Benoxacor |
| B-210 | Hexazinon | Benoxacor |
| B-211 | Isoproturon | Benoxacor |
| B-212 | Metribuzin | Benoxacor |
| B-213 | Propanil | Benoxacor |
| B-214 | Terbuthylazin | Benoxacor |
| B-215 | Paraquat-dichlorid | Benoxacor |
| B-216 | Flumioxazin | Benoxacor |
| B-217 | Oxyfluorfen | Benoxacor |
| B-218 | Sulfentrazon | Benoxacor |
| B-219 | H-1 | Benoxacor |
| B-220 | H-2 | Benoxacor |
| B-221 | Glyphosat | Benoxacor |
| B-222 | Glyphosat-isopropylammonium | Benoxacor |
| B-223 | Glyphosat-trimesium (sulfosate) | Benoxacor |
| B-224 | Glufosinat | Benoxacor |
| B-225 | Glufosinat-ammonium | Benoxacor |
| B-226 | Pendimethalin | Benoxacor |
| B-227 | Trifluralin | Benoxacor |
| B-228 | Acetochlor | Benoxacor |
| B-229 | Cafenstrol | Benoxacor |
| B-230 | Dimethenamid-P | Benoxacor |
| B-231 | Fentrazamid | Benoxacor |
| B-232 | Flufenacet | Benoxacor |
| B-233 | Mefenacet | Benoxacor |
| B-234 | Metazachlor | Benoxacor |
| B-235 | Metolachlor-S | Benoxacor |
| B-236 | Pyroxasulfon | Benoxacor |
| B-237 | Isoxaben | Benoxacor |
| B-238 | Dymron | Benoxacor |
| B-239 | Indanofan | Benoxacor |
| B-240 | Oxaziclomefon | Benoxacor |
| B-241 | Triaziflam | Benoxacor |
| B-242 | Atrazin + H-1 | Benoxacor |
| B-243 | Atrazin + Glyphosat | Benoxacor |
| B-244 | Atrazin + Mesotrion | Benoxacor |
| B-245 | Atrazin + Nicosulfuron | Benoxacor |
| B-246 | Atrazin + Tembotrion | Benoxacor |
| B-247 | Atrazin + Topramezone | Benoxacor |
| B-248 | Clomazon + Glyphosat | Benoxacor |
| B-249 | Diflufenican + Clodinafop-propargyl | Benoxacor |
| B-250 | Diflufenican + Fenoxaprop-P-ethyl | Benoxacor |
| B-251 | Diflufenican + Flupyrsulfuron-methyl-natrium | Benoxacor |
| B-252 | Diflufenican + Glyphosat | Benoxacor |
| B-253 | Diflufenican + Mesosulfuron-methyl | Benoxacor |
| B-254 | Diflufenican + Pinoxaden | Benoxacor |
| B-255 | Diflufenican + Pyroxsulam | Benoxacor |
| B-256 | Flumetsulam + Glyphosat | Benoxacor |
| B-257 | Flumioxazin + Glyphosat | Benoxacor |
| B-258 | Imazapic + Glyphosat | Benoxacor |
| B-259 | Imazethapyr + Glyphosat | Benoxacor |
| B-260 | Isoxaflutol + H-1 | Benoxacor |
| B-261 | Isoxaflutol + Glyphosat | Benoxacor |
| B-262 | Metazachlor + H-1 | Benoxacor |
| B-263 | Metazachlor + Glyphosat | Benoxacor |
| B-264 | Metazachlor + Mesotrion | Benoxacor |
| B-265 | Metazachlor + Nicosulfuron | Benoxacor |
| B-266 | Metazachlor + Terbuthylazin | Benoxacor |
| B-267 | Metazachlor + Topramezon | Benoxacor |
| B-268 | Metribuzin + Glyphosat | Benoxacor |
| B-269 | Pendimethalin + H-1 | Benoxacor |
| B-270 | Pendimethalin + Clodinafop-propargyl | Benoxacor |
| B-271 | Pendimethalin + Fenoxaprop-P-ethyl | Benoxacor |
| B-272 | Pendimethalin + Flupyrsulfuron-methyl-natrium | Benoxacor |
| B-273 | Pendimethalin + Glyphosat | Benoxacor |
| B-274 | Pendimethalin + Mesosulfuron-methyl | Benoxacor |
| B-275 | Pendimethalin + Mesotrione | Benoxacor |
| B-276 | Pendimethalin + Nicosulfuron | Benoxacor |
| B-277 | Pendimethalin + Pinoxaden | Benoxacor |
| B-278 | Pendimethalin + Pyroxsulam | Benoxacor |
| B-279 | Pendimethalin + Tembotrione | Benoxacor |
| B-280 | Pendimethalin + Topramezone | Benoxacor |
| B-281 | Pyroxasulfon + Tembotrion | Benoxacor |
| B-282 | Pyroxasulfon + Topramezon | Benoxacor |
| B-283 | Sulfentrazon + Glyphosat | Benoxacor |
| B-284 | Terbuthylazin + H-1 | Benoxacor |
| B-285 | Terbuthylazin + Foramsulfuron | Benoxacor |
| B-286 | Terbuthylazin + Glyphosat | Benoxacor |
| B-287 | Terbuthylazin + Mesotrion | Benoxacor |
| B-288 | Terbuthylazin + Nicosulfuron | Benoxacor |
| B-289 | Terbuthylazin + Tembotrion | Benoxacor |
| B-290 | Terbuthylazin + Topramezon | Benoxacor |
| B-291 | Trifluralin + Glyphosat | Benoxacor |
| B-292 | Clodinafop-propargyl | Cloquintocet |
| B-293 | Cycloxydim | Cloquintocet |
| B-294 | Cyhalofop-butyl | Cloquintocet |
| B-295 | Fenoxaprop-P-ethyl | Cloquintocet |
| B-296 | Pinoxaden | Cloquintocet |
| B-297 | Profoxydim | Cloquintocet |
| B-298 | Tepraloxydim | Cloquintocet |
| B-299 | Tralkoxydim | Cloquintocet |
| B-300 | Esprocarb | Cloquintocet |
| B-301 | Prosulfocarb | Cloquintocet |
| B-302 | Thiobencarb | Cloquintocet |
| B-303 | Triallat | Cloquintocet |
| B-304 | Bensulfuron-methyl | Cloquintocet |
| B-305 | Bispyribac-natrium | Cloquintocet |
| B-306 | Cyclosulfamuron | Cloquintocet |
| B-307 | Flumetsulam | Cloquintocet |
| B-308 | Flupyrsulfuron-methyl-natrium | Cloquintocet |
| B-309 | Foramsulfuron | Cloquintocet |
| B-310 | Imazamox | Cloquintocet |
| B-311 | Imazapic | Cloquintocet |
| B-312 | Imazapyr | Cloquintocet |
| B-313 | Imazaquin | Cloquintocet |
| B-314 | Imazethapyr | Cloquintocet |
| B-315 | Imazosulfuron | Cloquintocet |
| B-316 | Iodosulfuron-methyl-natrium | Cloquintocet |
| B-317 | Mesosulfuron | Cloquintocet |
| B-318 | Nicosulfuron | Cloquintocet |
| B-319 | Penoxsulam | Cloquintocet |
| B-320 | Propoxycarbazon-natrium | Cloquintocet |
| B-321 | Pyrazosulfuron-ethyl | Cloquintocet |
| B-322 | Pyroxsulam | Cloquintocet |
| B-323 | Rimsulfuron | Cloquintocet |
| B-324 | Sulfosulfuron | Cloquintocet |
| B-325 | Thiencarbazon-methyl | Cloquintocet |
| B-326 | Tritosulfuron | Cloquintocet |
| B-327 | 2,4-D und seine Salze und Ester | Cloquintocet |
| B-328 | Aminopyralid und seine Salze und Ester | Cloquintocet |
| B-329 | Clopyralid und seine Salze und Ester | Cloquintocet |
| B-330 | Dicamba und seine Salze und Ester | Cloquintocet |
| B-331 | Fluroxypyr-meptyl | Cloquintocet |
| B-332 | Quinclorac | Cloquintocet |
| B-333 | Quinmerac | Cloquintocet |
| B-334 | H-9 | Cloquintocet |
| B-335 | Diflufenzopyr | Cloquintocet |
| B-336 | Diflufenzopyr-natrium | Cloquintocet |
| B-337 | Clomazon | Cloquintocet |
| B-338 | Diflufenican | Cloquintocet |
| B-339 | Flurochloridon | Cloquintocet |
| B-340 | lsoxaflutol | Cloquintocet |
| B-341 | Mesotrion | Cloquintocet |
| B-342 | Picolinafen | Cloquintocet |
| B-343 | Sulcotrion | Cloquintocet |
| B-344 | Tefuryltrion | Cloquintocet |
| B-345 | Tembotrion | Cloquintocet |
| B-346 | Topramezon | Cloquintocet |
| B-347 | H-7 | Cloquintocet |
| B-348 | Atrazin | Cloquintocet |
| B-349 | Diuron | Cloquintocet |
| B-350 | Fluometuron | Cloquintocet |
| B-351 | Hexazinon | Cloquintocet |
| B-352 | Isoproturon | Cloquintocet |
| B-353 | Metribuzin | Cloquintocet |
| B-354 | Propanil | Cloquintocet |
| B-355 | Terbuthylazin | Cloquintocet |
| B-356 | Paraquat-dichlorid | Cloquintocet |
| B-357 | Flumioxazin | Cloquintocet |
| B-358 | Oxyfluorfen | Cloquintocet |
| B-359 | Sulfentrazon | Cloquintocet |
| B-360 | H-1 | Cloquintocet |
| B-361 | H-2 | Cloquintocet |
| B-362 | Glyphosat | Cloquintocet |
| B-363 | Glyphosat-isopropylammonium | Cloquintocet |
| B-364 | Glyphosat-trimesium (sulfosate) | Cloquintocet |
| B-365 | Glufosinat | Cloquintocet |
| B-366 | Glufosinat-ammonium | Cloquintocet |
| B-367 | Pendimethalin | Cloquintocet |
| B-368 | Trifluralin | Cloquintocet |
| B-369 | Acetochlor | Cloquintocet |
| B-370 | Cafenstrol | Cloquintocet |
| B-371 | Dimethenamid-P | Cloquintocet |
| B-372 | Fentrazamid | Cloquintocet |
| B-373 | Flufenacet | Cloquintocet |
| B-374 | Mefenacet | Cloquintocet |
| B-375 | Metazachlor | Cloquintocet |
| B-376 | Metolachlor-S | Cloquintocet |
| B-377 | Pyroxasulfon | Cloquintocet |
| B-378 | Isoxaben | Cloquintocet |
| B-379 | Dymron | Cloquintocet |
| B-380 | Indanofan | Cloquintocet |
| B-381 | Oxaziclomefon | Cloquintocet |
| B-382 | Triaziflam | Cloquintocet |
| B-383 | Atrazin + H-1 | Cloquintocet |
| B-384 | Atrazin + Glyphosat | Cloquintocet |
| B-385 | Atrazin + Mesotrion | Cloquintocet |
| B-386 | Atrazin + Nicosulfuron | Cloquintocet |
| B-387 | Atrazin + Tembotrion | Cloquintocet |
| B-388 | Atrazin + Topramezone | Cloq uintocet |
| B-389 | Clomazon + Glyphosat | Cloq uintocet |
| B-390 | Diflufenican + Clodinafop-propargyl | Cloquintocet |
| B-391 | Diflufenican + Fenoxaprop-P-ethyl | Cloquintocet |
| B-392 | Diflufenican + Flupyrsulfuron-methyl-natrium | Cloquintocet |
| B-393 | Diflufenican + Glyphosat | Cloquintocet |
| B-394 | Diflufenican + Mesosulfuron-methyl | Cloquintocet |
| B-395 | Diflufenican + Pinoxaden | Cloquintocet |
| B-396 | Diflufenican + Pyroxsulam | Cloquintocet |
| B-397 | Flumetsulam + Glyphosat | Cloquintocet |
| B-398 | Flumioxazin + Glyphosat | Cloquintocet |
| B-399 | Imazapic + Glyphosat | Cloquintocet |
| B-400 | Imazethapyr + Glyphosat | Cloquintocet |
| B-401 | Isoxaflutol + H-1 | Cloquintocet |
| B-402 | Isoxaflutol + Glyphosat | Cloquintocet |
| B-403 | Metazachlor + H-1 | Cloquintocet |
| B-404 | Metazachlor + Glyphosat | Cloquintocet |
| B-405 | Metazachlor + Mesotrion | Cloquintocet |
| B-406 | Metazachlor + Nicosulfuron | Cloquintocet |
| B-407 | Metazachlor + Terbuthylazin | Cloquintocet |
| B-408 | Metazachlor + Topramezon | Cloquintocet |
| B-409 | Metribuzin + Glyphosat | Cloquintocet |
| B-410 | Pendimethalin + H-1 | Cloquintocet |
| B-411 | Pendimethalin + Clodinafop-propargyl | Cloquintocet |
| B-412 | Pendimethalin + Fenoxaprop-P-ethyl | Cloquintocet |
| B-413 | Pendimethalin + Flupyrsulfuron-methyl-natrium | Cloquintocet |
| B-414 | Pendimethalin + Glyphosat | Cloquintocet |
| B-415 | Pendimethalin + Mesosulfuron-methyl | Cloquintocet |
| B-416 | Pendimethalin + Mesotrione | Cloquintocet |
| B-417 | Pendimethalin + Nicosulfuron | Cloquintocet |
| B-418 | Pendimethalin + Pinoxaden | Cloquintocet |
| B-419 | Pendimethalin + Pyroxsulam | Cloquintocet |
| B-420 | Pendimethalin + Tembotrione | Cloquintocet |
| B-421 | Pendimethalin + Topramezone | Cloquintocet |
| B-422 | Pyroxasulfon + Tembotrion | Cloquintocet |
| B-423 | Pyroxasulfon + Topramezon | Cloquintocet |
| B-424 | Sulfentrazon + Glyphosat | Cloquintocet |
| B-425 | Terbuthylazin + H-1 | Cloquintocet |
| B-426 | Terbuthylazin + Foramsulfuron | Cloquintocet |
| B-427 | Terbuthylazin + Glyphosat | Cloquintocet |
| B-428 | Terbuthylazin + Mesotrion | Cloquintocet |
| B-429 | Terbuthylazin + Nicosulfuron | Cloquintocet |
| B-430 | Terbuthylazin + Tembotrion | Cloquintocet |
| B-431 | Terbuthylazin + Topramezon | Cloquintocet |
| B-432 | Trifluralin + Glyphosat | Cloquintocet |
| B-433 | Clodinafop-propargyl | Dichlormid |
| B-434 | Cycloxydim | Dichlormid |
| B-435 | Cyhalofop-butyl | Dichlormid |
| B-436 | Fenoxaprop-P-ethyl | Dichlormid |
| B-437 | Pinoxaden | Dichlormid |
| B-438 | Profoxydim | Dichlormid |
| B-439 | Tepraloxydim | Dichlormid |
| B-440 | Tralkoxydim | Dichlormid |
| B-441 | Esprocarb | Dichlormid |
| B-442 | Prosulfocarb | Dichlormid |
| B-443 | Thiobencarb | Dichlormid |
| B-444 | Triallat | Dichlormid |
| B-445 | Bensulfuron-methyl | Dichlormid |
| B-446 | Bispyribac-natrium | Dichlormid |
| B-447 | Cyclosulfamuron | Dichlormid |
| B-448 | Flumetsulam | Dichlormid |
| B-449 | Flupyrsulfuron-methyl-natrium | Dichlormid |
| B-450 | Foramsulfuron | Dichlormid |
| B-451 | Imazamox | Dichlormid |
| B-452 | Imazapic | Dichlormid |
| B-453 | Imazapyr | Dichlormid |
| B-454 | Imazaquin | Dichlormid |
| B-455 | Imazethapyr | Dichlormid |
| B-456 | Imazosulfuron | Dichlormid |
| B-457 | Iodosulfuron-methyl-natrium | Dichlormid |
| B-458 | Mesosulfuron | Dichlormid |
| B-459 | Nicosulfuron | Dichlormid |
| B-460 | Penoxsulam | Dichlormid |
| B-461 | Propoxycarbazon-natrium | Dichlormid |
| B-462 | Pyrazosulfuron-ethyl | Dichlormid |
| B-463 | Pyroxsulam | Dichlormid |
| B-464 | Rimsulfuron | Dichlormid |
| B-465 | Sulfosulfuron | Dichlormid |
| B-466 | Thiencarbazon-methyl | Dichlormid |
| B-467 | Tritosulfuron | Dichlormid |
| B-468 | 2,4-D und seine Salze und Ester | Dichlormid |
| B-469 | Aminopyralid und seine Salze und Ester | Dichlormid |
| B-470 | Clopyralid und seine Salze und Ester | Dichlormid |
| B-471 | Dicamba und seine Salze und Ester | Dichlormid |
| B-472 | Fluroxypyr-meptyl | Dichlormid |
| B-473 | Quinclorac | Dichlormid |
| B-474 | Quinmerac | Dichlormid |
| B-475 | H-9 | Dichlormid |
| B-476 | Diflufenzopyr | Dichlormid |
| B-477 | Diflufenzopyr-natrium | Dichlormid |
| B-478 | Clomazon | Dichlormid |
| B-479 | Diflufenican | Dichlormid |
| B-480 | Flurochloridon | Dichlormid |
| B-481 | Isoxaflutol | Dichlormid |
| B-482 | Mesotrion | Dichlormid |
| B-483 | Picolinafen | Dichlormid |
| B-484 | Sulcotrion | Dichlormid |
| B-485 | Tefuryltrion | Dichlormid |
| B-486 | Tembotrion | Dichlormid |
| B-487 | Topramezon | Dichlormid |
| B-488 | H-7 | Dichlormid |
| B-489 | Atrazin | Dichlormid |
| B-490 | Diuron | Dichlormid |
| B-491 | Fluometuron | Dichlormid |
| B-492 | Hexazinon | Dichlormid |
| B-493 | Isoproturon | Dichlormid |
| B-494 | Metribuzin | Dichlormid |
| B-495 | Propanil | Dichlormid |
| B-496 | Terbuthylazin | Dichlormid |
| B-497 | Paraquat-dichlorid | Dichlormid |
| B-498 | Flumioxazin | Dichlormid |
| B-499 | Oxyfluorfen | Dichlormid |
| B-500 | Sulfentrazon | Dichlormid |
| B-501 | H-1 | Dichlormid |
| B-502 | H-2 | Dichlormid |
| B-503 | Glyphosat | Dichlormid |
| B-504 | Glyphosat-isopropylammonium | Dichlormid |
| B-505 | Glyphosat-trimesium (sulfosate) | Dichlormid |
| B-506 | Glufosinat | Dichlormid |
| B-507 | Glufosinat-ammonium | Dichlormid |
| B-508 | Pendimethalin | Dichlormid |
| B-509 | Trifluralin | Dichlormid |
| B-510 | Acetochlor | Dichlormid |
| B-511 | Cafenstrol | Dichlormid |
| B-512 | Dimethenamid-P | Dichlormid |
| B-513 | Fentrazamid | Dichlormid |
| B-514 | Flufenacet | Dichlormid |
| B-515 | Mefenacet | Dichlormid |
| B-516 | Metazachlor | Dichlormid |
| B-517 | Metolachlor-S | Dichlormid |
| B-518 | Pyroxasulfon | Dichlormid |
| B-519 | Isoxaben | Dichlormid |
| B-520 | Dymron | Dichlormid |
| B-521 | Indanofan | Dichlormid |
| B-522 | Oxaziclomefon | Dichlormid |
| B-523 | Triaziflam | Dichlormid |
| B-524 | Atrazin + H-1 | Dichlormid |
| B-525 | Atrazin + Glyphosat | Dichlormid |
| B-526 | Atrazin + Mesotrion | Dichlormid |
| B-527 | Atrazin + Nicosulfuron | Dichlormid |
| B-528 | Atrazin + Tembotrion | Dichlormid |
| B-529 | Atrazin + Topramezone | Dichlormid |
| B-530 | Clomazon + Glyphosat | Dichlormid |
| B-531 | Diflufenican + Clodinafop-propargyl | Dichlormid |
| B-532 | Diflufenican + Fenoxaprop-P-ethyl | Dichlormid |
| B-533 | Diflufenican + Flupyrsulfuron-methyl-natrium | Dichlormid |
| B-534 | Diflufenican + Glyphosat | Dichlormid |
| B-535 | Diflufenican + Mesosulfuron-methyl | Dichlormid |
| B-536 | Diflufenican + Pinoxaden | Dichlormid |
| B-537 | Diflufenican + Pyroxsulam | Dichlormid |
| B-538 | Flumetsulam + Glyphosat | Dichlormid |
| B-539 | Flumioxazin + Glyphosat | Dichlormid |
| B-540 | Imazapic + Glyphosat | Dichlormid |
| B-541 | Imazethapyr + Glyphosat | Dichlormid |
| B-542 | Isoxaflutol + H-1 | Dichlormid |
| B-543 | Isoxaflutol + Glyphosat | Dichlormid |
| B-544 | Metazachlor + H-1 | Dichlormid |
| B-545 | Metazachlor + Glyphosat | Dichlormid |
| B-546 | Metazachlor + Mesotrion | Dichlormid |
| B-547 | Metazachlor + Nicosulfuron | Dichlormid |
| B-548 | Metazachlor + Terbuthylazin | Dichlormid |
| B-549 | Metazachlor + Topramezon | Dichlormid |
| B-550 | Metribuzin + Glyphosat | Dichlormid |
| B-551 | Pendimethalin + H-1 | Dichlormid |
| B-552 | Pendimethalin + Clodinafop-propargyl | Dichlormid |
| B-553 | Pendimethalin + Fenoxaprop-P-ethyl | Dichlormid |
| B-554 | Pendimethalin + Flupyrsulfuron-methyl-natrium | Dichlormid |
| B-555 | Pendimethalin + Glyphosat | Dichlormid |
| B-556 | Pendimethalin + Mesosulfuron-methyl | Dichlormid |
| B-557 | Pendimethalin + Mesotrione | Dichlormid |
| B-558 | Pendimethalin + Nicosulfuron | Dichlormid |
| B-559 | Pendimethalin + Pinoxaden | Dichlormid |
| B-560 | Pendimethalin + Pyroxsulam | Dichlormid |
| B-561 | Pendimethalin + Tembotrione | Dichlormid |
| B-562 | Pendimethalin + Topramezone | Dichlormid |
| B-563 | Pyroxasulfon + Tembotrion | Dichlormid |
| B-564 | Pyroxasulfon + Topramezon | Dichlormid |
| B-565 | Sulfentrazon + Glyphosat | Dichlormid |
| B-566 | Terbuthylazin + H-1 | Dichlormid |
| B-567 | Terbuthylazin + Foramsulfuron | Dichlormid |
| B-568 | Terbuthylazin + Glyphosat | Dichlormid |
| B-569 | Terbuthylazin + Mesotrion | Dichlormid |
| B-570 | Terbuthylazin + Nicosulfuron | Dichlormid |
| B-571 | Terbuthylazin + Tembotrion | Dichlormid |
| B-572 | Terbuthylazin + Topramezon | Dichlormid |
| B-573 | Trifluralin + Glyphosat | Dichlormid |
| B-574 | Clodinafop-propargyl | Fenchlorazol |
| B-575 | Cycloxydim | Fenchlorazol |
| B-576 | Cyhalofop-butyl | Fenchlorazol |
| B-577 | Fenoxaprop-P-ethyl | Fenchlorazol |
| B-578 | Pinoxaden | Fenchlorazol |
| B-579 | Profoxydim | Fenchlorazol |
| B-580 | Tepraloxydim | Fenchlorazol |
| B-581 | Tralkoxydim | Fenchlorazol |
| B-582 | Esprocarb | Fenchlorazol |
| B-583 | Prosulfocarb | Fenchlorazol |
| B-584 | Thiobencarb | Fenchlorazol |
| B-585 | Triallat | Fenchlorazol |
| B-586 | Bensulfuron-methyl | Fenchlorazol |
| B-587 | Bispyribac-natrium | Fenchlorazol |
| B-588 | Cyclosulfamuron | Fenchlorazol |
| B-589 | Flumetsulam | Fenchlorazol |
| B-590 | Flupyrsulfuron-methyl-natrium | Fenchlorazol |
| B-591 | Foramsulfuron | Fenchlorazol |
| B-592 | Imazamox | Fenchlorazol |
| B-593 | Imazapic | Fenchlorazol |
| B-594 | Imazapyr | Fenchlorazol |
| B-595 | Imazaquin | Fenchlorazol |
| B-596 | Imazethapyr | Fenchlorazol |
| B-597 | Imazosulfuron | Fenchlorazol |
| B-598 | Iodosulfuron-methyl-natrium | Fenchlorazol |
| B-599 | Mesosulfuron | Fenchlorazol |
| B-600 | Nicosulfuron | Fenchlorazol |
| B-601 | Penoxsulam | Fenchlorazol |
| B-602 | Propoxycarbazon-natrium | Fenchlorazol |
| B-603 | Pyrazosulfuron-ethyl | Fenchlorazol |
| B-604 | Pyroxsulam | Fenchlorazol |
| B-605 | Rimsulfuron | Fenchlorazol |
| B-606 | Sulfosulfuron | Fenchlorazol |
| B-607 | Thiencarbazon-methyl | Fenchlorazol |
| B-608 | Tritosulfuron | Fenchlorazol |
| B-609 | 2,4-D und seine Salze und Ester | Fenchlorazol |
| B-610 | Aminopyralid und seine Salze und Ester | Fenchlorazol |
| B-611 | Clopyralid und seine Salze und Ester | Fenchlorazol |
| B-612 | Dicamba und seine Salze und Ester | Fenchlorazol |
| B-613 | Fluroxypyr-meptyl | Fenchlorazol |
| B-614 | Quinclorac | Fenchlorazol |
| B-615 | Quinmerac | Fenchlorazol |
| B-616 | H-9 | Fenchlorazol |
| B-617 | Diflufenzopyr | Fenchlorazol |
| B-618 | Diflufenzopyr-natrium | Fenchlorazol |
| B-619 | Clomazon | Fenchlorazol |
| B-620 | Diflufenican | Fenchlorazol |
| B-621 | Flurochloridon | Fenchlorazol |
| B-622 | Isoxaflutol | Fenchlorazol |
| B-623 | Mesotrion | Fenchlorazol |
| B-624 | Picolinafen | Fenchlorazol |
| B-625 | Sulcotrion | Fenchlorazol |
| B-626 | Tefuryltrion | Fenchlorazol |
| B-627 | Tembotrion | Fenchlorazol |
| B-628 | Topramezon | Fenchlorazol |
| B-629 | H-7 | Fenchlorazol |
| B-630 | Atrazin | Fenchlorazol |
| B-631 | Diuron | Fenchlorazol |
| B-632 | Fluometuron | Fenchlorazol |
| B-633 | Hexazinon | Fenchlorazol |
| B-634 | Isoproturon | Fenchlorazol |
| B-635 | Metribuzin | Fenchlorazol |
| B-636 | Propanil | Fenchlorazol |
| B-637 | Terbuthylazin | Fenchlorazol |
| B-638 | Paraquat-dichlorid | Fenchlorazol |
| B-639 | Flumioxazin | Fenchlorazol |
| B-640 | Oxyfluorfen | Fenchlorazol |
| B-641 | Sulfentrazon | Fenchlorazol |
| B-642 | H-1 | Fenchlorazol |
| B-643 | H-2 | Fenchlorazol |
| B-644 | Glyphosat | Fenchlorazol |
| B-645 | Glyphosat-isopropylammonium | Fenchlorazol |
| B-646 | Glyphosat-trimesium (sulfosate) | Fenchlorazol |
| B-647 | Glufosinat | Fenchlorazol |
| B-648 | Glufosinat-ammonium | Fenchlorazol |
| B-649 | Pendimethalin | Fenchlorazol |
| B-650 | Trifluralin | Fenchlorazol |
| B-651 | Acetochlor | Fenchlorazol |
| B-652 | Cafenstrol | Fenchlorazol |
| B-653 | Dimethenamid-P | Fenchlorazol |
| B-654 | Fentrazamid | Fenchlorazol |
| B-655 | Flufenacet | Fenchlorazol |
| B-656 | Mefenacet | Fenchlorazol |
| B-657 | Metazachlor | Fenchlorazol |
| B-658 | Metolachlor-S | Fenchlorazol |
| B-659 | Pyroxasulfon | Fenchlorazol |
| B-660 | Isoxaben | Fenchlorazol |
| B-661 | Dymron | Fenchlorazol |
| B-662 | Indanofan | Fenchlorazol |
| B-663 | Oxaziclomefon | Fenchlorazol |
| B-664 | Triaziflam | Fenchlorazol |
| B-665 | Atrazin + H-1 | Fenchlorazol |
| B-666 | Atrazin + Glyphosat | Fenchlorazol |
| B-667 | Atrazin + Mesotrion | Fenchlorazol |
| B-668 | Atrazin + Nicosulfuron | Fenchlorazol |
| B-669 | Atrazin + Tembotrion | Fenchlorazol |
| B-670 | Atrazin + Topramezone | Fenchlorazol |
| B-671 | Clomazon + Glyphosat | Fenchlorazol |
| B-672 | Diflufenican + Clodinafop-propargyl | Fenchlorazol |
| B-673 | Diflufenican + Fenoxaprop-P-ethyl | Fenchlorazol |
| B-674 | Diflufenican + Flupyrsulfuron-methyl-natrium | Fenchlorazol |
| B-675 | Diflufenican + Glyphosat | Fenchlorazol |
| B-676 | Diflufenican + Mesosulfuron-methyl | Fenchlorazol |
| B-677 | Diflufenican + Pinoxaden | Fenchlorazol |
| B-678 | Diflufenican + Pyroxsulam | Fenchlorazol |
| B-679 | Flumetsulam + Glyphosat | Fenchlorazol |
| B-680 | Flumioxazin + Glyphosat | Fenchlorazol |
| B-681 | Imazapic + Glyphosat | Fenchlorazol |
| B-682 | Imazethapyr + Glyphosat | Fenchlorazol |
| B-683 | Isoxaflutol + H-1 | Fenchlorazol |
| B-684 | Isoxaflutol + Glyphosat | Fenchlorazol |
| B-685 | Metazachlor + H-1 | Fenchlorazol |
| B-686 | Metazachlor + Glyphosat | Fenchlorazol |
| B-687 | Metazachlor + Mesotrion | Fenchlorazol |
| B-688 | Metazachlor + Nicosulfuron | Fenchlorazol |
| B-689 | Metazachlor + Terbuthylazin | Fenchlorazol |
| B-690 | Metazachlor + Topramezon | Fenchlorazol |
| B-691 | Metribuzin + Glyphosat | Fenchlorazol |
| B-692 | Pendimethalin + H-1 | Fenchlorazol |
| B-693 | Pendimethalin + Clodinafop-propargyl | Fenchlorazol |
| B-694 | Pendimethalin + Fenoxaprop-P-ethyl | Fenchlorazol |
| B-695 | Pendimethalin + Flupyrsulfuron-methyl-natrium | Fenchlorazol |
| B-696 | Pendimethalin + Glyphosat | Fenchlorazol |
| B-697 | Pendimethalin + Mesosulfuron-methyl | Fenchlorazol |
| B-698 | Pendimethalin + Mesotrione | Fenchlorazol |
| B-699 | Pendimethalin + Nicosulfuron | Fenchlorazol |
| B-700 | Pendimethalin + Pinoxaden | Fenchlorazol |
| B-701 | Pendimethalin + Pyroxsulam | Fenchlorazol |
| B-702 | Pendimethalin + Tembotrione | Fenchlorazol |
| B-703 | Pendimethalin + Topramezone | Fenchlorazol |
| B-704 | Pyroxasulfon + Tembotrion | Fenchlorazol |
| B-705 | Pyroxasulfon + Topramezon | Fenchlorazol |
| B-706 | Sulfentrazon + Glyphosat | Fenchlorazol |
| B-707 | Terbuthylazin + H-1 | Fenchlorazol |
| B-708 | Terbuthylazin + Foramsulfuron | Fenchlorazol |
| B-709 | Terbuthylazin + Glyphosat | Fenchlorazol |
| B-710 | Terbuthylazin + Mesotrion | Fenchlorazol |
| B-711 | Terbuthylazin + Nicosulfuron | Fenchlorazol |
| B-712 | Terbuthylazin + Tembotrion | Fenchlorazol |
| B-713 | Terbuthylazin + Topramezon | Fenchlorazol |
| B-714 | Trifluralin + Glyphosat | Fenchlorazol |
| B-715 | Clodinafop-propargyl | Isoxadifen |
| B-716 | Cycloxydim | Isoxadifen |
| B-717 | Cyhalofop-butyl | Isoxadifen |
| B-718 | Fenoxaprop-P-ethyl | Isoxadifen |
| B-719 | Pinoxaden | Isoxadifen |
| B-720 | Profoxydim | Isoxadifen |
| B-721 | Tepraloxydim | Isoxadifen |
| B-722 | Tralkoxydim | Isoxadifen |
| B-723 | Esprocarb | Isoxadifen |
| B-724 | Prosulfocarb | Isoxadifen |
| B-725 | Thiobencarb | Isoxadifen |
| B-726 | Triallat | Isoxadifen |
| B-727 | Bensulfuron-methyl | Isoxadifen |
| B-728 | Bispyribac-natrium | Isoxadifen |
| B-729 | Cyclosulfamuron | Isoxadifen |
| B-730 | Flumetsulam | Isoxadifen |
| B-731 | Flupyrsulfuron-methyl-natrium | Isoxadifen |
| B-732 | Foramsulfuron | Isoxadifen |
| B-733 | Imazamox | Isoxadifen |
| B-734 | Imazapic | Isoxadifen |
| B-735 | Imazapyr | Isoxadifen |
| B-736 | Imazaquin | Isoxadifen |
| B-737 | Imazethapyr | Isoxadifen |
| B-738 | Imazosulfuron | Isoxadifen |
| B-739 | lodosulfuron-methyl-natrium | Isoxadifen |
| B-740 | Mesosulfuron | Isoxadifen |
| B-741 | Nicosulfuron | Isoxadifen |
| B-742 | Penoxsulam | Isoxadifen |
| B-743 | Propoxycarbazon-natrium | Isoxadifen |
| B-744 | Pyrazosulfuron-ethyl | Isoxadifen |
| B-745 | Pyroxsulam | Isoxadifen |
| B-746 | Rimsulfuron | Isoxadifen |
| B-747 | Sulfosulfuron | Isoxadifen |
| B-748 | Thiencarbazon-methyl | Isoxadifen |
| B-749 | Tritosulfuron | Isoxadifen |
| B-750 | 2,4-D und seine Salze und Ester | Isoxadifen |
| B-751 | Aminopyralid und seine Salze und Ester | Isoxadifen |
| B-752 | Clopyralid und seine Salze und Ester | Isoxadifen |
| B-753 | Dicamba und seine Salze und Ester | Isoxadifen |
| B-754 | Fluroxypyr-meptyl | Isoxadifen |
| B-755 | Quinclorac | Isoxadifen |
| B-756 | Quinmerac | Isoxadifen |
| B-757 | H-9 | Isoxadifen |
| B-758 | Diflufenzopyr | Isoxadifen |
| B-759 | Diflufenzopyr-natrium | Isoxadifen |
| B-760 | Clomazon | Isoxadifen |
| B-761 | Diflufenican | Isoxadifen |
| B-762 | Flurochloridon | Isoxadifen |
| B-763 | Isoxaflutol | Isoxadifen |
| B-764 | Mesotrion | Isoxadifen |
| B-765 | Picolinafen | Isoxadifen |
| B-766 | Sulcotrion | Isoxadifen |
| B-767 | Tefuryltrion | Isoxadifen |
| B-768 | Tembotrion | Isoxadifen |
| B-769 | Topramezon | Isoxadifen |
| B-770 | H-7 | Isoxadifen |
| B-771 | Atrazin | Isoxadifen |
| B-772 | Diuron | Isoxadifen |
| B-773 | Fluometuron | Isoxadifen |
| B-774 | Hexazinon | Isoxadifen |
| B-775 | Isoproturon | Isoxadifen |
| B-776 | Metribuzin | Isoxadifen |
| B-777 | Propanil | Isoxadifen |
| B-778 | Terbuthylazin | Isoxadifen |
| B-779 | Paraquat-dichlorid | Isoxadifen |
| B-780 | Flumioxazin | Isoxadifen |
| B-781 | Oxyfluorfen | Isoxadifen |
| B-782 | Sulfentrazon | Isoxadifen |
| B-783 | H-1 | Isoxadifen |
| B-784 | H-2 | Isoxadifen |
| B-785 | Glyphosat | Isoxadifen |
| B-786 | Glyphosat-isopropylammonium | Isoxadifen |
| B-787 | Glyphosat-trimesium (sulfosate) | Isoxadifen |
| B-788 | Glufosinat | Isoxadifen |
| B-789 | Glufosinat-ammonium | Isoxadifen |
| B-790 | Pendimethalin | Isoxadifen |
| B-791 | Trifluralin | Isoxadifen |
| B-792 | Acetochlor | Isoxadifen |
| B-793 | Cafenstrol | Isoxadifen |
| B-794 | Dimethenamid-P | Isoxadifen |
| B-795 | Fentrazamid | Isoxadifen |
| B-796 | Flufenacet | Isoxadifen |
| B-797 | Mefenacet | Isoxadifen |
| B-798 | Metazachlor | Isoxadifen |
| B-799 | Metolachlor-S | Isoxadifen |
| B-800 | Pyroxasulfon | Isoxadifen |
| B-801 | Isoxaben | Isoxadifen |
| B-802 | Dymron | Isoxadifen |
| B-803 | Indanofan | Isoxadifen |
| B-804 | Oxaziclomefon | Isoxadifen |
| B-805 | Triaziflam | Isoxadifen |
| B-806 | Atrazin + H-1 | Isoxadifen |
| B-807 | Atrazin + Glyphosat- | Isoxadifen |
| B-808 | Atrazin + Mesotrion | Isoxadifen |
| B-809 | Atrazin + Nicosulfuron | Isoxadifen |
| B-810 | Atrazin + Tembotrion | Isoxadifen |
| B-811 | Atrazin + Topramezone | Isoxadifen |
| B-812 | Clomazon + Glyphosat | Isoxadifen |
| B-813 | Diflufenican + Clodinafop-propargyl | Isoxadifen |
| B-814 | Diflufenican + Fenoxaprop-P-ethyl | Isoxadifen |
| B-815 | Diflufenican + Flupyrsulfuron-methyl-natrium | Isoxadifen |
| B-816 | Diflufenican + Glyphosat | Isoxadifen |
| B-817 | Diflufenican + Mesosulfuron-methyl | Isoxadifen |
| B-818 | Diflufenican + Pinoxaden | Isoxadifen |
| B-819 | Diflufenican + Pyroxsulam | Isoxadifen |
| B-820 | Flumetsulam + Glyphosat | Isoxadifen |
| B-821 | Flumioxazin + Glyphosat | Isoxadifen |
| B-822 | Imazapic + Glyphosat | Isoxadifen |
| B-823 | Imazethapyr + Glyphosat | Isoxadifen |
| B-824 | Isoxaflutol + H-1 | Isoxadifen |
| B-825 | Isoxaflutol + Glyphosat | Isoxadifen |
| B-826 | Metazachlor + H-1 | Isoxadifen |
| B-827 | Metazachlor + Glyphosat | Isoxadifen |
| B-828 | Metazachlor + Mesotrion | Isoxadifen |
| B-829 | Metazachlor + Nicosulfuron | Isoxadifen |
| B-830 | Metazachlor + Terbuthylazin | Isoxadifen |
| B-831 | Metazachlor + Topramezon | Isoxadifen |
| B-832 | Metribuzin + Glyphosat | Isoxadifen |
| B-833 | Pendimethalin + H-1 | Isoxadifen |
| B-834 | Pendimethalin + Clodinafop-propargyl | Isoxadifen |
| B-835 | Pendimethalin + Fenoxaprop-P-ethyl | Isoxadifen |
| B-836 | Pendimethalin + Flupyrsulfuron-methyl-natrium | Isoxadifen |
| B-837 | Pendimethalin + Glyphosat | Isoxadifen |
| B-838 | Pendimethalin + Mesosulfuron-methyl | Isoxadifen |
| B-839 | Pendimethalin + Mesotrione | Isoxadifen |
| B-840 | Pendimethalin + Nicosulfuron | Isoxadifen |
| B-841 | Pendimethalin + Pinoxaden | Isoxadifen |
| B-842 | Pendimethalin + Pyroxsulam | Isoxadifen |
| B-843 | Pendimethalin + Tembotrione | Isoxadifen |
| B-844 | Pendimethalin + Topramezone | Isoxadifen |
| B-845 | Pyroxasulfon + Tembotrion | Isoxadifen |
| B-846 | Pyroxasulfon + Topramezon | Isoxadifen |
| B-847 | Sulfentrazon + Glyphosat | Isoxadifen |
| B-848 | Terbuthylazin + H-1 | Isoxadifen |
| B-849 | Terbuthylazin + Foramsulfuron | Isoxadifen |
| B-850 | Terbuthylazin + Glyphosat | Isoxadifen |
| B-851 | Terbuthylazin + Mesotrion | Isoxadifen |
| B-852 | Terbuthylazin + Nicosulfuron | Isoxadifen |
| B-853 | Terbuthylazin + Tembotrion | Isoxadifen |
| B-854 | Terbuthylazin + Topramezon | Isoxadifen |
| B-855 | Trifluralin + Glyphosat | Isoxadifen |
| B-856 | Clodinafop-propargyl | Mefenpyr |
| B-857 | Cycloxydim | Mefenpyr |
| B-858 | Cyhalofop-butyl | Mefenpyr |
| B-859 | Fenoxaprop-P-ethyl | Mefenpyr |
| B-860 | Pinoxaden | Mefenpyr |
| B-861 | Profoxydim | Mefenpyr |
| B-862 | Tepraloxydim | Mefenpyr |
| B-863 | Tralkoxydim | Mefenpyr |
| B-864 | Esprocarb | Mefenpyr |
| B-865 | Prosulfocarb | Mefenpyr |
| B-866 | Thiobencarb | Mefenpyr |
| B-867 | Triallat | Mefenpyr |
| B-868 | Bensulfuron-methyl | Mefenpyr |
| B-869 | Bispyribac-natrium | Mefenpyr |
| B-870 | Cyclosulfamuron | Mefenpyr |
| B-871 | Flumetsulam | Mefenpyr |
| B-872 | Flupyrsulfuron-methyl-natrium | Mefenpyr |
| B-873 | Foramsulfuron | Mefenpyr |
| B-874 | Imazamox | Mefenpyr |
| B-875 | Imazapic | Mefenpyr |
| B-876 | Imazapyr | Mefenpyr |
| B-877 | Imazaquin | Mefenpyr |
| B-878 | Imazethapyr | Mefenpyr |
| B-879 | Imazosulfuron | Mefenpyr |
| B-880 | lodosulfuron-methyl-natrium | Mefenpyr |
| B-881 | Mesosulfuron | Mefenpyr |
| B-882 | Nicosulfuron | Mefenpyr |
| B-883 | Penoxsulam | Mefenpyr |
| B-884 | Propoxycarbazon-natrium | Mefenpyr |
| B-885 | Pyrazosulfuron-ethyl | Mefenpyr |
| B-886 | Pyroxsulam | Mefenpyr |
| B-887 | Rimsulfuron | Mefenpyr |
| B-888 | Sulfosulfuron | Mefenpyr |
| B-889 | Thiencarbazon-methyl | Mefenpyr |
| B-890 | Tritosulfuron | Mefenpyr |
| B-891 | 2,4-D und seine Salze und Ester | Mefenpyr |
| B-892 | Aminopyralid und seine Salze und Ester | Mefenpyr |
| B-893 | Clopyralid und seine Salze und Ester | Mefenpyr |
| B-894 | Dicamba und seine Salze und Ester | Mefenpyr |
| B-895 | Fluroxypyr-meptyl | Mefenpyr |
| B-896 | Quinclorac | Mefenpyr |
| B-897 | Quinmerac | Mefenpyr |
| B-898 | H-9 | Mefenpyr |
| B-899 | Diflufenzopyr | Mefenpyr |
| B-900 | Diflufenzopyr-natrium | Mefenpyr |
| B-901 | Clomazon | Mefenpyr |
| B-902 | Diflufenican | Mefenpyr |
| B-903 | Flurochloridon | Mefenpyr |
| B-904 | Isoxaflutol | Mefenpyr |
| B-905 | Mesotrion | Mefenpyr |
| B-906 | Picolinafen | Mefenpyr |
| B-907 | Sulcotrion | Mefenpyr |
| B-908 | Tefuryltrion | Mefenpyr |
| B-909 | Tembotrion | Mefenpyr |
| B-910 | Topramezon | Mefenpyr |
| B-911 | H-7 | Mefenpyr |
| B-912 | Atrazin | Mefenpyr |
| B-913 | Diuron | Mefenpyr |
| B-914 | Fluometuron | Mefenpyr |
| B-915 | Hexazinon | Mefenpyr |
| B-916 | Isoproturon | Mefenpyr |
| B-917 | Metribuzin | Mefenpyr |
| B-918 | Propanil | Mefenpyr |
| B-919 | Terbuthylazin | Mefenpyr |
| B-920 | Paraquat-dichlorid | Mefenpyr |
| B-921 | Flumioxazin | Mefenpyr |
| B-922 | Oxyfluorfen | Mefenpyr |
| B-923 | Sulfentrazon | Mefenpyr |
| B-924 | H-1 | Mefenpyr |
| B-925 | H-2 | Mefenpyr |
| B-926 | Glyphosat | Mefenpyr |
| B-927 | Glyphosat-isopropylammonium | Mefenpyr |
| B-928 | Glyphosat-trimesium (sulfosate) | Mefenpyr |
| B-929 | Glufosinat | Mefenpyr |
| B-930 | Glufosinat-ammonium | Mefenpyr |
| B-931 | Pendimethalin | Mefenpyr |
| B-932 | Trifluralin | Mefenpyr |
| B-933 | Acetochlor | Mefenpyr |
| B-934 | Cafenstrol | Mefenpyr |
| B-935 | Dimethenamid-P | Mefenpyr |
| B-936 | Fentrazamid | Mefenpyr |
| B-937 | Flufenacet | Mefenpyr |
| B-938 | Mefenacet | Mefenpyr |
| B-939 | Metazachlor | Mefenpyr |
| B-940 | Metolachlor-S | Mefenpyr |
| B-941 | Pyroxasulfon | Mefenpyr |
| B-942 | Isoxaben | Mefenpyr |
| B-943 | Dymron | Mefenpyr |
| B-944 | Indanofan | Mefenpyr |
| B-945 | Oxaziclomefon | Mefenpyr |
| B-946 | Triaziflam | Mefenpyr |
| B-947 | Atrazin + H-1 | Mefenpyr |
| B-948 | . Atrazin + Glyphosat | Mefenpyr |
| B-949 | Atrazin + Mesotrion | Mefenpyr |
| B-950 | Atrazin + Nicosulfuron | Mefenpyr |
| B-951 | Atrazin + Tembotrion | Mefenpyr |
| B-952 | Atrazin + Topramezone | Mefenpyr |
| B-953 | Clomazon + Glyphosat | Mefenpyr |
| B-954 | Diflufenican + Clodinafop-propargyl | Mefenpyr |
| B-955 | Diflufenican + Fenoxaprop-P-ethyl | Mefenpyr |
| B-956 | Diflufenican + Flupyrsulfuron-methyl-natrium | Mefenpyr |
| B-957 | Diflufenican + Glyphosat | Mefenpyr |
| B-958 | Diflufenican + Mesosulfuron-methyl | Mefenpyr |
| B-959 | Diflufenican + Pinoxaden | Mefenpyr |
| B-960 | Diflufenican + Pyroxsulam | Mefenpyr |
| B-961 | Flumetsulam + Glyphosat | Mefenpyr |
| B-962 | Flumioxazin + Glyphosat | Mefenpyr |
| B-963 | Imazapic + Glyphosat | Mefenpyr |
| B-964 | Imazethapyr + Glyphosat | Mefenpyr |
| B-965 | Isoxaflutol + H-1 | Mefenpyr |
| B-966 | Isoxaflutol + Glyphosat | Mefenpyr |
| B-967 | Metazachlor + H-1 | Mefenpyr |
| B-968 | Metazachlor + Glyphosat | Mefenpyr |
| B-969 | Metazachlor + Mesotrion | Mefenpyr |
| B-970 | Metazachlor + Nicosulfuron | Mefenpyr |
| B-971 | Metazachlor + Terbuthylazin | Mefenpyr |
| B-972 | Metazachlor + Topramezon | Mefenpyr |
| B-973 | Metribuzin + Glyphosat | Mefenpyr |
| B-974 | Pendimethalin + H-1 | Mefenpyr |
| B-975 | Pendimethalin + Clodinafop-propargyl | Mefenpyr |
| B-976 | Pendimethalin + Fenoxaprop-P-ethyl | Mefenpyr |
| B-977 | Pendimethalin + Flupyrsulfuron-methyl-natrium | Mefenpyr |
| B-978 | Pendimethalin + Glyphosat | Mefenpyr |
| B-979 | Pendimethalin + Mesosulfuron-methyl | Mefenpyr |
| B-980 | Pendimethalin + Mesotrione | Mefenpyr |
| B-981 | Pendimethalin + Nicosulfuron | Mefenpyr |
| B-982 | Pendimethalin + Pinoxaden | Mefenpyr |
| B-983 | Pendimethalin + Pyroxsulam | Mefenpyr |
| B-984 | Pendimethalin + Tembotrione | Mefenpyr |
| B-985 | Pendimethalin + Topramezone | Mefenpyr |
| B-986 | Pyroxasulfon + Tembotrion | Mefenpyr |
| B-987 | Pyroxasulfon + Topramezon | Mefenpyr |
| B-988 | Sulfentrazon + Glyphosat | Mefenpyr |
| B-989 | Terbuthylazin + H-1 | Mefenpyr |
| B-990 | Terbuthylazin + Foramsulfuron | Mefenpyr |
| B-991 | Terbuthylazin + Glyphosat | Mefenpyr |
| B-992 | Terbuthylazin + Mesotrion | Mefenpyr |
| B-993 | Terbuthylazin + Nicosulfuron | Mefenpyr |
| B-994 | Terbuthylazin + Tembotrion | Mefenpyr |
| B-995 | Terbuthylazin + Topramezon | Mefenpyr |
| B-996 | Trifluralin + Glyphosat | Mefenpyr |
| B-997 | Clodinafop-propargyl | H-12 |
| B-998 | Cycloxydim | H-12 |
| B-999 | Cyhalofop-butyl | H-12 |
| B-1000 | Fenoxaprop-P-ethyl | H-12 |
| B-1001 | Pinoxaden | H-12 |
| B-1002 | Profoxydim | H-12 |
| B-1003 | Tepraloxydim | H-12 |
| B-1004 | Tralkoxydim | H-12 |
| B-1005 | Esprocarb | H-12 |
| B-1006 | Prosulfocarb | H-12 |
| B-1007 | Thiobencarb | H-12 |
| B-1008 | Triallat | H-12 |
| B-1009 | Bensuifuron-methyl | H-12 |
| B-1010 | Bispyribac-natrium | H-12 |
| B-1011 | Cyclosulfamuron | H-12 |
| B-1012 | Flumetsulam | H-12 |
| B-1013 | Flupyrsulfuron-methyl-natrium | H-12 |
| B-1014 | Foramsulfuron | H-12 |
| B-1015 | Imazamox | H-12 |
| B-1016 | Imazapic | H-12 |
| B-1017 | imazapyr | H-12 |
| B-1018 | Imazaquin | H-12 |
| B-1019 | Imazethapyr | H-12 |
| B-1020 | Imazosulfuron | H-12 |
| B-1021 | lodosulfuron-methyl-natrium | H-12 |
| B-1022 | Mesosulfuron | H-12 |
| B-1023 | Nicosulfuron | H-12 |
| B-1024 | Penoxsulam | H-12 |
| B-1025 | Propoxycarbazon-natrium | H-12 |
| B-1026 | Pyrazosulfuron-ethyl | H-12 |
| B-1027 | Pyroxsulam | H-12 |
| B-1028 | Rimsulfuron | H-12 |
| B-1029 | Sulfosulfuron | H-12 |
| B-1030 | Thiencarbazon-methyl | H-12 |
| B-1031 | Tritosulfuron | H-12 |
| B-1032 | 2,4-D und seine Salze und Ester | H-12 |
| B-1033 | Aminopyralid und seine Salze und Ester | H-12 |
| B-1034 | Clopyralid und seine Salze und Ester | H-12 |
| B-1035 | Dicamba und seine Salze und Ester | H-12 |
| B-1036 | Fluroxypyr-meptyl | H-12 |
| B-1037 | Quinclorac | H-12 |
| B-1038 | Quinmerac | H-12 |
| B-1039 | H-9 | H-12 |
| B-1040 | Diflufenzopyr | H-12 |
| B-1041 | Diflufenzopyr-natrium | H-12 |
| B-1042 | Clomazon | H-12 |
| B-1043 | Diflufenican | H-12 |
| B-1044 | Flurochloridon | H-12 |
| B-1045 | Isoxaflutol | H-12 |
| B-1046 | Mesotrion | H-12 |
| B-1047 | Picolinafen | H-12 |
| B-1048 | Sulcotrion | H-12 |
| B-1049 | Tefuryltrion | H-12 |
| B-1050 | Tembotrion | H-12 |
| B-1051 | Topramezon | H-12 |
| B-1052 | H-7 | H-12 |
| B-1053 | Atrazin | H-12 |
| B-1054 | Diuron | H-12 |
| B-1055 | Fluometuron | H-12 |
| B-1056 | Hexazinon | H-12 |
| B-1057 | Isoproturon | H-12 |
| B-1058 | Metribuzin | H-12 |
| B-1059 | Propanil | H-12 |
| B-1060 | Terbuthylazin | H-12 |
| B-1061 | Paraquat-dichlorid | H-12 |
| B-1062 | Flumioxazin | H-12 |
| B-1063 | Oxyfluorfen | H-12 |
| B-1064 | Sulfentrazon | H-12 |
| B-1065 | H-1 | H-12 |
| B-1066 | H-2 | H-12 |
| B-1067 | Glyphosat | H-12 |
| B-1068 | Glyphosat-isopropylammonium | H-12 |
| B-1069 | Glyphosat-trimesium (sulfosate) | H-12 |
| B-1070 | Glufosinat | H-12 |
| B-1071 | Glufosinat-ammonium | H-12 |
| B-1072 | Pendimethalin | H-12 |
| B-1073 | Trifluralin | H-12 |
| B-1074 | Acetochlor | H-12 |
| B-1075 | Cafenstrol | H-12 |
| B-1076 | Dimethenamid-P | H-12 |
| B-1077 | Fentrazamid | H-12 |
| B-1078 | Flufenacet | H-12 |
| B-1079 | Mefenacet | H-12 |
| B-1080 | Metazachlor | H-12 |
| B-1081 | Metolachlor-S | H-12 |
| B-1082 | Pyroxasulfon | H-12 |
| B-1083 | Isoxaben | H-12 |
| B-1084 | Dymron | H-12 |
| B-1085 | Indanofan | H-12 |
| B-1086 | Oxaziclomefon | H-12 |
| B-1087 | Triaziflam | H-12 |
| B-1088 | Atrazin + H-1 | H-12 |
| B-1089 | Atrazin + Glyphosat | H-12 |
| B-1090 | Atrazin + Mesotrion | H-12 |
| B-1091 | Atrazin + Nicosulfuron | H-12 |
| B-1092 | Atrazin + Tembotrion | H-12 |
| B-1093 | Atrazin + Topramezone | H-12 |
| B-1094 | Clomazon + Glyphosat | H-12 |
| B-1095 | Diflufenican + Clodinafop-propargyl | H-12 |
| B-1096 | Diflufenican + Fenoxaprop-P-ethyl | H-12 |
| B-1097 | Diflufenican + Flupyrsulfuron-methyl- | H-12 |
| | natrium | |
| B-1098 | Diflufenican + Glyphosat | H-12 |
| B-1099 | Diflufenican + Mesosulfuron-methyl | H-12 |
| B-1100 | Diflufenican + Pinoxaden | H-12 |
| B-1101 | Diflufenican + Pyroxsulam | H-12 |
| B-1102 | Flumetsulam + Glyphosat | H-12 |
| B-1103 | Flumioxazin + Glyphosat | H-12 |
| B-1104 | Imazapic + Glyphosat | H-12 |
| B-1105 | Imazethapyr + Glyphosat | H-12 |
| B-1106 | Isoxaflutol + H-1 | H-12 |
| B-1107 | Isoxaflutol + Glyphosat | H-12 |
| B-1108 | Metazachlor + H-1 | H-12 |
| B-1109 | Metazachlor + Glyphosat | H-12 |
| B-1110 | Metazachlor + Mesotrion | H-12 |
| B-1111 | Metazachlor + Nicosulfuron | H-12 |
| B-1112 | Metazachlor + Terbuthylazin | H-12 |
| B-1113 | Metazachlor + Topramezon | H-12 |
| B-1114 | Metribuzin + Glyphosat | H-12 |
| B-1115 | Pendimethalin + H-1 | H-12 |
| B-1116 | Pendimethalin + Clodinafop-propargyl | H-12 |
| B-1117 | Pendimethalin + Fenoxaprop-P-ethyl | H-12 |
| B-1118 | Pendimethalin + Flupyrsulfuron-methyl-natrium | H-12 |
| B-1119 | Pendimethalin + Glyphosat | H-12 |
| B-1120 | Pendimethalin + Mesosulfuron-methyl | H-12 |
| B-1121 | Pendimethalin + Mesotrione | H-12 |
| B-1122 | Pendimethalin + Nicosulfuron | H-12 |
| B-1123 | Pendimethalin + Pinoxaden | H-12 |
| B-1124 | Pendimethalin + Pyroxsulam | H-12 |
| B-1125 | Pendimethalin + Tembotrione | H-12 |
| B-1126 | Pendimethalin + Topramezone | H-12 |
| B-1127 | Pyroxasulfon + Tembotrion | H-12 |
| B-1128 | Pyroxasulfon + Topramezon | H-12 |
| B-1129 | Sulfentrazon + Glyphosat | H-12 |
| B-1130 | Terbuthylazin + H-1 | H-12 |
| B-1131 | Terbuthylazin + Foramsulfuron | H-12 |
| B-1132 | Terbuthylazin + Glyphosat | H-12 |
| B-1133 | Terbuthylazin + Mesotrion | H-12 |
| B-1134 | Terbuthylazin + Nicosulfuron | H-12 |
| B-1135 | Terbuthylazin + Tembotrion | H-12 |
| B-1136 | Terbuthylazin + Topramezon | H-12 |
| B-1137 | Trifluralin + Glyphosat | H-12 |
| B-1138 | 2-1 | - |
| B-1139 | 2-2 | - |
| B-1140 | 2-3 | - |
| B-1141 | 2-4 | - |
| B-1142 | 2-5 | - |
| B-1143 | 2-6 | - |
| B-1144 | 2-7 | - |
| B-1145 | 2-8 | - |
| B-1146 | 2-9 | - |
| B-1147 | 2-1 | Benoxacor |
| B-1148 | 2-2 | Benoxacor |
| B-1149 | 2-3 | Benoxacor |
| B-1150 | 2-4 | Benoxacor |
| B-1151 | 2-5 | Benoxacor |
| B-1152 | 2-6 | Benoxacor |
| B-1153 | 2-7 | Benoxacor |
| B-1154 | 2-8 | Benoxacor |
| B-1155 | 2-9 | Benoxacor |
| B-1156 | 2-1 | Cloquintocet |
| B-1157 | 2-2 | Cloquintocet |
| B-1158 | 2-3 | Cloquintocet |
| B-1159 | 2-4 | Cloquintocet |
| B-1160 | 2-5 | Cloquintocet |
| B-1161 | 2-6 | Cloquintocet |
| B-1162 | 2-7 | Cloquintocet |
| B-1163 | 2-8 | Cloquintocet |
| B-1164 | 2-9 | Cloquintocet |
| B-1165 | 2-1 | Cyprosulfamid |
| B-1166 | 2-2 | Cyprosulfamid |
| B-1167 | 2-3 | Cyprosulfamid |
| B-1168 | 2-4 | Cyprosulfamid |
| B-1169 | 2-5 | Cyprosulfamid |
| B-1170 | 2-6 | Cyprosulfamid |
| B-1171 | 2-7 | Cyprosulfamid |
| B-1172 | 2-8 | Cyprosulfamid |
| B-1173 | 2-9 | Cyprosulfamid |
| B-1174 | 2-1 | Dichlormid |
| B-1175 | 2-2 | Dichlormid |
| B-1176 | 2-3 | Dichlormid |
| B-1177 | 2-4 | Dichlormid |
| B-1178 | 2-5 | Dichlormid |
| B-1179 | 2-6 | Dichlormid |
| B-1180 | 2-7 | Dichlormid |
| B-1181 | 2-8 | Dichlormid |
| B-1182 | 2-9 | Dichlormid |
| B-1183 | 2-1 | Fenchlorazol |
| B-1184 | 2-2 | Fenchlorazol |
| B-1185 | 2-3 | Fenchlorazol |
| B-1186 | 2-4 | Fenchlorazol |
| B-1187 | 2-5 | Fenchlorazol |
| B-1188 | 2-6 | Fenchlorazol |
| B-1189 | 2-7 | Fenchlorazol |
| B-1190 | 2-8 | Fenchlorazol |
| B-1191 | 2-9 | Fenchlorazol |
| B-1192 | 2-1 | Isoxadifen |
| B-1193 | 2-2 | Isoxadifen |
| B-1194 | 2-3 | Isoxadifen |
| B-1195 | 2-4 | Isoxadifen |
| B-1196 | 2-5 | Isoxadifen |
| B-1197 | 2-6 | Isoxadifen |
| B-1198 | 2-7 | Isoxadifen |
| B-1199 | 2-8 | Isoxadifen |
| B-1200 | 2-9 | Isoxadifen |
| B-1201 | 2-1 | Mefenpyr |
| B-1202 | 2-2 | Mefenpyr |
| B-1203 | 2-3 | Mefenpyr |
| B-1204 | 2-4 | Mefenpyr |
| B-1205 | 2-5 | Mefenpyr |
| B-1206 | 2-6 | Mefenpyr |
| B-1207 | 2-7 | Mefenpyr |
| B-1208 | 2-8 | Mefenpyr |
| B-1209 | 2-9 | Mefenpyr |
| B-1210 | 2-1 | H-11 |
| B-1211 | 2-2 | H-11 |
| B-1212 | 2-3 | H-11 |
| B-1213 | 2-4 | H-11 |
| B-1214 | 2-5 | H-11 |
| B-1215 | 2-6 | H-11 |
| B-1216 | 2-7 | H-11 |
| B-1217 | 2-8 | H-11 |
| B-1218 | 2-9 | H-11 |
| B-1219 | 2-1 | H-12 |
| B-1220 | 2-2 | H-12 |
| B-1221 | 2-3 | H-12 |
| B-1222 | 2-4 | H-12 |
| B-1223 | 2-5 | H-12 |
| B-1224 | 2-6 | H-12 |
| B-1225 | 2-7 | H-12 |
| B-1226 | 2-8 | H-12 |
| B-1227 | 2-9 | H-12 |

Die Verbindungen I und die erfindungsgemäßen Zusammensetzungen können auch eine pflanzenstärkende Wirkung aufweisen. Sie eigenen sich daher zu Mobilisierung pflanzeneigener Abwehrkräfte gegen Befall durch unerwünschte Mikroorganismen, wie Schadpilze, aber auch Viren und Bakterien. Unter pflanzenstärkenden (resistenzinduzierenden) Stoffen sind in diesem Zusammenhang solche Substanzen zu verstehen, die in der Lage sind, das Abwehrsystem von behandelten Pflanzen so zu stimulieren, dass diese bei nachfolgender Inokulation mit unerwünschten Mikroorganismen weitgehende Resistenz gegen diese Mikroorganismen entfalten.

Die Verbindungen I können eingesetzt werden, um Pflanzen innerhalb eines gewissen Zeitraumes nach der Behandlung gegen den Befall durch unerwünschte Mikroorganismen zu schützen. Der Zeitraum, innerhalb dessen Schutz herbeigeführt wird, erstreckt sich im Allgemeinen auf 1 bis 28 Tage, vorzugsweise 1 bis 14 Tage nach der Behandlung der Pflanzen mit den Verbindungen I bzw. nach Behandlung des Saatguts, auf bis zu 9 Monate nach Aussaat.

Die Verbindungen I und die erfindungsgemäßen Zusammensetzungen eignen sich auch zur Steigerung des Ernteertrages.

Sie sind außerdem mindertoxisch und weisen eine gute Pflanzenverträglichkeit auf.

Im Folgenden wird die Herstellung von Pyridinverbindungen der Formel I anhand von Beispielen erläutert ohne dabei den Gegenstand der vorliegenden Erfindung auf die gezeigten Beispiele zu begrenzen.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Angaben aufgeführt.

### I. Herstellungsbeispiele

### Beispiel 1: Herstellung von 4-Hydroxy-3-(3-trifluormethoxy-phenyl)-pyrano[3,2-b]pyridin-2-on [I-27]

### Stufe 1: 3-Hydroxy-pyridin-2-carbonsäure-pentafluorphenylester

Eine Lösung von 14g 3-Hydroxy-pyridin-2-carbonsäure und 18,5g Pentafluorphenol in 700ml CH₂Cl₂ wurde bei 20-25°C tropfenweise mit 13g N,N' -Diisopropylcarbodiimid (DIC) versetzt. Nach vollständiger Umsetzung (ca. 40min) wurde die Lösung für etwa 12 Std. bei 20-25°C stehen gelassen. Nach Entfernen des Lösungsmittels wurde der entstandene Rückstand in Wasser aufgenommen und die Lösung mit CH₂Cl₂ extrahiert. Aus der organischen Phase wurde nach Trocknen und Entfernen des Lösungsmittels 29g der Titelverbindung erhalten.

### Stufe 2: 4-Hydroxy-3-(3-trifluormethoxy-phenyl)-pyrano[3,2-b]pyridin-2-on

Eine Lösung von 0,64g 3-Hydroxy-pyridin-2-carbonsäure-pentafluorphenylester (aus Stufe 1) und 0,5g (3-Trifluormethoxy-phenyl)-acetylchlorid in 150ml Acetonitril wurden mit 3,5g K₂CO₃ versetzt und unter N₂-Atmosphäre etwa 12 Std. bei 20-25°C gerührt. Nach Filtration wurde das Eluat vom Lösungsmittel befreit, der erhaltene Rückstand in Wasser aufgenommen und nach Ansäuern auf pH < 4 mit CH₂Cl₂ extrahiert. Die organische Phase wurde getrocknet, dann vom Lösungsmittel befreit. Aus dem Rückstand wurde mittels präparativer HPLC [Säule: Luna (2), Fa. Phenomenex, 300*50mm 10µ m; mobile Phase: Wasser (+ 0,0375% Trifluoressigsäure) und Acetonitril in Mischungsverhältnissen von 80:20 bzw. 50:50; Flußrate 80 ml/min; Detektion bei 220, bzw. 254 nm), 20-25°C] 300mg der Titelverbindung erhalten.
¹H-NMR (CDCl₃) δ 8,55 (m, 1 H), 7,74 (m, 1 H), 7,58-7,73 (m, 3H), 7,48-7,50 (m, 1 H), 7,22-7,26 (m, 1 H).

**Tabelle I: Verbindungen der Formel welches der Formel I.A entsprechen:**

| | | | | | | | | | | |
|---|---|---|---|---|---|---|---|---|---|---|
| 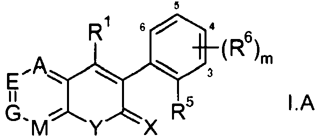 | | | | | | | | | | |

| Nr. | A | E | G | M | Y | X | R¹ | R⁵ | (R⁶)ₘ | Phys. Daten ¹H-NMR (δ [ppm])^{#}) |
|---|---|---|---|---|---|---|---|---|---|---|
| I-1 | N | CH | CH | CH | O | O | OH | H | 3-CF₃ | (M): 8,57 (d), 7,96 (s), 7,78 (d), 7,75 (d), 7,66-7,59 (m) |
| I-2 | N | CH | CH | CH | O | O | OH | H | 4-CF₃ | (M): 8,57 (d), 7,82 (d), 7,76-7,73 (m), 7,63 (d) |
| I-3 | N | CH | CH | CH | O | O | OH | OCH₃ | H | (M): 8,52 (d), 7,72 (d), 7,58-7,55 (m), 7,41-7,35 (m), 7,08-7,02 (m), 3,36 (s) |
| I-4 | N | CH | CH | CH | O | O | OH | CN | H | (M): 8,47 (d), 7,69 (d), 7,59 (d), 7,53-7,50 (m), 7,34 (t) |
| I-5 | N | CH | CH | CH | O | O | OH | NO₂ | H | (D): 8,69 (d), 8,08 (d), 8,01 (d), 7,83-7,78 (m), 7,67-7,62 (m) |
| I-6 | N | CH | CH | CH | O | O | OH | F | H | (M): 8,50 (d), 7,78 (d), 7,53 (m), 7,44 (m), 7,34-7,30 (m), 7,19-7,09 (m) |
| I-7 | N | CH | CH | CH | O | O | OH | H | 3-F | (D): 8,86 (d), 7,96 (d), 7,79-7,74 (m), 7,48-7,42 (m), 7,36-7,30 (m), 7,16 (t) |
| I-8 | N | CH | CH | CH | O | O | OH | Cl | H | (M): 8,18 (d), 7,84 (s), 7,63-7,57 (m), 7,43-7,21 (m) |
| I-9 | N | CH | CH | CH | O | O | OH | H | 3-Cl | (M): 8,16 (m), 7,78 (m), 7,59 (m), 7,20-7,06 (m) |
| I-10 | N⁺ CF₃COO⁻ | CH | CH | CH | O | O | OH | CHF₂ | H | (D): 8,65 (d), 7,96 (d), 7,78 (m), 7,68 (d), 7,58-7,52 (m), 734 (d), 7,19-6,71 (m) |
| I-11 | N⁺ CF₃COO⁻ | CH | CH | CH | O | O | OH | OCF₃ | H | (M): 8,66 (d), 7,97 (d), 780-777 (m), 754-741 (m) |
| I-12 | N | CH | CH | CH | O | O | OH | OCHF₂ | H | (D): 8,68 (d), 7,98 (d), 7,81-7,78 (m), 7,49-6,95 (m) |
| I-13 | N | CH | CH | CH | O | O | OH | Br | H | (M): 8,44 (s), 7,78 (d), 7,67 (d), 7,49 (s), 7,37-7,33 (m), 7,24-7,20 (m) |
| I-14 | CH | CH | CH | CH | O | O | OH | CF₃ | H | (M): 7,90 (t), 7,73 (t), 7,66-7,61 (m), 7,46 (d), 7,40 (d), 7,33 (t) |
| I-15 | CH | CH | N | CH | O | O | OH | CF₃ | H | (M): 8,59 (s), 8,39 (d), 7,91-7,90 (m), 7,72 (d), 7,61 (t), 7,45 (t), 7,34 (d) |
| I-16 | CH | CH | CH | N | O | O | OH | CF₃ | H | (M): 8,43-8,40 (m), 7,71 (d), 7,59 (d), 7,44 (t), 7,37-7,34 (m) |
| I-17 | N | CH | CH | CH | S | O | OH | CF₃ | H | (M): 8,63 (d), 7,88 (d), 7,81 (d), 7,65 (t), 7,58-7,50 (m), 7,35 (d) |
| I-18 | N | CH | CH | CH | O | O | OH | CF₃ | H | (M): 8,64 (s), 7,86 (d), 779 (d), 7,72-7,69 (m), 7,58 (t), 7,43 (d) |
| I-19 | N-O ¹⁾ | CH | CH | CH | O | O | OH | CF₃ | H | (M): 8,39 (d), 7,80 (d), 7,77-7,68 (m), 7,60 (t), 7,52 (t), 7,34 (d) |
| I-20 | N⁺ CF₃COO⁻ | CH | CH | CH | O | O | OH | Cl | 4-Cl | (D): 8,65 (d), 7,96 (d), 7,78-7,75 (m), 7,69 (s), 7,47 (d), 7,41 (d) |
| I-21 | N | C-Br | CH | CH | O | O | OH | CF₃ | H | (C): 7,81 (d), 7,36 (d), 7,68-7,55 (m), 738 (d) |
| 1-22 | N | C-OCH₂C₆H₅ | CH | CH | O | O | OH | CF₃ | H | (C): 7,79(d), 7,69-7,65(m), 7,59 (m), 7,46-7,26(m), 7,12(d), 5,42(d) |
| I-23 | N | C-OH | CH | CH | O | O | OH | CF₃ | H | (M): 7,8 (d), 7,68-7,58 (m), 7,58 (m), 7,38 (t), 6,92 (d) |
| I-24 | N-O ¹⁾ | CH | CH | CH | O | O | OH | CHF₂ | H | (C): 8,18(d), 7,75-7,73 (m), 7,58-7,51 (m), 7,40-7,38 (m), 6,75 (t) |
| I-25 | N-O ¹⁾ | CH | CH | CH | O | O | OH | OCF₃ | H | (C): 8,18 (d), 7,54-7,53 (m), 7,48-7,44 (m), 7,39-7,36 (m) |
| I-26 | N-O ¹⁾ | CH | CH | CH | O | O | OH | OCHF₂ | H | (C): 8,18 (d), 7,53-7,52 (m), 7,46-7,42 (m), 7,34-7,26 (m), 6,50 (t) |
| I-27 | N | CH | CH | CH | O | O | OH | H | 3-OCF₃ | (C): 8,55 (m), 7,74 (m), 7,73-7,58 (m), 7,50-7,48 (m), 7,26-7,22 (m) |
| I-28 | N | CH | CH | CH | O | O | OH | H | 4-OCF₃ | (C): 8,56 (d), 7,75-7,72 (m), 7,63-7,59 (m), 7,32-7,30 (m) |
| I-29 | N | CH | CH | CH | O | O | OH | H | 3-OCH₃ | (C): 8,54 (d), 7,71 (m), 7,60 (m), 7,37 (m), 7,25 (m), 6,95 (m), 3,85 (s) |
| I-30 | N | CH | CH | CH | O | O | OH | H | 4-OCH₃ | (C): 8,53 (d), 7,71 (m), 7,62 (m), 7,56 (m), 7,01 (m), 3,85 (s) |
| I-31 | N | CH | CH | CH | O | O | OH | H | 3-SCF₃ | (C): 8,56 (d), 8,01 (s), 7,82 (m), 7,76 (m), 7,67 (m), 7,60 (m), 7,51 (m) |
| I-32 | N | CH | CH | CH | S | O | OH | OCF₃ | H | (C): 8,64 (d), 7,88 (m), 7,54 (m), 7,46-7,39 (m), 7,39-7,36 (m) |
| I-33 | N | CH | CH | CH | S | O | OH | CHF₂ | H | (C): 8,62 (d), 7,88 (m), 7,74 (m), 7,56-7,50 (m), 7,30 (m), 6,60 (t) |
| I-34 | N | CH | CH | CH | O | O | OC(O)C(CH₃)₃ | CF₃ | H | (C): 8,55 (d), 7,76 (d), 7,69 (d), 7,61-7,48 (m), 7,31 (d), 1,06 (s) |
| I-35 | N | CH | CH | CH | O | O | OH | CF₃ | 5-CF₃ | (C): 8,50-8,49 (m), 7,94 (d), 7,78 (d), 7,71 (d), 7,67 (s), 7,54-7,50 (m) |
| I-36 | N | CH | CH | CH | O | O | OH | CF₃ | 4-CF₃ | (C): 8,53-8,52 (m), 8,15 (s), 7,85 (d), 7,68 (d), 7,56-7,53 (m), 7,38-7,36 (m) |
| I-37 | N | CH | CH | CH | O | O | OH | CF₃ | 4-F | (M): 8,50-8,49 (m), 7,21 (d), 7,55-7,51 (m), 7,45 (d), 7,36-7,33 (m) |
| I-38 | N | CH | CH | CH | O | O | OH | CF₃ | 6-F | (C): 8,55-8,53 (m), 7,69 (d), 7,58-7,53 (m), 7,51-7,48 (m), 7,34-7,30 (m) |
| I-39 | N | CH | CH | CH | O | O | OH | CF₃ | - | (C): 8,49-8,48 (m), 7,71 (d), 7,57-7,54 (m), 7,4 (s), 7,20 (s), 2,18 (s) |
| I-40 | N | CH | CH | CH | O | O | OH | Cl | 6-F | (M): 8,65-8,64 (m), 7,87 (d), 7,73-7,70 (m), 7,43-7,35 (m), 7,19-7,17 (m) |
| I-41 | N | CH | CH | CH | O | O | OH | Cl | 5-CF₃ | (C): 8,58-8,57 (m), 7,79-7,77 (m), 7,69-7,60 (m) |
| I-42 | N | CH | CH | CH | O | O | OH | I | - | (C): 8,56-8,55 (m), 7,98-7,96 (m), 7,77-7,75 (m), 7,63-7,60 (m), 7,48-7,44 (m), 7,35-7,33 (m), 7,13-7,09 (m) |
| I-43 | N | CH | CH | CH | O | O | OH | OCH₃ | 4,5-F₂ | (C): 8,54-8,53 (m), 7,74-7,71 (m), 7,61-7,58 (m), 7,21-7,18 (m), 7,85-7,80 (m), 3,78 (s) |
| I-44 | N | CH | CH | CH | O | O | OH | CF₃ | 5-F | (C): 8,55-8,50 (m), 7,81-7,73 (m), 7,64-7,61 (m), 7,24-7,12 (m), 7,12-7,10 (m) |
| I-45 | N | CH | CH | CH | O | O | OH | CF₃ | 5-Cl | (C): 8,55-8,54 (m), 7,75-7,71 (m), 7,74-7,61 (m), 7,51 (d), 7,24 (s) |
| I-46 | N | CH | CH | CH | O | O | OC(O)CH₃ | CF₃ | - | (C): 8,54-8,53 (m), 7,74-7,72 (m), 7,66-7,64 (m), 7,58-7,28 (m), 7,28-7,27 (m), 2,14 (s) |
| I-47 | N | CH | CH | CH | O | O | OH | Cl | 3,6-F₂ | (C): 8,52-8,50 (m), 7,72-7,70 (m), 7,60-7,57(m), 7,17-7,13 (m), 7,05-7,00 (m) |
| I-48 | N | CH | CH | CH | O | O | OH | CF₃ | 3-F | (C): 8,57-8,56 (m), 7,76 (d), 7,65-7,59 (m), 7,28-7,26 (m), 3,87 (d) |
| I-49 | N | CH | CH | CH | O | O | OH | OCF₃ | - | (C): 8,56 (d), 7,76 (d), 7,64-7,61 (m), 7,56-7,51 (m), 7,41-7,38 (m) |
| I-50 | N | CH | CH | CH | O | O | OH | Br | 4-CF₃ | (C): 8,59-8,58 (m), 7,98 (s), 7,80-7,77 (m), 7,69-7,65 (m), 7,54-7,52 (m) |
| I-51 | N | CH | CH | CH | O | O | OH | Cl | 5-F | (C): 8,51-8,50 (m), 7,71-7,70 (m), 7,68-7,55 (m), 7,43-7,39 (m), 7,10-7,00 (m) |
| I-52 | N | CH | CH | CH | O | O | OH | Cl | 4,5-F₂ | (C): 8,87-8,57 (m), 7,78-7,75 (m), 7,66-7,63 (m), 7,39-7,34 (m), 7,28-7,24 (m) |
| I-53 | N | CH | CH | CH | O | O | OH | Cl | 4-OCF₃, 6-Cl | (C): 8,51-8,50 (m), 7,72-7,70 (m), 7,60-7,57 (m), 7,27 (s) |
| I-54 | N | CH | CH | CH | O | O | OH | Cl | 4-CF3, 6-Cl | (C): 8,60-8,59 (m), 7,80-7,78 (m), 7,71 (s), 7,69-7,65 (m) |
| I-55 | N | CH | CH | CH | O | O | OH | CF₃ | 6-Br | (M): 8,66-8,65 (m), 8,00-7,98 (m), 7,90-7,87 (m), 7,82-7,80 (m), 7,74-7,71 (m), 7,52-7,48 (m) |
| I-56 | N | CH | CH | CH | O | O | O-SO₂CH₃ | CF₃ | H | (C): 8,67 (d), 7,83 (d), 7,79-7,77 (m), 7,74-7,71 (m), 7,65-7,60 (m), 7,50 (d), 3,48 (s) |

| | | | | | | | | | | |
|---|---|---|---|---|---|---|---|---|---|---|
| ¹⁾ N-O = N-Oxid ^{#)} Angabe des Lösungsmittels: (C) = CDCl₃; (M) = CH₃OD; (D) = DMSO-d₆ | | | | | | | | | | |

### Anwendungsbeispiele

Die herbizide Wirkung der Verbindungen der Formel I ließ sich durch Gewächshausversuche zeigen:
Als Kulturgefäße dienten Plastiktöpfe mit lehmigem Sand mit etwa 3,0% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf. Eine gute herbizide Aktivität ist bei Werten von wenigstens 70 und eine sehr gute herbizide Aktivität ist bei Werten von wenigstens 85 gegeben.

Die in den Gewächshausversuchen verwendeten Pflanzen setzten sich aus folgenden Arten zusammen:

| Bayercode | Lateinischer Name | Deutscher Name | Englischer Name |
|---|---|---|---|
| ABUTH | Abutilon theophrasti | Chinesischer Hanf | China jute |
| ALOMY | Alopecurus myosuroides | Ackerfuchsschwanzgras | Blackgrass |
| AMARE | Amaranthus retroflexus | Krummer Amarant | Carelessweed |
| AVEFA | Avena fatua | Flughafer | spring wild-oat |
| CHEAL | Chenopodium album | Melde | Pigweed |
| GALAP | Galium aparine | Klettenlabkraut | Goosegrass |
| SETFA | Setaria faberi | Fabers Borstenhirse | giant foxtail |
| SETVI | Setaria viridis | Grüne Borstenhirse | Green foxtail |

| | | | |
|---|---|---|---|
| 1) Der Wirkstoff I-35 zeigte bei einer Aufwandmenge von 0,5 kg/ha im Vorauflauf gegen AMARE eine sehr gute herbizide Wirkung. 2) Die Wirkstoffe I-10, I-11, I-13, I-20, I-22, I-26, bzw. I-35 zeigten bei einer Aufwandmenge von 3,0 kg/ha und der Wirkstoff I-20 bei 2,0 kg/ha im Nachauflauf gegen ABUTH eine sehr gute und der Wirkstoff I-23 bei 3,0 kg/ha eine gute herbizide Wirkung. 3) Die Wirkstoffe I-46, I-54, bzw. I-55 zeigten bei einer Aufwandmenge von 3,0 kg/ha im Nachauflauf gegen ALOMY eine sehr gute herbizide Wirkung. 4) Die Wirkstoffe I-36, I-37, I-39, I-40, I-41, I-43, I-44, I-45, I-47, I-48, I-49, I-51, bzw. I-52 zeigten bei einer Aufwandmenge von 0,5 kg/ha und der Wirkstoff I-42 bei 1,0 kg/ha im Nachauflauf gegen AMARE eine sehr gute herbizide Wirkung. 5) Die Wirkstoffe I-17, I-18, I-19, I-21, I-46, I-53, bzw. I-55 zeigten bei einer Aufwandmenge von 3,0 kg/ha im Nachauflauf gegen AVEFA eine sehr gute herbizide Wirkung. 6) Die Wirkstoffe I-10, I-11, I-12, I-13, I-17, I-19, I-20, I-21, I-26., bzw. I-27 zeigten bei einer Aufwandmenge von 3,0 kg/ha und der Wirkstoff I-20 bei 2,0 kg/ha im Nachauflauf gegen SETFA eine sehr gute und die Wirkstoffe I-22, bzw. I-23 bei 3,0 kg/ha eine gute herbizide Wirkung. 7) Die Wirkstoffe I-34, I-35, I-36, I-37, I-38, I-39, I-40, I-41, I-47, I-48, I-49, I-51, bzw. I-52 zeigten bei einer Aufwandmenge von 0,5 kg/ha und der Wirkstoff I-50 bei 1,0 kg/ha im Nachauflauf gegen CHEAL eine sehr gute herbizide Wirkung. 8) Die Wirkstoffe I-35, I-37, I-38, I-44, I-45, I-48, bzw. I-49 zeigten bei einer Aufwandmenge von 0,5 kg/ha im Nachauflauf gegen ECHCG eine sehr gute herbizide Wirkung. 9) Die Wirkstoffe I-34, bzw. I-38 zeigten bei einer Aufwandmenge von 0,5 kg/ha im Nachauflauf gegen GALAP eine sehr gute herbizide Wirkung. 10) Die Wirkstoffe I-40, I-44, I-45, bzw. I-51 zeigten bei einer Aufwandmenge von 0,5 kg/ha und der Wirkstoff I-50 bei 1,0 kg/ha im Nachauflauf gegen SETVI eine sehr gute herbizide Wirkung. | | | |

## Patentansprüche

1. Pyridinverbindungen der Formel I 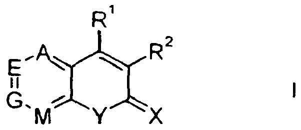 worin die Variablen folgende Bedeutung haben
R¹ O-R^{A}, S(O)ₙ-R^{A} oder O-S(O)ₙ-R^{A};
R^{A} Wasserstoff, C₁-C₄-Alkyl, Z-C₃-C₆-Cycloalkyl, C₁-C₄-Haloalkyl, C₂-C₆-Alkenyl, Z-C₃-C₆-Cycloalkenyl, C₂-C₆-Alkinyl, Z-(Tri-C₁-C₄-alkyl)silyl, Z-C(=O)-R^{a}, Z-NRⁱ-C(O)-NRⁱRⁱⁱ, Z-P(=O)(R^{a})₂, NRⁱRⁱⁱ, über C oder N gebundener 3- bis 7-gliedriger monocyclischer oder 9- oder 10-gliedriger bicyclischer gesättigter, ungesättigter oder aromatischer Heterocyclus, enthaltend 1, 2, 3 oder 4 Heteroatome ausgewählt aus O, N und S, der teilweise oder vollständig durch Gruppen R^{a} und/oder R^{b} substituiert sein kann,
R^{a} Wasserstoff, OH, C₁-C₈-Alkyl, C₁-C₄-Haloalkyl, Z-C₃-C₆-Cycloalkyl, C₂-C₈-Alkenyl, Z-C₅-C₆-Cycloalkenyl, C₂-C₈-Alkinyl, Z-C₁-C₆-Alkoxy, Z-C₁-C₄-Haloalkoxy, Z-C₃-C₈-Alkenyloxy, Z-C₃-C₈-Alkinyloxy, NRⁱRⁱⁱ, C₁-C₆-Alkylsulfonyl, Z-(Tri-C₁-C₄-alkyl)silyl, Z-Phenyl, Z-Phenoxy, Z-Phenylamino und 5- oder 6-gliedriger monocyclischer oder 9- oder 10-gliedriger bicyclischer Heterocyclus, enthaltend 1, 2, 3 oder 4 Heteroatome ausgewählt aus O, N und S, wobei die cyclischen Gruppen unsubstituiert oder durch 1, 2, 3 oder 4 Gruppen R^{b} substituiert sind, bedeutet;
Rⁱ,Rⁱⁱ unabhängig voneinander Wasserstoff, C₁-C₈-Alkyl, C₁-C₄-Haloalkyl, C₃-C₈-Alkenyl, C₃-C₈-Alkinyl, Z-C₃-C₆-Cycloalkyl, Z-C₁-C₈-Alkoxy, Z-C₁-C₈-Haloalkoxy, Z-C(=O)-R^{a}, Z-Phenyl, über Z gebundener 3- bis 7-gliedriger monocyclischer oder 9- oder 10-gliedriger bicyclischer gesättigter, ungesättigter oder aromatischer Heterocyclus, enthaltend 1, 2, 3 oder 4 Heteroatome ausgewählt aus O, N und S; Rⁱ und Rⁱⁱ können auch gemeinsam mit dem N-Atom, an das sie gebunden sind, einen 5- oder 6-gliedrigen monocyclischen oder 9- oder 10-gliedrigen bicyclischer Heterocyclus bilden, enthaltend 1, 2, 3 oder 4 Heteroatome ausgewählt aus O, N und S;
Z eine kovalente Bindung oder C₁-C₄-Alkylen;
n 0, 1 oder 2;
R² Phenyl, Naphthyl oder und 5- oder 6-gliedriger monocyclischer oder 9- oder 10-gliedriger bicyclischer aromatischer Heterocyclus, enthaltend 1, 2, 3 oder 4 Heteroatome ausgewählt aus O, N und S, wobei die cyclischen Gruppen unsubstituiert oder durch 1, 2, 3 oder 4 Gruppen R^{b} substituiert sind, bedeutet;
R^{b} unabhängig voneinander Z-CN, Z-OH, Z-NO₂, Z-Halogen, C₁-C₈-Alkyl, C₁-C₄-Haloalkyl, C₂-C₈-Alkenyl, C₂-C₈-Alkinyl, Z-C₁-C₈-Alkoxy, Z-C₁-C₈-Haloalkoxy, Z-C₃-C₁₀-Cycloalkyl, O-Z-C₃-C₁₀-Cycloalkyl, Z-C(=O)-R^{a}, NRⁱRⁱⁱ, Z-(Tri-C₁-C₄-alkyl)silyl, Z-Phenyl und S(O)ₙR^{bb}, wobei R^{bb} C₁-C₈-Alkyl und C₁-C₆-Haloalkyl bedeutet und
n für 0, 1 oder 2 steht;
R^{b} kann auch gemeinsam mit der an das benachbarte C-Atom gebundene Gruppe R^{b} einen fünf- oder sechsgliedrigen gesättigten, teilweise oder vollständig ungesättigten Ring bilden, der neben Kohlenstoff- 1, 2 oder 3 Heteroatome ausgewählt aus O, N und S enthalten kann;
X O, S oder N-R³;
Y O oder S;
R³ Wasserstoff, C₁-C₆-Alkyl, C₁-C₄-Haloalkyl, C₂-C₆-Alkenyl, C₃-C₆-Alkinyl, Z-C₃-C₁₀-Cycloalkyl, C₁-C₆-Alkoxy-C₁-C₆-alkyl, C₁-C₆-Cyanoalkyl, Z-Phenyl, Z-C(=O)-R^{a2} und Tri-C₁-C₄-alkylsilyl;
R^{a2} C₁-C₆-Alkyl, C₁-C₄-Haloalkyl, Z-C₁-C₆-Alkoxy, Z-C₁-C₄-Haloalkoxy und NRⁱRⁱⁱ;
A,E,G,M N und C-R^{c}, wobei eine Gruppe davon N bedeutet,
R^{c} Wasserstoff oder eine der bei R^{b} genannten Gruppen;
wobei in Gruppen R^{A}, R³ und deren Untersubstituenten die Kohlenstoffketten und/oder die cyclischen Gruppen teilweise oder vollständig durch Gruppen R^{b} substituiert sein können,
sowie deren N-Oxide und landwirtschaftlich geeignete Salze.

2. Verbindungen der Formel I gemäß Anspruch 1, worin
R¹ O-R^{A} oder S(O)ₙ-R^{A}; und
R^{A} Wasserstoff, C₁-C₄-Alkyl, Z-C₃-C₆-Cycloalkyl, C₁-C₄-Haloalkyl, C₂-C₆-Alkenyl, Z-C₃-C₆-Cycloalkenyl, C₂-C₆-Alkinyl, Z-(Tri-C₁-C₄-alkyl)silyl, Z-C(=O)-R^{a}, Z-P(=O)(R^{a})₂, über C oder N gebundener 3- bis 7-gliedriger monocyclischer oder 9- oder 10-gliedriger bicyclischer gesättigter, ungesättigter oder aromatischer Heterocyclus, enthaltend 1, 2, 3 oder 4 Heteroatome ausgewählt aus O, N und S, der teilweise oder vollständig durch Gruppen R^{a} und/oder R^{b} substituiert sein kann,
R^{a} Wasserstoff, OH, C₁-C₈-Alkyl, C₁-C₄-Haloalkyl, Z-C₃-C₆-Cycloalkyl, C₂-C₈-Alkenyl, Z-C₅-C₆-Cycloalkenyl, C₂-C₈-Alkinyl, Z-C₁-C₆-Alkoxy, Z-C₁-C₄-Haloalkoxy, Z-C₃-C₈-Alkenyloxy, Z-C₃-C₈-Alkinyloxy, NRⁱRⁱⁱ, C₁-C₆-Alkylsulfonyl, Z-(Tri-C₁-C₄-alkyl)silyl, Z-Phenyl, Z-Phenoxy, Z-Phenylamino und 5- oder 6-gliedriger monocyclischer oder 9- oder 10-gliedriger bicyclischer Heterocyclus, enthaltend 1, 2, 3 oder 4 Heteroatome ausgewählt aus O, N und S, wobei die cyclischen Gruppen unsubstituiert oder durch 1, 2, 3 oder 4 Gruppen R^{b} substituiert sind, bedeutet;
Rⁱ,Rⁱⁱ unabhängig voneinander Wasserstoff, C₁-C₈-Alkyl, C₁-C₄-Haloalkyl, C₃-C₈-Alkenyl, C₃-C₈-Alkinyl, Z-C₃-C₆-Cycloalkyl, Z-C₁-C₈-Alkoxy, Z-C₁-C₈-Haloalkoxy;
Rⁱ und Rⁱⁱ können auch gemeinsam mit dem N-Atom, an das sie gebunden sind, einen 5- oder 6-gliedrigen monocyclischen oder 9- oder 10-gliedrigen bicyclischer Heterocyclus bilden, enthaltend 1, 2, 3 oder 4 Heteroatome ausgewählt aus O, N und S;
bedeuten.

3. Verbindungen der Formel I gemäß Anspruch 1, worin Y für O steht.

4. Verbindungen der Formel I gemäß Anspruch 1, worin Y für S steht.

5. Verbindungen der Formel I gemäß einem der Ansprüche 1 bis 4, worin R^{A} für Wasserstoff oder C₁-C₆-Alkylcarbonyl steht.

6. Verbindungen der Formel I gemäß einem der Ansprüche 1 bis 5, worin X für O steht.

7. Verbindungen der Formel I gemäß einem der Ansprüche 1 bis 5, worin X für S steht.

8. Verbindungen der Formel I gemäß einem der Ansprüche 1 bis 7, welche der Formel I.1 entsprechen, 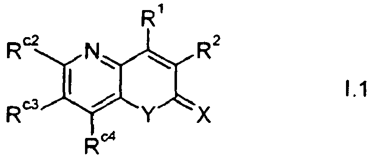 in der R^{c2}, R^{c3} und R^{c4} je einer Gruppe R^{c} entsprechen.

9. Verbindungen der Formel I gemäß Anspruch 8, in der R^{c2}, R^{c3} und R^{c4} für Wasserstoff stehen.

10. Verbindungen der Formel I gemäß einem der Ansprüche 1 bis 9, welche der Formel I.A entsprechen, 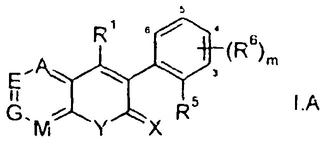 in der R⁵ und R⁶ für Gruppen R^{b} und m für Null oder eine ganze Zahl von eins bis vier stehen.

11. Verbindungen der Formel 1, welche der Formel I.1 gemäß Anspruch 8 entsprechen, worin
R^{c2}, R^{c3} und R^{c4} für H stehen,
R¹ OH, OCH₃, OC(O)CH₃, OC(O)CH₂CH₃, OC(O)CH(CH₃)₂, OC(O)C(CH₃)₃, OC(O)-c-C₃H₅, OC(O)-C₆H_{5,} OC(O)-CH₂C₆H₅, OC(O)CH₂Cl, OC(O)-CF₃, OC(O)-CH₂OCH₃, OC(O)-N(CH₃)₂ oder OC(O)-OCH₂CH₃;
R² Phenyl, welches substituiert ist durch eine Gruppe ausgewählt aus 2-Br, 2-Cl, 2,4-Cl₂, 2-Cl-4-F, 2-Cl-5-F, 2-Cl-6-F, 2-Cl-4-CF₃, 2-Cl-5-CF₃, 2-Cl-6-CF₃, 2-Cl-3,6-F₂, 2-F, 2,4-F₂, 2,5-F₂, 2,6-F₂, 2-F-4-CF₃, 2-F-5-CF₃, 2-F-6-CF₃, 2,3,6-F₃, 2-NO₂, 2-NO₂-4-F, 2-NO₂-5-F, 2-NO₂-6-F, 2-NO₂-4-CF₃. 2-NO₂-5-CF₃, 2-NO₂-6-CF₃, 2-NO₂-3,6-F₂, 2-CN, 2-CH₃, 2-CH₃-4-F, 2-CH₃-5-F, 2-CH₃-6-F, 2-CH₃-4-CF₃, 2-CH₃-5-CF₃, 2-CH₃-6-CF₃, 2-CH₃-3,6-F₂, 2-OCH₃, 2-OCH₃-4-F, 2-OCH₃-5-F, 2-OCH₃-6-F, 2-OCH₃-4-CF₃, 2-OCH₃-5-CF₃, 2-OCH₃-6-CF₃, 2-OCH₃-3,6-F₂, 2-CHF₂, 2-CHF₂-4-F, 2-CHF₂-5-F, 2-CHF₂-6-F, 2-CHF₂-4-CF₃, 2-CHF₂-5-CF₃, 2-CHF₂-6-CF₃, 2-CHF₂-3,6-F₂, 2-CF₃, 2-C_{F}3-4-F, 2-CF₃-5-F, 2-CF₃-6-F, 2-CF₃-4-CF₃, 2-CF₃-5-CF₃, 2-CF₃-6-CF₃, 2-CF₃-3,6-F₂, 2-OCHF₂, 2-OCHF₂-4-F, 2-OCHF₂-5-F, 2-OCHF₂-6-F, 2-OCHF₂-4-CF₃, 2-OCHF₂-5-CF₃, 2-OCHF₂-6-CF₃, 2-OCHF₂-3,6-F₂. 2-OCF₃, 2-OCF₃-4-F, 2-OCF₃-5-F, 2-OCF₃-6-F, 2-OCF₃-4-CF₃, 2-OCF₃-5-CF₃, 2-OCF₃-6-CF₃ oder 2-OCF₃-3,6-F₂; und
X,Y unabhängig voneinander für O oder S stehen.

12. Mittel, enthaltend eine herbizid wirksame Menge mindestens einer Pyridinverbindung der Formel 1 oder eines landwirtschaftlich geeigneten Salzes davon nach einem der Ansprüche 1 bis 11 und für die Formulierung von Pflanzenschutzmitteln übliche Hilfsmittel.

13. Mittel gemäß Anspruch 12, enthaltend mindestens einen weiteren Wirkstoff.

14. Mittel gemäß Anspruch 12 oder 13, enthaltend zwei weitere Wirkstoffe aus der Gruppe der Herbizide und/oder Safener.

15. Verfahren zur Bekämpfung von unerwünschtem Pflanzenwuchs, **dadurch gekennzeichnet, dass** man eine herbizid wirksame Menge mindestens einer Pyridinverbindung der Formel I oder eines landwirtschaftlich brauchbaren Salzes davon nach einem der Ansprüche 1 bis 11 auf Pflanzen, deren Samen und/oder deren Lebensraum einwirken lässt.

## Claims

1. A pyridine compound of the formula I 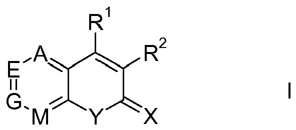 in which the variables have the following meaning:
R is O-R^{A}, S(O)ₙ-R^{A} or O-S(O)ₙ-R^{A};
R^{A} is hydrogen, C₁-C₄-alkyl, Z-C₃-C₆-cycloalkyl, C₁-C₄-haloalkyl, C₂-C₆-alkenyl, Z-C₃-C₆-cycloalkenyl, C₂-C₆-alkynyl, Z-(tri-C₁-C₄-alkyl) silyl, Z-C(=O)-R^{a}, Z-NR¹-C(O)-NR¹R¹¹, Z-P(=O)(R^{a})₂, NR¹R¹¹, a 3- to 7-membered monocyclic or 9- or 10-membered bicyclic saturated, unsaturated or aromatic heterocycle which contains 1, 2, 3 or 4 heteroatoms selected from the group consisting of 0, N and S, which may be partially or fully substituted by groups R^{a} and/or R^{b} and which is attached via carbon or nitrogen,
R^{a} is hydrogen, OH, C₁-C₈-alkyl, C₁-C₄-haloalkyl, Z-C₃-C₆-cycloalkyl, C₂-C₈-alkenyl, Z-C₅-C₆-cycloalkenyl, C₂-C₈-alkynyl, Z-C₁-C₆-alkoxy, Z-C₁-C₄-haloalkoxy, Z-C₃-C₈-alkenyloxy,
Z-C₃-C₈-alkynyloxy, NR¹R¹¹, C₁-C₆-alkylsulfonyl, Z-(tri-C₁-C₄-alkyl)silyl, Z-phenyl, Z-phenoxy, Z-phenylamino or a 5- or 6-membered monocyclic or 9- or 10-membered bicyclic heterocycle which contains 1, 2, 3 or 4 heteroatoms selected from the group consisting of 0, N and S, where the cyclic groups are unsubstituted or substituted by 1, 2, 3 or 4 groups R^{b};
R¹, R¹¹ independently of one another are hydrogen, C₁-C₈-alkyl, C₁-C₄-haloalkyl, C₃-C₈-alkenyl, C₃-C₈-alkynyl, Z-C₃-C₆-cycloalkyl, Z-C₁-C₈-alkoxy, Z-C₁-C₈-haloalkoxy, Z-C(=O)-R^{a}, Z-phenyl, a 3- to 7-membered monocyclic or 9- or 10-membered bicyclic saturated, unsaturated or aromatic heterocycle which contains 1, 2, 3 or 4 heteroatoms selected from the group consisting of 0, N and S and which is attached via Z;
Rⁱ and Rⁱⁱ together with the nitrogen atom to which they are attached may also form a 5- or 6-membered monocyclic or 9- or 10-membered bicyclic heterocycle which contains 1, 2, 3 or 4 heteroatoms selected from the group consisting of 0, N and S;
Z is a covalent bond or C₁-C₄-alkylene;
n is 0, 1 or 2;
R² is phenyl, naphthyl or a 5- or 6-membered monocyclic or 9- or 10-membered bicyclic aromatic heterocycle which contains 1, 2, 3 or 4 heteroatoms selected from the group consisting of 0, N and S, where the cyclic groups are unsubstituted or substituted by 1, 2, 3 or 4 groups R^{b};
R^{b} independently of one another are Z-CN, Z-OH, Z-NO₂, Z-halogen, C₁-C₈-alkyl, C₁-C₄-haloalkyl, C₂-C₈-alkenyl, C₂-C₈-alkynyl, Z-C₁-C₈-alkoxy, Z-C₁-C₈-haloalkoxy, Z-C₃-C₁₀-cycloalkyl, O-Z-C₃-C₁₀-cycloalkyl, Z-C(=O)-R^{a}, NR¹R¹¹, Z-(tri-C₁-C₄-alkyl)silyl, Z-phenyl and S(O)ₙR^{bb}, where R^{bb} is C₁-C₈-alkyl or C₁-C₆-haloalkyl and n is 0, 1 or 2;
R^{b} together with the group R^{b} attached to the adjacent carbon atom may also form a five- or six-membered saturated or partially or fully unsaturated ring which, in addition to carbon atoms, may contain 1, 2 or 3 heteroatoms selected from the group consisting of 0, N and S;
X is 0, S or N-R³;
Y is 0 or S;
R³ is hydrogen, C₁-C₆-alkyl, C₁-C₄-haloalkyl, C₂-C₆-alkenyl, C₃-C₆-alkynyl, Z-C₃-C₁₀-cycloalkyl, C₁-C₆-alkoxy-C₁-C₆-alkyl, C₁-C₆-cyanoalkyl, Z-phenyl, Z-C(=O)-R^{a2} or tri-C₁-C₄-alkylsilyl;
R^{a2} is C₁-C₆-alkyl, C₁-C₄-haloalkyl, Z-C₁-C₆-alkoxy, Z-C₁-C₄-haloalkoxy or NR¹R¹¹;
A, E, G, M are N or C-R^{c}, one of these groups being N;
R^{c} is hydrogen or one of the groups mentioned for R^{b};
where in the groups R^{A}, R³ and their subsubtituents, the carbon chains and/or the cyclic groups may be partially or fully substituted by groups R^{b},
or a N-oxide or an agriculturally suitable salt thereof.

2. The compound of the formula I according to claim 1 in which
R¹ O-R^{A} or S(O)ₙ-R^{A}; and
R^{A} is hydrogen, C₁-C₄-alkyl, Z-C₃-C₆-cycloalkyl, C₁-C₄-haloalkyl, C₂-C₆-alkenyl, Z-C₃-C₆-cycloalkenyl, C₂-C₆-alkynyl, Z-(tri-C₁-C₄-alkyl)silyl, Z-C(=O)-R^{a}, Z-P(=O)(R^{a})₂, a 3- to 7-membered monocyclic or 9- or 10-membered bicyclic saturated, unsaturated or aromatic heterocycle which contains 1, 2, 3 or 4 heteroatoms selected from the group consisting of 0, N and S which may be partially or fully substituted by groups R^{a} and/or R^{b} and which is attached via C or N,
R^{a} is hydrogen, OH, C₁-C₈-alkyl, C₁-C₄-haloalkyl, Z-C₃-C₆-cycloalkyl, C₂-C₈-alkenyl, Z-C₅-C₆-cycloalkenyl, C₂-C₈-alkynyl, Z-C₁-C₆-alkoxy, Z-C₁-C₄-haloalkoxy, Z-C₃-C₈-alkenyloxy, Z-C₃-C₈-alkynyloxy, NR¹R¹¹, C₁-C₆-alkylsulfonyl, Z-(tri-C₁-C₄-alkyl)silyl, Z-phenyl, Z-phenoxy, Z-phenylamino or a 5- or 6-membered monocyclic or 9- or 10-membered bicyclic heterocycle, comprising 1, 2, 3 or 4heteroatoms selected from the group consisting of 0, N and S, where the cyclic groups are unsubstituted or substituted by 1, 2, 3 or 4 groups R^{b};
R¹,R¹¹ independently of one another are hydrogen, C₁-C₈-alkyl, C₁-C₄-haloalkyl, C₃-C₈-alkenyl, C₃-C₈-alkynyl, Z-C₃-C₆-cycloalkyl, Z-C₁-C₈-alkoxy, Z-C₁-C₈-haloalkoxy;
Rⁱ and Rⁱⁱ together with the nitrogen atom to which they are attached may also form a 5- or 6-membered monocyclic or 9- or 10-membered bicyclic heterocycle which contains 1, 2, 3 or 4 heteroatoms selected from the group consisting of 0, N and S.

3. The compound of the formula I according to claim 1 in which Y is O.

4. The compound of the formula I according to claim 1 in which Y is S.

5. The compound of the formula I according to any of claims 1 to 4 in which R^{A} is hydrogen or C₁-C₆-alkylcarbonyl.

6. The compound of the formula I according to any of claims 1 to 5 in which X is O.

7. The compound of the formula I according to any of claims 1 to 5 in which X is S.

8. The compound of the formula I according to any of claims 1 to 7 which corresponds to the formula I.1 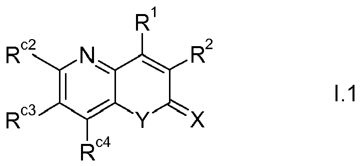 which R^{c2}, R^{c3} and R^{c4} each correspond to a group R^{c}.

9. The compound of the formula I according to claim 8, in which R^{c2}, R^{c3} and R^{c4} are hydrogen.

10. The compound of the formula I according to any of claims 1 to 9 which corresponds to the formula I.A 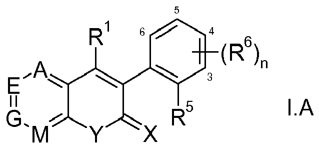 in which R⁵ and R⁶ are groups R^{b} and m is zero or an integer from one to four.

11. The compound of the formula I which corresponds to the formula I.1 according to claim 8 and in which
R^{c2}, R^{c3} and R^{c4} are H,
R¹ is OH, OCH₃, OC(O)CH₃, OC(O)CH₂CH₃, OC (0) CH (CH₃)₂, OC(O)C(CH₃)₃, OC(O)-C-C₃H₅, OC (0) -C₆H₅, OC (0) -CH₂C₆H₅, OC(O)CH₂Cl, OC(O)-CF₃, OC(O)-CH₂OCH₃, OC(O)-N(CH₃)₂ or OC(O) -OCH₂CH₃;
R² is phenyl substituted by a group selected from the group consisting of 2-Br,
2-Cl, 2,4-Cl₂, 2-Cl-4-F, 2-Cl-5-F, 2-Cl-6-F, 2-Cl-4-CF₃, 2-Cl-5-CF₃, 2-Cl-6-CF₃, 2-Cl-3,6-F₂, 2-F, 2,4-F₂, 2,5-F₂, 2,6-F₂, 2-F-4-CF₃, 2-F-5-CF₃, 2-F-6-CF₃, 2, 3, 6-F₃, 2-NO₂, 2-NO₂-4-F, 2-NO₂-5-F, 2-NO₂-6-F, 2-NO₂-4-CF₃,
2-NO₂-5-CF₃, 2-NO₂-6-CF₃, 2-NO₂-3,6-F₂, 2-CN, 2-CH₃, 2-CH₃-4-F, 2-CH₃-5-F, 2-CH₃-6-F, 2-CH₃-4-CF₃, 2-CH₃-5-CF₃, 2-CH₃-6-CF₃, 2-CH₃-3, 6-_{F}2,
2-OCH₃, 2-OCH₃-4-F, 2-OCH₃-5-F, 2-OCH₃-6-F, 2-OCH₃-4-CF₃, 2-OCH₃-5-CF₃, 2-OCH₃-6-CF₃, 2-OCH₃-3, 6-F₂, 2-CHF₂, 2-CHF₂-4-F, 2-CHF₂-5-F,
2-CHF₂-6-F, 2-CHF₂-4-CF₃, 2-CHF₂-5-CF₃, 2-CHF₂-6-CF₃, 2-CHF₂-3,6-F₂, 2-CF₃, 2-CF₃-4-F, 2-CF₃-5-F, 2-CF₃-6-F, 2-CF₃-4-CF₃, 2-CF₃-5-CF₃, 2-CF₃-6-CF₃, 2-CF₃-3, 6-F₂, 2-OCHF₂, 2-OCHF₂-4-F, 2-OCHF₂-5-F, 2-OCHF₂-6-F, 2-OCHF₂-4-CF₃, 2-OCHF₂-5-CF₃, 2-OCHF₂-6-CF₃, 2-OCHF₂-3,6-F₂,
2-OCF₃, 2-OCF₃-4-F, 2-OCF₃-5-F, 2-OCF₃-6-F, 2-OCF₃-4-CF₃, 2-OCF₃-5-CF₃, 2-OCF₃-6-CF₃ and 2-OCF₃-3, 6-F₂; and
X, Y independently of one another are 0 or S.

12. A composition comprising a herbicidally effective amount of at least one pyridine compound of the formula I or an agriculturally suitable salt thereof according to any of claims 1 to 11 and auxiliaries customary for formulating crop protection agents.

13. The composition according to claim 12 which comprises at least one further active compound.

14. The composition according to claim 12 or 13 which comprises two further active compounds from the group of the herbicides and/or safeners.

15. A method for controlling unwanted vegetation which comprises allowing a herbicidally effective amount of at least one pyridine compound of the formula I or of an agriculturally suitable salt thereof according to any of claims 1 to 11 to act on plants, their seed and/or their habitat.

## Revendications

1. Composés de type pyridine de formule I 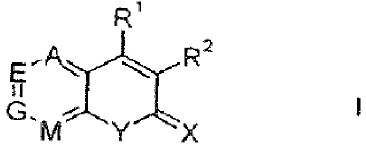 dans laquelle les variables ont la signification suivante
R¹ représente O-R^{A}, S(O)ₙ-R^{A} ou O-S(O)ₙ-R^{A} ;
R^{A} représente un atome d'hydrogène, un groupe alkyle en C₁-C₄, 2-cycloalkyle(C₃-C₆), halogénoalkyle en C₁-C₄, alcényle en C₂-C₆, 2-cycloalcényle(C₃-C₆), alcynyle en C₂-C₆, Z-[trialkyl(C₁-C₄)]]silyle, 2-C(=O)-R^{a}, 2-NR¹-C(O)-NR¹R¹¹, Z-P(=O)(R^{a})₂, NR¹R¹¹, un hétérocycle monocyclique à 3-7 chaînons ou bicyclique à 9 ou 10 chaînons, saturé, insaturé ou aromatique, lié par C ou N, contenant 1, 2, 3 ou 4 hétéroatomes choisis parmi 0, N et S, qui peut être partiellement ou totalement substitué par des groupes R^{a} et/ou R^{b}
R^{a} représente un atome d'hydrogène, OH, un groupe alkyle en C₁-C₈, halogénoalkyle en C₁-C₄, Z-cycloalkyle(C₃-C₆), alcényle en C₂-C₈, Z-cycloalcényle(C₅-C₆), alcynyle en C₂-C₈, Z-alcoxy(C₁-C₆), Z-halogéno-alcoxy(C₁-C₄), Z-alcényloxy(C₃-C₈), Z-alcynyloxy (C₃-C₈), NRⁱRⁱⁱ, alkyl(C₁-C₆)sulfonyle, 2-[trialkyl(C₁-C₄)]silyle, Z-phényle, Z-phénoxy, Z-phénylamino et un hétérocycle monocyclique à 5 ou 6 chaînons ou bicyclique à 9 ou 10 chaînons, contenant 1, 2, 3 ou 4 hétéroatomes choisis parmi 0, N et S, les groupes cycliques étant non substitués ou substitués par 1, 2, 3 ou 4 groupes R^{b} ;
R¹, R¹¹ représentent indépendamment l'un de l'autre un atome d'hydrogène, un groupe alkyle en C₁-C₈, halogénoalkyle en C₁-C₄, alcényle en C₃-C₈, alcynyle en C₃-C₈, 2-cycloalkyle(C₃-C₆), Z-alcoxy(C₁-C₈), 2-halogénoalcoxy(C₁-C₈), Z-C(=O)-R^{a}, Z-phényle, un hétérocycle monocyclique à 3-7 chaînons ou bicyclique à 9 ou 10 chaînons, saturé, insaturé ou aromatique, lié par Z, contenant 1, 2, 3 ou 4 hétéroatomes choisis parmi 0, N et S ;
Rⁱ et Rⁱⁱ peuvent également former ensemble avec l'atome d'azote, auquel ils sont liés, un hétérocycle monocyclique à 5 ou 6 chaînons ou bicyclique à 9 ou 10 chaînons, contenant 1, 2, 3 ou 4 hétéroatomes choisis parmi 0, N et S ;
Z représente une liaison covalente ou un groupe alkylène en C₁-C₄ ;
n vaut 0, 1 ou 2 ;
R² représente un groupe phényle, naphtyle ou un hétérocycle aromatique monocyclique à 5 ou 6 chaînons ou bicyclique à 9 ou 10 chaînons, contenant 1, 2, 3 ou 4 hétéroatomes choisis parmi 0, N et S, les groupes cycliques étant non substitués ou substitués par 1, 2, 3 ou 4 groupes R^{b} ;
R^{b} représente chaque fois indépendamment Z-CN, Z-OH, Z-NO₂, Z-halogène, alkyle en C₁-C₈, halogénoalkyle en C₁-C₄, alcényle en C₂-C₈, alcynyle en C₂-C₈, Z-alcoxy(C₁-C₈), Z-halogénoalcoxy(C₁-C₈), Z-cycloalkyle(C₃-C₁₀), O-Z-cycloalkyle (C₃-C₁₀) Z-C(=O)-R^{a}, NRⁱRⁱⁱ, Z-[trialkyl(C₁-C₄)]silyle, Z-phényle et S(O)ₙR^{bb}, R^{bb} représentant un groupe alkyle en C₁-C₈ ou halogénoalkyle en C₁-C₆ et
n valant 0, 1 ou 2 ;
R^{b} peut également former conjointement avec le groupe R^{b} lié à l'atome de carbone voisin un cycle à cinq ou six chaînons saturé, partiellement ou totalement insaturé, qui peut en plus d'atomes de carbone contenir 1, 2 ou 3 hétéroatomes choisis parmi 0, N et S ;
X représente 0, S ou N-R³;
Y représente 0 ou S ;
R³ représente un atome d'hydrogène, un groupe alkyle en C₁-C₆, halogénoalkyle en C₁-C₄, alcényle en C₂-C₆, alcynyle en C₃-C₆, Z-cycloalkyle(C₃-C₁₀), alcoxy(C₁-C₆)-alkyle(C₁-C₆), cyanoalkyle(C₁-C₆), Z-phényle, Z-C(=O)-R^{a2} et trialkyl(C₁-C₄)silyle ;
R^{a2} représente un groupe alkyle en C₁-C₆, halogénoalkyle en C₁-C₄, Z-alcoxy(C₁-C₆) Z-halogénoalcoxy(C₁-C₄) ou NRⁱRⁱⁱ;
A, E, G, M représentent N et C-R^{c}, un groupe parmi ceux-ci représentant N,
R^{c} représente un atome d'hydrogène ou l'un des groupes nommés pour R^{b} ;
dans les groupes R^{A}, R³ et leurs sous-substituants les chaînes carbonées et/ou les groupes cycliques pouvant être partiellement ou totalement substitués par des groupes R^{b},
ainsi que leurs N-oxydes et sels appropriés en agriculture.

2. Composés de formule I selon la revendication 1, dans lesquels
R¹ représente O-R^{A} ou S(O)ₙ-R^{A} ; et
R^{A} représente un atome d'hydrogène, un groupe alkyle en C₁-C₄, Z-cycloalkyle(C₃-C₆), halogénoalkyle en C₁-C₄, alcényle en C₂-C₆, 2-cycloalcényle(C₃-C₆), alcynyle en C₂-C₆, Z-[trialkyl(C₁-C₄)] silyle, 2-C(=O)-R^{a}, Z-P(=O)(R^{a})₂, un hétérocycle monocyclique à 3-7 chaînons ou bicyclique à 9 ou 10 chaînons, saturé, insaturé ou aromatique, lié par C ou N, contenant 1, 2, 3 ou 4 hétéroatomes choisis parmi 0, N et S, qui peut être partiellement ou totalement substitué par des groupes R^{a} et/ou R^{b},
R^{a} représente un atome d'hydrogène, OH, un groupe alkyle en C₁-C₈, halogénoalkyle en C₁-C₄, Z-cycloalkyle(C₃-C₆), alcényle en C₂-C₈, Z-cycloalcényle(C₅-C₆), alcynyle en C₂-C₈, Z-alcoxy(C₁-C₆), Z-halogéno-alcoxy(C₁-C₄), Z-alcényloxy(C₃-C₈), Z-alcynyloxy (C₃-C₈), NRⁱRⁱⁱ, alkyl(C₁-C₆)sulfonyle, Z-[trialkyl(C₁-C₄)]silyle, Z-phényle, Z-phénoxy, Z-phénylamino et un hétérocycle monocyclique à 5 ou 6 chaînons ou bicyclique à 9 ou 10 chaînons, contenant 1, 2, 3 ou 4 hétéroatomes choisis parmi 0, N et S, les groupes cycliques étant non substitués ou substitués par 1, 2, 3 ou 4 groupes R^{b} ;
Rⁱ, Rⁱⁱ représentent indépendamment l'un de l'autre un atome d'hydrogène, un groupe alkyle en C₁-C₈, halogénoalkyle en C₁-C₄, alcényle en C₃-C₈, alcynyle en C₃-C₈, Z-cycloalkyle(C₃-C₆), Z-alcoxy(C₁-C₈), Z-halogénoalcoxy(C₁-C₈) ;
R¹ et R¹¹ peuvent également former ensemble avec l'atome d'azote, auquel ils sont liés, un hétérocycle monocyclique à 5 ou 6 chaînons ou bicyclique à 9 ou 10 chaînons, contenant 1, 2, 3 ou 4 hétéroatomes choisis parmi 0, N et S.

3. Composés de formule I selon la revendication 1, dans lesquels Y représente O.

4. Composés de formule I selon la revendication 1, dans lesquels Y représente S.

5. Composés de formule I selon l'une quelconque des revendications 1 à 4, dans lesquels R^{A} représente un atome d'hydrogène ou un groupe alkyl(C₁-C₆)carbonyle.

6. Composés de formule I selon l'une quelconque des revendications 1 à 5, dans lesquels X représente O.

7. Composés de formule I selon l'une quelconque des revendications 1 à 5, dans lesquels X représente S.

8. Composés de formule I selon l'une quelconque des revendications 1 à 7, qui correspondent à la formule I.1 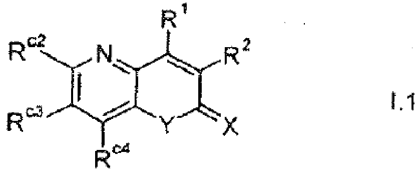 dans laquelle R^{c2}, R^{c3} et R^{c4} correspondent chacun à un groupe R^{c}.

9. Composés de formule I selon la revendication 8, dans lesquels R^{c2}, R^{c3} et R^{c4} représentent des atomes d'hydrogène.

10. Composés de formule I selon l'une quelconque des revendications 1 à 9, qui correspondent à la formule I.A, 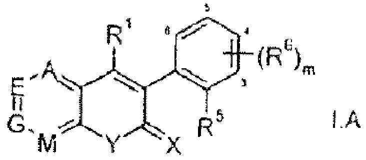 dans laquelle R⁵ et R⁶ représentent des groupes R^{b} et m représente zéro ou un nombre entier valant de un à quatre.

11. Composés de formule I, qui correspondent à la formule I.1 selon la revendication 8, dans lesquels
R^{c2}, R^{c3} et R^{c4} représentent H,
R¹ représente OH, OCH₃, OC(O)CH₃, OC (0) CH₂CH₃, OC(O)CH(CH₃)₂, OC(O)C(CH₃)₃, OC(O) -c-C₃H₅, OC(O) -C₆H₅, OC(O) -CH₂C₆H₅, OC(O)CH₂Cl, OC(O) -CF₃, OC(O)-CH₂OCH₃, OC(O)-N(CH₃)₂ ou OC(O)-OCH₂CH₃ ;
R² représente un groupe phényle qui est substitué par un groupe choisi parmi 2-Br, 2-Cl, 2,4-Cl_{2,} 2-Cl-4-F, 2-Cl-5-F, 2-Cl-6-F, 2-Cl-4-CF₃, 2-Cl-5-F₃, 2-Cl-6-CF₃, 2-Cl-3,6-F₂, 2-F, 2,4-F₂, 2,5-F₂, 2,6-F₂, 2-F-4-CF₃, 2-F-5-CF₃, 2-F-6-CF₃, 2,3,6-F₃, 2-NO₂, 2-NO₂-4-F, 2-NO₂-5-F, 2-NO₂-6-F, 2-NO₂-4-CF₃, 2-NO₂-5-CF₃, 2-NO₂-6-CF₃, 2-NO₂-3, 6-_{F}2, 2-CN, 2-CH₃, 2-CH₃-4-F, 2-CH₃-5-F, 2-CH₃-6-F, 2-CH₃-4-CF₃, 2-CH₃-5-CF₃, 2-CH₃-6-CF₃, 2-CH₃-3, 6-F₂, 2-OCH₃, 2-OCH₃-4-F, 2-OCH₃-5-F, 2-OCH₃-6-F, 2-OCH₃-4-CF₃, 2-OCH₃-5-CF₃, 2-OCH₃-6-CF₃, 2-OCH₃-3, 6-F₂, 2-CHF₂, 2-CHF₂-4-F, 2-CHF₂-5-F, 2-CHF₂-6-F, 2-CHF₂-4-CF₃, 2-CHF₂-5-CF₃, 2-CHF₂-6-CF₃, 2-CHF₂-3,6-F₂, 2-CF₃, 2-CF₃-4-F, 2-CF₃-5-F, 2-CF₃-6-F, 2-CF₃-4-CF₃, 2-CF₃-5-CF₃, 2-CF₃-6-CF₃, 2-CF₃-3, 6-_{F}2, 2-OCHF₂, 2-OCHF₂-4-F, 2-OCHF₂-5-F, 2-OCHF₂-6-F, 2-OCHF₂-4-CF₃, 2-OCHF₂-5-CF₃, 2-OCHF₂-6-CF₃, 2-OCHF₂-3, 6-F₂, 2-OCF₃, 2-OCF₃-4-F, 2-OCF₃-5-F, 2-OCF₃-6-F, 2-OCF₃-4-CF₃, 2-OCF₃-5-CF₃, 2-OCF₃-6-CF₃ ou 2-OCF₃-3,6-F₂ ; et
X, Y représentent indépendamment l'un de l'autre 0 ou S.

12. Composition, contenant une quantité à activité herbicide d'au moins un composé de type pyridine de formule I ou d'un sel approprié en agriculture d'un tel composé, selon l'une quelconque des revendications 1 à 11, et des adjuvants usuels pour la formulation de produits phytosanitaires.

13. Composition selon la revendication 12, contenant au moins une autre substance active.

14. Composition selon la revendication 12 ou 13, contenant deux autres substances actives choisies dans le groupe des herbicides et/ou des phytoprotecteurs.

15. Procédé pour la lutte contre la croissance indésirable de plantes, **caractérisé en ce qu'**on fait agir sur des plantes, leurs semences et/ou leur habitat une quantité à activité herbicide d'au moins un composé de type pyridine de formule I ou d'un sel utilisable en agriculture d'un tel composé, selon l'une quelconque des revendications 1 à 11.
